(19) Europäisches Patentamt
European Patent Office
Office européen des brevets

(11) EP 3 656 382 A1

(12) EUROPEAN PATENT APPLICATION

(43) Date of publication:
**27.05.2020 Bulletin 2020/22**

(21) Application number: **19193344.9**

(22) Date of filing: **29.01.2018**

(51) Int Cl.:
***A61K 31/165*** *(2006.01)* ***A61K 31/4706*** *(2006.01)*
***A61K 31/519*** *(2006.01)* ***A61P 25/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **30.01.2017 EP 17153834**
**10.10.2017 EP 17195823**

(62) Document number(s) of the earlier application(s) in accordance with Art. 76 EPC:
**18701194.5 / 3 573 608**

(27) Previously filed application:
**29.01.2018 PCT/EP2018/052165**

(71) Applicant: **Université de Liège**
**4000 Liège (BE)**

(72) Inventors:
- **Nguyen, Laurent**
  **4130 Tilff (BE)**
- **Creppe, Catherine**
  **4000 Liège (BE)**
- **Alfano, Christian**
  **4000 Boncelles (BE)**
- **Gladwyn-ng, Ivan**
  **4000 Liège (BE)**
- **Cordon Barris, Lluis**
  **B-4000 Liège (BE)**

(74) Representative: **LC Patents**
**Kempische Steenweg 542A**
**3500 Hasselt (BE)**

# (54) PERK AND IRE-1A INHIBITORS AGAINST NEURODEVELOPMENTAL DISORDERS

(57) The present invention relates to UPR pathway inhibitors, in particular PERK and IRE-1A inhibitors for use in the prevention or treatment of neurodevelopmental disorders, such as microcephaly caused by ZIKV.

EP 3 656 382 A1

## Description

### FIELD OF THE INVENTION

The present invention relates to molecules capable of reducing the endoplasmic reticulum (ER) stress-induced activation of the Unfolded Protein Response (UPR) pathway, hereinafter referred to as UPR pathway inhibitors for use in the prevention or treatment of ER stress-related (or caused) disorders, in particular disorders originating from disturbances in the development of the fetal nervous system; more in particular neurodevelopmental disorders, such as microcephaly caused by ZIKV. In one embodiment said UPR inhibitors are PERK and/or IRE-1A inhibitors for use in the prevention or treatment of endoplasmic reticulum (ER) stress disorders, in particular disorders originating from disturbances in the development of the fetal nervous system; more in particular neurodevelopmental disorders, such as microcephaly caused by ZIKV.

### BACKGROUND TO THE INVENTION

The cerebral cortex is a highly evolved and complex brain region that computes higher cognitive functions. Its development commences from the formation and patterning of the neural tube, which progressively generates different classes of neurons, that eventually settle into six radially organized layers. In human, most malformations of cortical development (MCD) affect cells that contribute to the formation of the cerebral cortex mostly during the first two trimesters of pregnancy. Microcephaly is a common MCD, resulting clinically in a reduction in head circumference and brain size, due to impaired generation and/or decreased survival of neurons or of their progenitors. Whilst congenital microcephaly is often linked to genetic alterations, environmental factors and infectious agents transmitted vertically may also lead to this defect. Recent observations of human fetuses, as well as experimental data obtained by using animal models, strongly support that maternal-fetal transmission of ZIKV, a mosquito-borne virus of the *Flaviviridae* family, leads to an increased incidence of microcephaly. Furthermore, once ZIKV infects cortical neuronal progenitors it impairs both the generation and survival of neurons and thus selectively interferes with important developmental steps underlying cortex formation. However, the *primum movens* and the underlying molecular mechanisms of ZIKV-induced microcephaly remain unclear.

The present invention relates to:

- protein kinase RNA-like endoplasmic reticulum kinase (PERK) inhibitors which are already known for elevating blood insulin and treatment of diabetes, (e.g. WO2016126026; WO2012158123), (combination) therapies of cancer (e.g. WO2016043874; WO2016025635; US20120077828; WO2011119663, US20160015709, US20140364453), cell stabilization for dry storage at ambient temperatures (e.g. WO2015002729), vanishing white matter disease or childhood ataxia (WO2014144952), etc.
- Inositol Requiring Enzymes inhibitors (IRE-1A inhibitors, also called IRE-1$\alpha$ inhibitors) which are already known for elevating blood insulin and treatment of diabetes, therapies of cancer (e.g. WO2008154484, EP3150589, EP2532643).

The present invention relates to molecules capable of reducing the endoplasmic reticulum (ER) stress-induced activation of the Unfolded Protein Response (UPR) pathway, hereinafter referred to as UPR pathway inhibitors for use in the prevention or treatment of endoplasmic reticulum (ER) stress-induced disorders. It is well known that UPR is activated by alteration of the normal ER activity due to impaired protein translation and/or folding. The UPR pathway has three arms, which are triggered by three receptors: protein kinase RNA-like endoplasmic reticulum kinases (PERK), inositol-requiring enzymes (IRE-1) and activating transcription factor 6 (ATF6). These three branches are interconnected through several mechanisms (Tomasio et al., 2013; Tsuru et al., 2016; Jiang et al, 2015). The binding immunoglobulin protein (BiP) for instance holds these proteins in an inactive state until it binds unfolded proteins and dissociates, allowing their activation (Walter & Ron, 2011; Halliday & Mallucci, 2015)

However, neither UPR inhibitors, more in particular PERK inhibitors and IRE-1A inhibitors, nor combinations or uses thereof in the prevention or treatment of endoplasmic reticulum (ER) stress disorders, in particular disorders originating from disturbances in the development of the fetal nervous system; more in particular neurodevelopmental disorders, such as microcephaly caused by ZIKV, have been described yet.

### SUMMARY OF THE INVENTION

The present invention relates to UPR pathway inhibitors, such as PERK inhibitors and/or IRE-1A inhibitors, for use in the prevention or treatment of endoplasmic reticulum (ER) stress disorders, in particular disorders originating from disturbances in the development of the fetal nervous system; more in particular neurodevelopmental disorders,

such as microcephaly caused by ZIKV

In an embodiment, the PERK inhibitors are represented by the following formula (I),

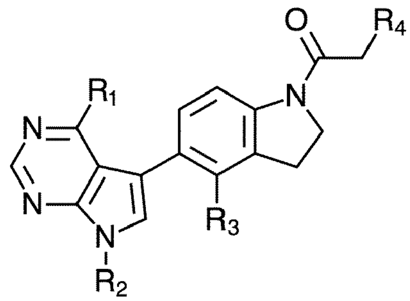

(I)

wherein

R1, R2 and R3 are independently selected from H, $C_{1-6}$alkyl, amino or halo; and
R4 is selected from:
Aryl;
aryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$ and-CN;
heteroaryl;
heteroaryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$, -CN and cyclo$C_{3-6}$alkyl, and;
cyclo$C_{3-6}$alkyl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$ and -CN;
or a salt thereof including a pharmaceutically acceptable salt thereof.
In another embodiment, the PERK inhibitors are represented by formula (I),
wherein
R1, R2 and R3 are independently selected from H, $C_{1-6}$alkyl, amino or halo; and
R4 is a heteroaryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$, -CN, and cyclo$C_{3-6}$alkyl;
or a salt thereof including a pharmaceutically acceptable salt thereof.

In a further embodiment, the PERK inhibitors are represented by formula (I), wherein

R1 is an amino;
R2 is an $C_{1-6}$alkyl;
R3 is a halo; and
R4 is a heteroaryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$, -CN, and cyclo$C_{3-6}$alkyl; in particular R4 is a heteroaryl substituted with one substituent independently selected from $C_{1-6}$alkyl, $C_{1-6}$alkyloxy, $-C_{1-6}$alkylO$C_{1-6}$alkyl, - $CF_3$, and cyclo$C_{3-6}$alkyl; more in particular R4 is pyridinyl substituted with one substituent independently selected from $C_{1-6}$alkyl, $C_{1-6}$alkyloxy, $-C_{1-6}$alkylO$C_{1-6}$alkyl, - $CF_3$, and cyclo$C_{3-6}$alkyl; even more in particular R4 is a heteroaryl substituted with one $C_{1-6}$alkyl; more in particular pyridinyl substituted with one $C_{1-6}$alkyl;
or a salt thereof including a pharmaceutically acceptable salt thereof.

In another embodiment, the PERK inhibitors are represented by the following formula (II),

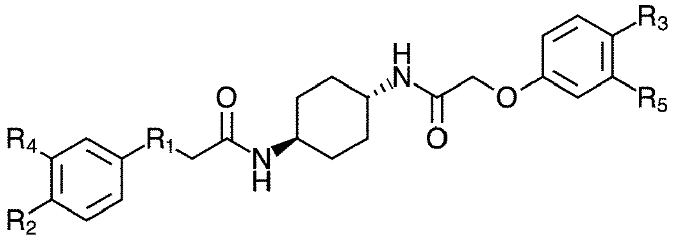

(II)

wherein

R1 is -O- or -NH-; and
R2, R3, R4 and R5 are independently selected from H, halo, $-CF_3$, $C_{1-6}$alkyl, -CN or $C_{1-6}$alkoxy; or a salt thereof including a pharmaceutically acceptable salt thereof.

In a further embodiment, the PERK inhibitors are represented by formula (II), wherein

R1 is -O- or -NH-;
R2 and R3 are independently selected from H, halo, $-CF_3$, $C_{1-6}$alkyl, -CN or $C_{1-6}$alkoxy; and
R4 and R5 are H;
or a salt thereof including a pharmaceutically acceptable salt thereof.

In a further embodiment, the PERK inhibitors are represented by formula (II), wherein

R1 is -O-;
R2 and R3 are independently selected halo; in particular chloro; and
R4 and R5 are H;
or a salt thereof including a pharmaceutically acceptable salt thereof.

In another embodiment, the PERK inhibitors are represented by the following formula (III),

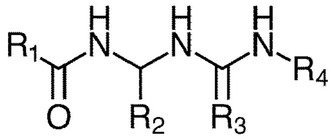

(III)

wherein

R1 and R4 are each independently selected from the group consisting of H, $C_{1-6}$alkyl, $C_{1-6}$alkenyl, $C_{1-6}$alkynyl, $C_{1-6}$alkoxy, $C_{1-6}$alkenoxy, $C_{1-6}$alkynoxy, thio$C_{1-6}$alkoxy, hydroxy$C_{1-6}$alkyl, aliphatic $C_{1-6}$acyl, $-CF_3$, carboxy, $-C_{1-6}$alkylamino, $C_{1-6}$alkenylamino, $C_{1-6}$alkynylamino, di($C_{1-6}$alkyl)amino,-C(O)O-($C_{1-6}$alkyl), -C(O)NH-($C_{1-6}$alkyl), -C(O)N($C_{1-6}$alkyl$)_2$, halo$C_{1-6}$alkyl, $C_{1-6}$alkoxy$C_{1-6}$alkoxy, carboxaldehyde, carboxamide, cyclo$C_{3-6}$alkyl, cyclo$C_{3-6}$alkenyl, cyclo$C_{3-6}$alkynyl, cyclo$C_{3-6}$alkyl$C_{1-6}$alkyl, aryl, aroyl, aryloxy, arylamino, biaryl, thioaryl, diarylamino, heterocyclyl, $C_{1-6}$alkylaryl, aralkenyl, aralkyl, alkylheterocyclyl, heterocyclylalkyl, aryloxy$C_{1-6}$alkyl, carboxyl, carbamate and -C(O)NH(benzyl);
R2 is selected from H, halo or a $C_{1-6}$alkyl optionally substituted with one or more halo; and
R3 is selected from the group consisting of O, S and NR5;
wherein R1, R4 and R5 are unsubstituted or substituted with at least one electron donating or electron withdrawing group;
or a salt thereof including a pharmaceutically acceptable salt thereof.

In a particular embodiment, the PERK inhibitors are represented by formula (III), wherein

R1 and R4 are each independently selected from the group consisting of aryl, heterocyclyl, aralkenyl, aralkyl and heterocyclylalkyl;
R2 is a $C_{1-6}$alkyl optionally substituted with one or more halo; and
R3 is S;
wherein R1 and R4 are unsubstituted or substituted with at least one electron donating or electron withdrawing group; or a salt thereof including a pharmaceutically acceptable salt thereof.

In a further embodiment, the PERK inhibitors are represented by formula (III), wherein

R1 and R4 are each independently selected from the group consisting of aryl and aralkenyl; in particular R1 is aryl and R4 is aralkenyl;
R2 is a $C_{1-6}$alkyl optionally substituted with one or more halo; in particular chloro; and
R3 is S;
wherein R1 and R4 are unsubstituted or substituted with at least one electron donating or electron withdrawing group; or a salt thereof including a pharmaceutically acceptable salt thereof.

In a preferred embodiment, the PERK inhibitors are selected from:

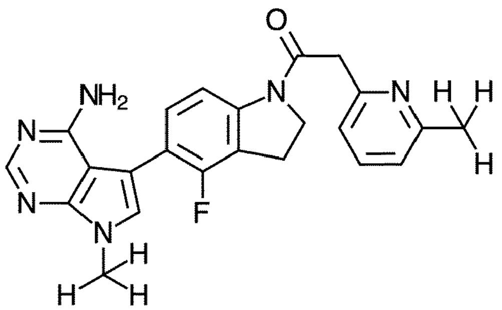

or

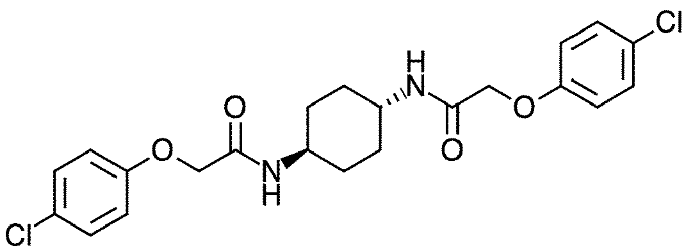

or

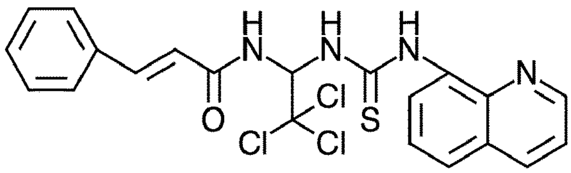

or a salt thereof including a pharmaceutically acceptable salt thereof.

As mentioned here above, the invention relates to UPR pathway inhibitors, such as PERK inhibitors inhibitors for use in the prevention or treatment of endoplasmic reticulum (ER) stress disorders, in particular disorders originating from disturbances in the development of the fetal nervous system; more in particular neurodevelopmental disorders, such as microcephaly caused by ZIKV.

In a certain embodiment, the endoplasmic reticulum (ER) stress disorders are neurodevelopmental disorders related to an endoplasmic reticulum (ER) stress disorder.

In another embodiment, the neurodevelopmental disorders related to an endoplasmic reticulum (ER) stress

disorder comprise but are not limited to microcephaly.

In a more specific embodiment, the neurodevelopmental disorders related to an endoplasmic reticulum (ER) stress disorder are caused by a viral infection in a fetal phase.

In an even more specific embodiment, the neurodevelopmental disorders related to an endoplasmic reticulum (ER) stress disorder are caused by Zika virus (ZIKV); more in particular by a Zika viral infection in a fetal phase.

Last, in a certain embodiment, the PERK inhibitors are developed for in-vitro research.

In an embodiment, the IRE-1A inhibitors are represented by the following formula (IV),

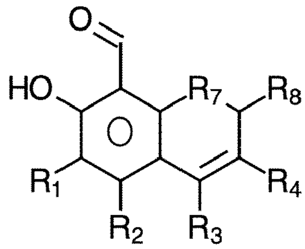

(IV)

wherein:

R1, R2 and R8 independently are H, =O, halogen, -OH, $C_1$-$C_6$ alkenyl, $C_1$-$C_6$ alkynyl, phenyl, optionally substituted $C_1$-$C_6$ alkyl, optionally substituted $C_1$-$C_6$ alkoxyl, or optionally substituted mono- or dialkylamino. Optional substituents for the $C_1$-$C_6$ alkyl, the $C_1$-$C_6$ alkoxyl or the mono- or dialkylamino are (1) a $C_1$-$C_6$ hydrocarbon chain containing a N or O atom and optionally substituted with halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, and (2) a cycloalkyl which may contain one or more heteroatoms selected from N, O, and S, and which is optionally substituted with halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl;

R3 or R4 are independently H, halogen, -OH, $C_1$-$C_6$ perfluoroalkyl, $C_1$-$C_6$ perfluoroalkoxy, -CN, $-CONH_2$, $-CON(CH_3)_2$, $C_1$-$C_6$ alkyl, $C_1$-$C_6$ alkenyl, $C_1$-$C_6$ alkoxylalkyl, $C_1$-$C_6$ alkynyl, $C_1$-$C_6$ alkoxy, $C_1$-$C_6$ hydroxylalkyl,

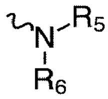

,

or phenyl, wherein said $C_1$-$C_6$ alkyl, $C_1$-$C_6$ alkenyl, $C_1$-$C_6$ alkoxylalkyl, $C_1$-$C_6$ alkynyl, $C_1$-$C_6$ alkoxy, and phenyl are optionally substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ perfluoroalkoxy,

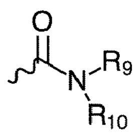

,

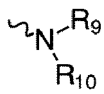

;

Or

R3 or R4, are a 5- or 6- membered cycloalkyl or heteroaryl that is substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

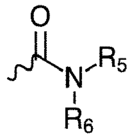 . 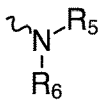 . 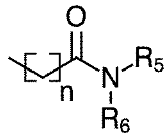

wherein n is 0, 1, or 2; or

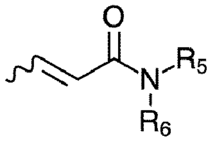 ;

R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;

R7 is -C, =C, O, S or N;

R9 and R10, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
or a salt thereof including a pharmaceutically acceptable salt thereof.

In another embodiment, the IRE-1A inhibitors are represented by formula (IV), wherein:

R1, R2 and R8 independently are H, =O, halogen, -OH, mono- or dialkylamino, $C_1$-$C_6$ alkenyl, $C_1$-$C_6$ alkynyl, phenyl, $C_1$-$C_6$ alkyl, or $C_1$-$C_6$ alkoxyl;

R3 or R4 are independently H, halogen, -OH, CN, -$CONH_2$, -$CON(CH_3)_2$, $C_1$-$C_6$ alkyl, $C_1$-$C_6$ alkoxy, $C_1$-$C_6$ hydroxylalkyl, $C_1$-$C_6$ alkoxylalkyl,

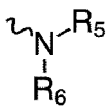 ,

$C_1$-$C_6$ alkenyl, $C_1$-$C_6$ alkynyl, or phenyl;
Or

R3 or R4 are a 5-or 6- membered heteroaryl that is substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

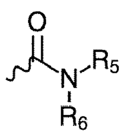 , 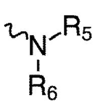 , 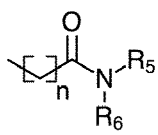

wherein n is 0, 1, or 2; or

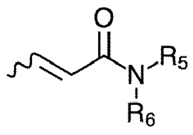 ;

R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;

R7 is =C or O;
or a salt thereof including a pharmaceutically acceptable salt thereof.

In a further embodiment, the IRE-1A inhibitors are represented by formula (IV), wherein:

R1, R2 and R8 independently are H or =O;
R3 or R4 are independently H or $C_1$-$C_6$ alkyl;
Or
R3 or R4 are a 5-or 6- membered heteroaryl that is substituted with one or more substituents independently selected from the group consisting of

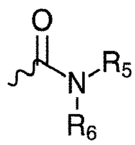 , 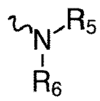 , 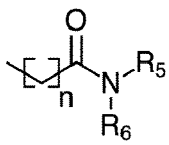

wherein n is 0, 1, or 2; or

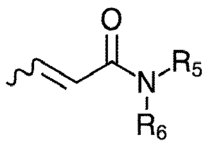 ;

R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R7 is =C or O;
or a salt thereof including a pharmaceutically acceptable salt thereof.

In yet a further embodiment, the IRE-1A inhibitors are represented by formula (IV), wherein:

R1, R2 and R8 independently are H or =O;
R3 and R4 are independently H or $C_1$-$C_6$ alkyl or a 5- or 6- membered heteroaryl that is substituted with one or more substituents independently selected from the group consisting of

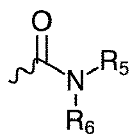 , 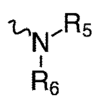 , 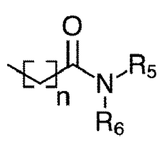

wherein n is 0, 1, or 2; or

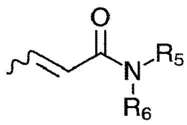

R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R7 is =C or O;
or a salt thereof including a pharmaceutically acceptable salt thereof.

In yet a further embodiment, the IRE-1A inhibitors are represented by formula (IV), wherein:

R1, R2 and R8 independently are H or =O;
R3 or R4 are independently H or $C_1$-$C_6$ alkyl or a 5-or 6- membered heteroaryl that is substituted with

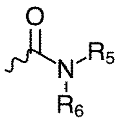

R5 and R6, together with the nitrogen atom to which they are attached, form a heterocycle containing one or more N heteroatoms, optionally substituted with one or more $C_1$-$C_6$ alkyl substituents;
R7 is =C or O;
or a salt thereof including a pharmaceutically acceptable salt thereof.

In another embodiment, the IRE-1A inhibitors are represented by formula (V),

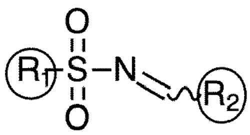

(V)

wherein:

R1 and R2 are a 5- or 6- membered cycloalkyl or heteroaryl that is optionally substituted with one or more substituents independently selected from the group consisting of halogen, -OH, - CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
or a salt thereof including a pharmaceutically acceptable salt thereof.

In another embodiment, the IRE-1A inhibitors are represented by formula (V), wherein:

R1 is a heteroaryl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
R2 is an aryl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
or a salt thereof including a pharmaceutically acceptable salt thereof.

In a further embodiment, the IRE-1A inhibitors are represented by formula (V), wherein:

R1 is a thiophene that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; more in particular R1 is thiophene.

R2 is a naphthalene that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl; more in particular R2 is naphthalene substituted with -OH;

or a salt thereof including a pharmaceutically acceptable salt thereof.

In another embodiment, the IRE-1A inhibitors are represented by the following formula (VI),

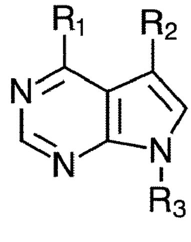

(VI)

wherein:

R1 is $-NH_2$, halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;

R2 is -H, -OH, -CN, -COOH, $-C(O)NH_2$, $-C(S)NH_2$, $-C(NH)NH_2$,

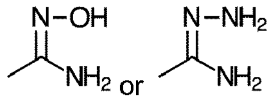

or ;

and

R3 is a 5- or 6- membered cycloalkyl, heterocycloalkyl or heteroaryl that is optionally substituted with one or more substituents independently selected from the group consisting of halogen, - OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;

or a salt thereof including a pharmaceutically acceptable salt thereof.

In another embodiment, the IRE-1A inhibitors are represented by formula (VI), wherein:

R1 is $-NH_2$ or -OH;

R2 is -H or -CN; and

R3 is a 5- membered heterocycloalkyl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; in particular R3 is a pentose; more in particular R3 is ribofuranose, ribopyranose, arabinofuranose, arabinopyranose, xylopyranose, or lyxopyranose; even more in particular R3 is ribofuranose;

or a salt thereof including a pharmaceutically acceptable salt thereof.

In a further embodiment, the IRE-1A inhibitors are represented by formula (VI), wherein

R1 is $-NH_2$ or -OH;

R2 is -H or -CN; and

R3 is β-D-ribofuranose or 2',3'-dideoxy- β-D-ribofuranose; more in particular β-D-ribofuranose;

or a salt thereof including a pharmaceutically acceptable salt thereof.

In another embodiment, the IRE-1A inhibitors are represented by the following formula (VII),

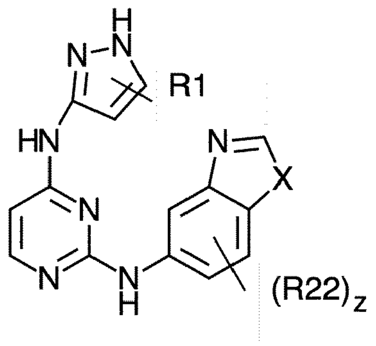

(VII)

wherein

R1 and R22 are independently -H, halogen, -CN, -NO, -$NO_2$, -NR9R10, -OR11, -COOR12, - SR13, -COR14, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
R9, R10, R11, R12, R13, and R14 are independently -H, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
X is NH or S; and z is an integer from 0 to 5;
or a salt thereof including a pharmaceutically acceptable salt thereof.

In another embodiment, the IRE-1A inhibitors are represented by formula (VII), wherein

R1 is -H, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
X is NH; and z is an integer with value 0;
or a salt thereof including a pharmaceutically acceptable salt thereof.

In a further embodiment, the IRE-1A inhibitors are represented by formula (VII), wherein

R1 is a $C_1$-$C_6$ cycloalkyl; more in particular R1 is cyclopropyl;
X is NH; and z is an integer with value 0.

In another embodiment, the IRE-1A inhibitors are represented by the following formula (VIII),

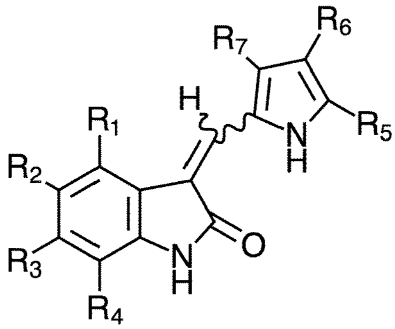

(VIII)

wherein:

R1, R3, R4, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
R2 and R16 are selected from the group consisting of -H, halo, $C_1$-$C_6$ alkyl, trihalomethyl, -OH, $C_1$-$C_6$ alkoxy, -NR13R14, -NR13C(O)R14, -C(O)R15, aryl, heteroaryl, and $-S(O)_2NR13R14$; R6 is -C(O)R10;
R10 is $-N(R11)(CH_2)_nR16$;
R11 is selected from the group consisting of -H and $C_1$-$C_6$ alkyl;
R13 and R14 are independently selected from the group consisting of -H, $C_1$-$C_6$ alkyl, $C_1$-$C_6$ cycloalkyl, aryl and heteroaryl; or R13 and R14, together with the nitrogen or carbon atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R15 is selected from the group consisting of -H, -OH, $C_1$-$C_6$ alkoxy and aryloxy; and
n is an integer from 0, 1, 2, 3, or 4;
or a salt thereof including a pharmaceutically acceptable salt thereof.

In another embodiment, the IRE-1A inhibitors are represented by formula (VIII), wherein:

R1, R3, R4 and R11 are -H;
R2 is halo;
R5 and R7 are independently selected from the group consisting of -H and $C_1$-$C_6$ alkyl;
R6 is -C(O)R10;
R10 is $-N(R11)(CH_2)_nR16$;
R13 and R14 are independently selected from the group consisting of -H, $C_1$-$C_6$ alkyl, $C_1$-$C_6$ cycloalkyl, aryl and heteroaryl; or R13 and R14, together with the nitrogen or carbon atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R16 is -NR13R14; and
n is an integer from 0, 1, 2, 3, or 4;
or a salt thereof including a pharmaceutically acceptable salt thereof.

In a further embodiment, the IRE-1A inhibitors are represented by formula (VIII), wherein:

R1, R3, R4 and R11 are -H;
R2 is halo; more in particular R2 is F;
R5 and R7 are $C_1$-$C_6$ alkyl; more in particular R5 and R7 are methyl;
R6 is -C(O)R10;
R10 is $-N(R11)(CH_2)_nR16$;
R13 and R14 are $C_1$-$C_6$ alkyl; more in particular R13 and R14 are ethyl;
R16 is -NR13R14; and
n is 2;

or a salt thereof including a pharmaceutically acceptable salt thereof.

In another embodiment, the IRE-1A inhibitors are represented by the following formula (IX),

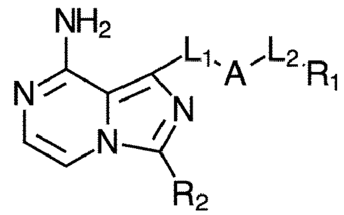

(IX)

wherein:

A is an optionally substituted $C_1$-$C_6$ cycloalkylene, optionally substituted $C_1$-$C_6$ heterocycloalkylene, optionally substituted arylene, or optionally substituted heteroarylene with one or more substituents independently selected from the group consisting of halogen, $-CF_3$,-OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl,
$C_1$-$C_6$ alkoxylalkyl, $C_1$-$C_6$ perfluoroalkoxyl or aryl;
L1 is a bond or unsubstituted $C_1$-$C_6$ alkylene;
L2 is a bond, -NR6-, -NR6C(O)-, -C(O)NR6-, -NR6C(O)O-, -NR6C(O)NR6-;
R1 is -H, oxo, halogen, $-CX_3$, -CN, $-SO_2Cl$, $-SO_nR10$, $-SO_vNR7R8$, $-NHNH_2$, -ONR7R8,-NHC=(O)$NHNH_2$, -NHC=(O)NR7R8, $-N(O)_m$, -NR7R8, -C(O)R9, -C(O)-OR9, -C(O)NR7R8,-OR10, $-NR7SO_nR10$, -NR7C=(O)R9, -NR7C(O)OR9, -NR7OR9, $-OCX_3$, $-OCHX_2$, $C_1$-$C_6$ alkyl, $C_1$-$C_6$ heteroalkyl, $C_1$-$C_6$ cycloalkyl, $C_1$-$C_6$ heterocycloalkyl, optionally substituted aryl, or optionally substituted heteroaryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
R2 is -H, -CN, -OH, halogen or $C_1$-$C_6$ alkyl;
R6, R7, R8, R9 and R10 are independently selected from -H, halogen, $-CF_3$, -CN, -OH, $-NH_2$,-COOH, $-CONH_2$, $-NO_2$, -SH, $-SO_2Cl$, $-SO_3H$, $-SO_4H$, $-SO_2NH_2$, $-NHNH_2$, $-ONH_2$,-NHC=(O)$NHNH_2$, -NHC=(O)$NH_2$, $-NHSO_2H$, -NHC=(O)H, -NHC(O)OH, -NHOH, $-OCF_3$,$-OCHF_2$, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
each occurrence of the symbol n is independently an integer from 0 to 4; each occurrence of the symbol m and v is independently an integer from 1 to 2; each occurrence of the symbol X is independently a halogen;
or a salt thereof including a pharmaceutically acceptable salt thereof.

In another embodiment, the IRE-1A inhibitors are represented by formula (IX),
wherein:

A is an optionally substituted arylene or optionally substituted heteroarylene with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
L1 is a bond;
L2 is a -NR6C(O)NR6-;
R1 is -H, $C_1$-$C_6$ alkyl, $C_1$-$C_6$ heteroalkyl, $C_1$-$C_6$ cycloalkyl, $C_1$-$C_6$ heterocycloalkyl, optionally substituted aryl, or optionally substituted heteroaryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl; R2 is $C_1$-$C_6$ alkyl;
R6 is -H, halogen, $-CF_3$, -CN, -OH, $-NH_2$, -COOH, $-CONH_2$, $-NO_2$, -SH, $-SO_2Cl$, $-SO_3H$, $-SO_4H$, $-SO_2NH_2$, $-NHNH_2$, $-ONH_2$, -NHC=(O)$NHNH_2$, -NHC=(O)$NH_2$, $-NHSO_2H$, -NHC=(O)H,-NHC(O)OH, -NHOH, $-OCF_3$, $-OCHF_2$;
or a salt thereof including a pharmaceutically acceptable salt thereof.

In a further embodiment, the IRE-1A inhibitors are represented by formula (IX),
wherein:

A is an arylene substituted with an aryl; more in particular A is naphthalene;
L1 is a bond;
L2 is a -NR6C(O)NR6-;
R1 is an aryl substituted with $-CF_3$;
R2 is $C_1$-$C_6$ alkyl; more in particular R2 is $C_4H_9$;
R6 is -H;
or a salt thereof including a pharmaceutically acceptable salt thereof.

In a preferred embodiment, the IRE-1A inhibitors are selected from:

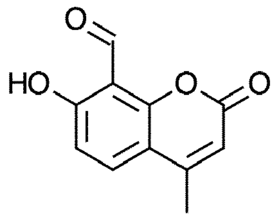

(4μ8c)

or

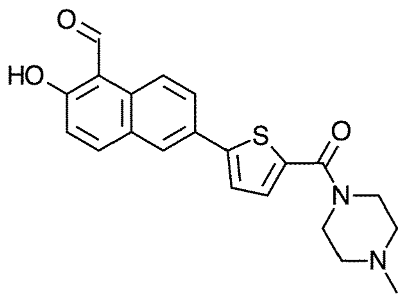

(MKC-3946)

or

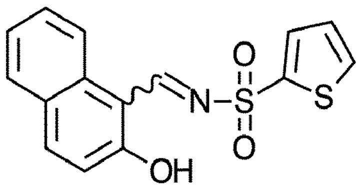

(STF-083010)

or

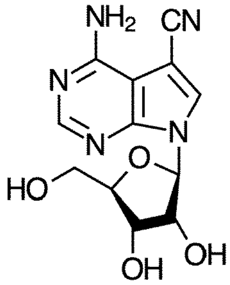

(toyocamycin)

or

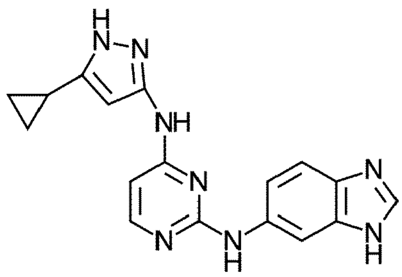

(APY29)

or

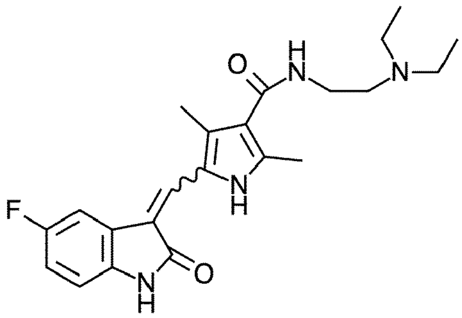

(Sunitinib)

or

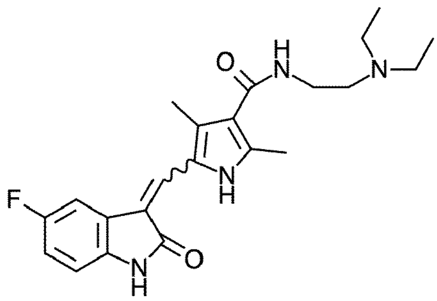

* $C_4H_6O_5$

(Sunitinib malate)

or

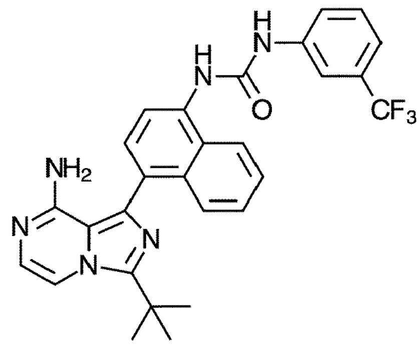

(KIRA-6)

or

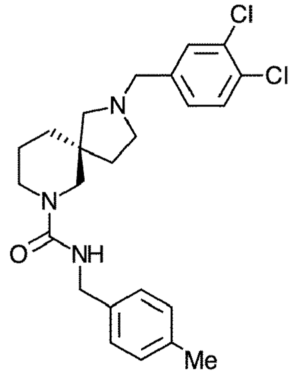

(GSK2850163)

or a salt thereof including a pharmaceutically acceptable salt thereof. Furthermore, IRE-1A inhibitors may be:

- Salicylaldimine analogs
- Mycotoxins
- (Poly)phenol- or flavonoid derived compounds, such as flavonol quercetin, kaempferol, apigenin and resveratrol

As mentioned here above, the invention relates to UPR pathway inhibitors, such as IRE-1A inhibitors for use in the prevention or treatment of endoplasmic reticulum (ER) stress disorders, in particular disorders originating from disturbances in the development of the fetal nervous system; more in particular neurodevelopmental disorders, such as microcephaly caused by ZIKV.

In a certain embodiment, the endoplasmic reticulum (ER) stress disorders are neurodevelopmental disorders related to an endoplasmic reticulum (ER) stress disorder.

In another embodiment, the neurodevelopmental disorders related to an endoplasmic reticulum (ER) stress disorder comprise but are not limited to microcephaly.

In a more specific embodiment, the neurodevelopmental disorders related to an endoplasmic reticulum (ER) stress disorder are caused by a viral infection in a fetal phase.

In an even more specific embodiment, the neurodevelopmental disorders related to an endoplasmic reticulum (ER) stress disorder are caused by Zika virus (ZIKV); more in particular by a Zika viral infection in a fetal phase.

Last, in a certain embodiment, the IRE-1A inhibitors are developed for in-vitro research.

Furthermore, the IRE-1A inhibitors may be linked to the PERK inhibition pathway as well. In that respect, the IRE-1A inhibitors as indicated above may combine with the PERK inhibitors as indicated above, for use in the prevention or treatment of endoplasmic reticulum (ER) stress disorders, in particular disorders originating from disturbances in the development of the fetal nervous system; more in particular neurodevelopmental disorders, such as microcephaly caused by ZIKV.

## BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

**Fig. 1: *ZIKV induced ER stress and UPR in human cortices in vivo and human neural stem cells in vitro.* A-F, histograms showing fold change of *MX1* (A), *ATF4* (B), *ATF5* (C), *CHAC1* (D), *CHOP* (E), Protein Disulphide Isomerase (PDI, F) transcripts in occipital cortex from three control fetuses (WT value is normalized to 1 and corresponds to three uninfected fetuses of 21GW, 23GW, or 24GW) and three fetuses infected by ZIKV during pregnancy (ZIKV#1, 25GW; ZIKV#2 and ZIKV#3, 22GW; average number of ZIKV copies/μg of extracted RNA = 2,50 x $10^6$ ± 1,27 x $10^6$; Mean ± SEM).**

**G-O, qRT-PCR performed on total RNA extract from Mock or ZIKV-infected hNSCs (average number of Zika copies/μg of extracted RNA = 1,10 x $10^9$ ± 3,28 x $10^8$; Mean ± SEM) to detect MX1 (G), PDI (H), ATF3 (I), CHAC1 (J), CHOP (K), SENS2 (L), HRD1 (M), Xbp1 S/U (N) and ZIKV (O). Data presented as mean ± SEM,**

**Student's t test, ***p<0.001, **p<0.01 and *p<0.05.**
**P, Model of cortical neurogenesis.**

**Fig. 2: *Intracerebroventricular injection of ZIKV induces microcephaly in mouse embryos with activation of ER stress and UPR.***
**A-D, qRT-PCR related histogram comparing morphological parameters between Mock or ZIKV-infected E18.5 cortical extracts and showing brain weight (A), normalized brain width (B), length (C) and cortical thickness (D).**
**E-H, qRT-PCR related assays performed on total RNA extract from Mock or ZIKV-infected E18.5 cortices to detect the progenitor markers *Pax6* and *Tbr2* (E, F) and the neuronal markers *NeuroD6* and *Tubb3* (G, H).**
**I-P, qRT-PCR performed on total RNA extracts from Mock or ZIKV-infected E18.5. cortices to detect the following ER stress or UPR actors: PDI (I), Atf4 (J), Atf3 (K), Atf5 (L), Chop (M), Slc7a3 (N), Xbp1 S/U (O) and Chac1 (P).**
**Data presented as mean ± SEM, Student's t test (A-D) and Mann-Whitney (E-O), ***p<0.001, **p<0.01 and *p<0.05.**
**Q-V, *Ifnr*$^{-/-}$ pregnant dams were injected with ZIKV particles intraperitoneally at gestational day 9.5 and embryos were collected at E14.5. Microdissected cortices were analysed by qRT-PCR to detect ZIKV (Q), PDI (R), Atf4 (S, Atf5 (T), Chac1 (U), and Chop1 (V); representative experiment for each condition.**

**Fig. 3: *Intracerebroventricular injection of ZIKV promotes direct neurogenesis at the expense of indirect neurogenesis in mouse cortices in vivo.***
**A-B, experimental strategy (A) to track the first generation of daughter cells of *in utero* targeted apical progenitors (AP) by ZIKV and electroporation (B).**
**C-F, assessment of the neurogenic balances in E14.5 mouse cortices upon Mock treatment or ZIKV infection with or without GSK2656157 (PERK inhibitor or PERKi). Percentages of cells expressing either Sox2, Tbr2 or both in GFP-labeled cells (C, E). Percentages of cells expressing either Tbr2, Tbr1 or both in GFP-labeled cells at E14.5 (D, F).**
**The number of analysed brains is 5 per condition (C-D: 4 Mock, 3 ZIKV; E-F: 5 ZIKV-PERKi, 5 ZIKV- DMSO, 4 Mock-PERKi, 4 Mock-DMSO)**
**G, Assessment of proliferation (Ki67+) rate of GFP+ apical progenitors in Mock condition or after ZIKV infection in mouse cortices.**
**H, Histogram representing cycling progenitors that express Ki67 (and expressed as percentage of control) in three different experimental conditions, as mentioned on the graph.**
**Data presented as mean ± SEM, ANOVA2, Bonferroni post-hoc tests, ***p<0.001, **p<0.01, and *p<0.05.**

**Fig. 4: *Intracerebroventricular (ICV) and intraplacental (IPL) injection of ZIKV, but not Yellow Fever (YFV) or West Nile (WNV) viruses, induces microcephaly and cell death in mouse brains.***
**A, histogram comparing the percentage of cells that co-express ZIKV and ac-caspase 3 after Mock-treatment, ZIKV-infection or co-administration of ZIKV with PERK inhibitor (PERKi) at E12.5 (6, 7, and 7 brains from separate litters per condition, respectively)**
**B-D, Histograms comparing brain weight (B), normalized brain width (C) and length (D) of E18.5 brains after injection of Mock, ZIKV, ZIKV and PERKi, or ZIKV and IRE-1 inhibitor (IRE-1i) at E12.5 (10, 9, 10, and 12 brains from separate litters per condition, respectively).**
**E, qRT-PCR performed on total RNA extract from placentas of embryos injected with ZIKV, YFV17D or WNV to detect expression of respective flaviviruses.**
**F-H, Histograms comparing brain weight (F), normalized brain width (G) and length (H) of E18.5 brains after intraplacental injection with ZIKV, YFV17D or WNV at E12.5 (13, 12, and 13 brains from separate litters per condition, respectively).**
**I, Schematic representation of mouse cerebral cortex. The black and gray double-head arrows show, respectively, the width and length measurements performed on ZIKV- and/or UPR inhibitors- and/or Mock-treated cerebral cortices.**
**J, Histogram showing the percentage of cells co-expressing (or not) ZIKV and ac-caspase 3 in upper regions (bins from 1 to 5) and lower regions (bins from 6 to 10) of E14.5 cortices after ZIKV infection at E12.5.**
**Scale bars represent 50μm (A). Data presented as mean ± SEM, One-way ANOVA followed by Bonferroni post-hoc tests, ***p<0.001, **p<0.01, and *p<0.05.**

**Fig. 5: *Assessment of the neurogenic balances upon Cre-GFP electroporation of Elp3 lox/lox embryos at E13.5 with or without ISRIB (DMSO used as vehicle control). Percentages* of *cells expressing either Tbr1,***

***Tbr2 or both in GFP-labeled cells at E14.5, whereby Tbr1-Tbr2- are apical progenitors, Tbr2+ are intermediate progenitors and Tbr2-Tbr1+ are Neurons. One representative experiment.***

**Fig. 6: Quantitative measurement of changes in Zika replication and UPR effectors levels in E18.5 brains *intracerebroventricularly injected at E12.5 with ZIKV + DMSO or PERK inhibitors (Salubrinal or ISRIB).***
**A, Absolute quantification of ZIKV (PERK-i) for ZIKV + DMSO, ZIKV + Salubrinal 2mM, ZIKV + Salubrinal 10mM, ZIKV + ISRIB 0.4mM and ZIKV + ISRIB 2mM;**
**B, Fold change of *mAtf4* for ZIKV + DMSO, ZIKV + ISRIB 0.4mM and ZIKV + ISRIB 10mM (left) and ZIKV + DMSO, ZIKV + Salubrinal 2mM and ZIKV + Salubrinal 10mM (right);**
**C, Fold change of *mAtf5* for ZIKV + DMSO, ZIKV + ISRIB 0.4mM and ZIKV + ISRIB 10mM (left) and ZIKV + DMSO, ZIKV + Salubrinal 2mM and ZIKV + Salubrinal 10mM (right);**
**D, Fold change of *mChac1* for ZIKV + DMSO, ZIKV + ISRIB 0.4mM and ZIKV + ISRIB 10mM (left) and ZIKV + DMSO, ZIKV + Salubrinal 2mM and ZIKV + Salubrinal 10mM (right);**

**Fig. 7: Quantitative measurement of changes in Zika replication and UPR effectors levels in E18.5 brains *intracerebroventricularly injected at E12.5 with ZIKV + DMSO or Resveratrol (a IRE1a inhibitor).***
**A, Absolute quantification of ZIKV (IRE1-i) for ZIKV + DMSO, ZIKV + Resveratrol 2mM, ZIKV + Resveratrol 10mM;**
**B, Fold change of *mAtf4* for ZIKV + DMSO, ZIKV + Resveratrol 5mM and ZIKV + Resveratrol 10mM;**
**C, Fold change of *mAtf3* for ZIKV + DMSO, ZIKV + Resveratrol 5mM and ZIKV + Resveratrol 10mM;**
**D, Fold change *of Xbp1 S/U* (left) for ZIKV + DMSO, ZIKV + Resveratrol 5mM and ZIKV + Resveratrol 10mM and fold change of *Xbp1 Tot* (right) for ZIKV + DMSO, ZIKV + Resveratrol 5mM and ZIKV + Resveratrol 10mM;**
**E, Fold change of *mChop* for ZIKV + DMSO, ZIKV + Resveratrol 5mM and ZIKV + Resveratrol 10mM;**

## DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In one embodiment the UPR pathway inhibitors used in the methods according to the invention consist of PERK inhibitors.

In a particular embodiment, the PERK inhibitors are represented by the following formula (I),

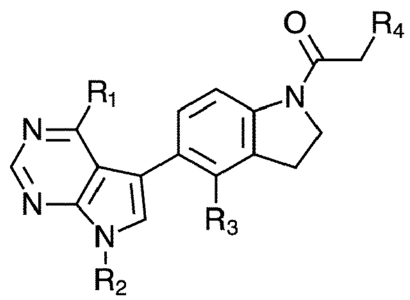

(I)

wherein

R1, R2 and R3 are independently selected from H, $C_{1-6}$alkyl, amino or halo; and
R4 is selected from:
Aryl;
aryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$ and-CN;
heteroaryl;
heteroaryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$, -CN and cyclo$C_{3-6}$alkyl, and;
cyclo$C_{3-6}$alkyl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$ and -CN;
or a salt thereof including a pharmaceutically acceptable salt thereof.

Particular embodiments of the PERK inhibitors of formula (I), are those wherein one or more of the following restrictions apply;

- R1, R2 and R3 are independently selected from H, $C_{1-6}$alkyl, amino or halo;
- R1 is an amino and R2 and R3 are independently selected from H, $C_{1-6}$alkyl, amino or halo;
- R3 is halo and R1 and R2 are independently selected from H, $C_{1-6}$alkyl, amino or halo;
- R2 is a $C_{1-6}$alkyl; and R1 and R3 are independently selected from H, $C_{1-6}$alkyl, amino or halo;
- R4 is heteroaryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$, -CN, and cyclo$C_{3-6}$alkyl;
- R4 is heteroaryl substituted with one substituent independently selected from $C_{1-6}$alkyl, $C_{1-6}$alkyloxy, $-C_{1-6}$alkylO$C_{1-6}$alkyl, - $CF_3$, and cyclo$C_{3-6}$alkyl;
- R4 is heteroaryl substituted with one substituent independently selected from $C_{1-6}$alkyl, $C_{1-6}$alkyloxy, $-C_{1-6}$alkylO$C_{1-6}$alkyl, and - $CF_3$;
- R4 is heteroaryl substituted with one substituent independently selected from $C_{1-6}$alkyl, $C_{1-6}$alkyloxy, and - $CF_3$;
- R4 is heteroaryl substituted with one $C_{1-6}$alkyl; in particular said $C_{1-6}$alkyl being in meta position;
- R4 is pyridinyl substituted with one substituent independently selected from $C_{1-6}$alkyl, $C_{1-6}$alkyloxy, $-C_{1-6}$alkylO$C_{1-6}$alkyl, - $CF_3$, and cyclo$C_{3-6}$alkyl;
- R4 is pyridinyl substituted with one substituent independently selected from $C_{1-6}$alkyl, $C_{1-6}$alkyloxy, $-C_{1-6}$alkylO$C_{1-6}$alkyl, and - $CF_3$;
- R4 is pyridinyl substituted with one substituent independently selected from $C_{1-6}$alkyl, $C_{1-6}$alkyloxy, and - $CF_3$;
- R4 is pyridinyl substituted with one $C_{1-6}$alkyl; in particular said $C_{1-6}$alkyl being in meta position.

In one embodiment, the PERK inhibitors of formula (I), are those wherein;

R1 is an amino;
R2 is an $C_{1-6}$alkyl;
R3 is a halo; and
R4 is a heteroaryl substituted with from one to five substituents independently selected from: fluoro, chloro, bromo, iodo, $C_{1-6}$alkyl, $C_{1-6}$alkyloxy, -OH, -COOH, $-CF_3$, $-C_{1-6}$alkylO$C_{1-6}$alkyl, $-NO_2$, $-NH_2$, -CN, and cyclo$C_{3-6}$alkyl; in particular R4 is pyridinyl substituted with one substituent independently selected from $C_{1-6}$alkyl, $C_{1-6}$alkyloxy, $-C_{1-6}$alkylO$C_{1-6}$alkyl, $-CF_3$, and cyclo$C_{3-6}$alkyl; more in particular R4 is pyridinyl substituted with one substituent independently selected from $C_{1-6}$alkyl, $C_{1-6}$alkyloxy, and - $CF_3$; even more in particular R4 is pyridinyl substituted with one $C_{1-6}$alkyl;
or a salt thereof including a pharmaceutically acceptable salt thereof.

In another embodiment, the PERK inhibitors are represented by the following formula (II),

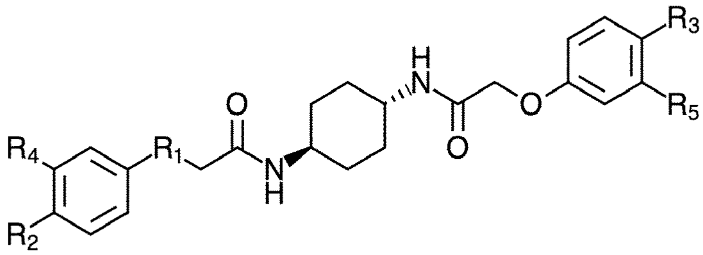

(II)

wherein

R1 is -O- or -NH-; and
R2, R3, R4 and R5 are independently selected from H, halo, $-CF_3$, $C_{1-6}$alkyl, -CN or $C_{1-6}$alkoxy;
or a salt thereof including a pharmaceutically acceptable salt thereof.

This structural formula is linked to a group of compounds, named bis-O-arylglycolamides.

Particular embodiments of the PERK inhibitors of formula (II), wherein one or more of the following restrictions apply;

- R1 is -O- or -NH-; in particular R1 is -O-;

- R2, R3, R4 and R5 are each independently selected from H, halo, or $-CF_3$;
- R2, R3, R4 and R5 are each independently selected from H, or halo;
- R2 and R3 are H; and R4 and R5 are each independently selected from H, halo, $-CF_3$, $C_{1-6}$alkyl, -CN or $C_{1-6}$alkoxy;
- R2 and R3 are independently selected from H, halo, $-CF_3$, $C_{1-6}$alkyl, -CN or $C_{1-6}$alkoxy; and R4 and R5 are H;
- R2 and R3 are H; and R4 and R5 are each an independently selected halo; in particular chloro;
- R2 and R3 are each an independently selected halo; in particular chloro; and R4 and R5 are H.

In another embodiment, the PERK inhibitors are represented by the following formula (III),

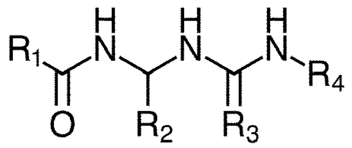

(III)

wherein

R1 and R4 are each independently selected from the group consisting of H, $C_{1-6}$alkyl, $C_{1-6}$alkenyl, $C_{1-6}$alkynyl, $C_{1-6}$alkoxy, $C_{1-6}$alkenoxy, $C_{1-6}$alkynoxy, thio$C_{1-6}$alkoxy, hydroxy$C_{1-6}$alkyl, aliphatic $C_{1-6}$acyl, $-CF_3$, carboxy, $-C_{1-6}$alkylamino, $C_{1-6}$alkenylamino, $C_{1-6}$alkynylamino, di($C_{1-6}$alkyl)amino,-C(O)O-($C_{1-6}$alkyl), -C(O)NH-($C_{1-6}$alkyl), -C(O)N($C_{1-6}$alkyl)$_2$, halo$C_{1-6}$alkyl, $C_{1-6}$alkoxy$C_{1-6}$alkoxy, carboxaldehyde, carboxamide, cyclo$C_{3-6}$alkyl, cyclo$C_{3-6}$alkenyl, cyclo$C_{3-6}$alkynyl, cyclo$C_{3-6}$alkyl$C_{1-6}$alkyl, aryl, aroyl, aryloxy, arylamino, biaryl, thioaryl, diarylamino, heterocyclyl, $C_{1-6}$alkylaryl, aralkenyl, aralkyl, alkylheterocyclyl, heterocyclylalkyl, aryloxy$C_{1-6}$alkyl, carboxyl, carbamate and -C(O)NH(benzyl);
R2 is selected from H, halo or a $C_{1-6}$alkyl optionally substituted with one or more halo; and
R3 is selected from the group consisting of O, S and NR5;
wherein R1, R4 and R5 are unsubstituted or substituted with at least one electron donating or electron withdrawing group;
or a salt thereof including a pharmaceutically acceptable salt thereof.

Particular embodiments of the PERK inhibitors of formula (III) are those wherein one or more of the following restrictions apply;

- R1 and R4 are each independently selected from the group consisting of aryl, heterocyclyl, aralkenyl, aralkyl, and heterocyclylalkyl;
- R1 and R4 are each independently selected from the group consisting of aryl, and aralkenyl; in particular R1 is aryl and R4 is aralkenyl;
- R2 is selected from H, halo or a $C_{1-6}$alkyl optionally substituted with one or more halo, in particular chloro; and
- R3 is S;

In a preferred embodiment, the PERK inhibitors are selected from:

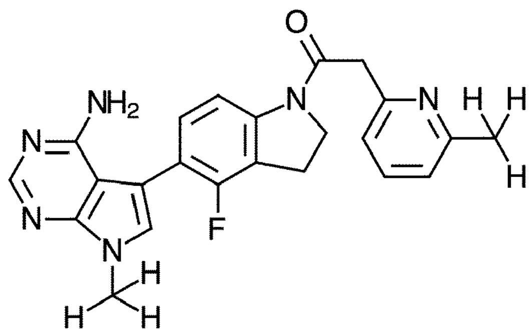

or

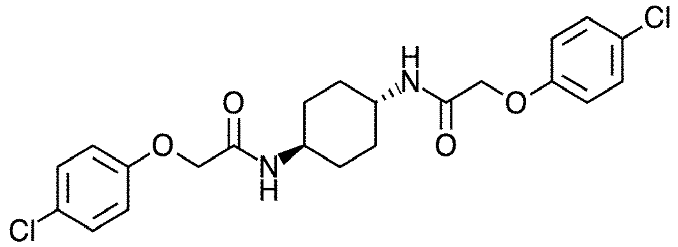

or

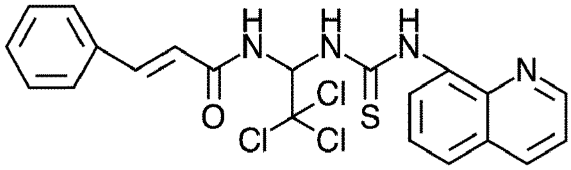

or a salt thereof including a pharmaceutically acceptable salt thereof.

In another embodiment the UPR pathway inhibitors used in the methods according to the invention consist of IRE-1A inhibitors.

In a particular embodiment, the IRE-1A inhibitors are represented by the following formula (IV),

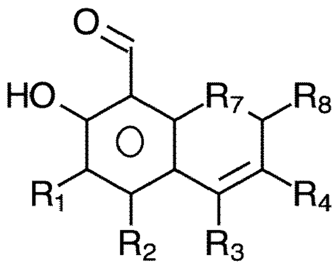

(IV)

wherein:

R1, R2 and R8 independently are H, =O, halogen, -OH, $C_1$-$C_6$ alkenyl, $C_1$-$C_6$ alkynyl, phenyl, optionally substituted $C_1$-$C_6$ alkyl, optionally substituted $C_1$-$C_6$ alkoxyl, or optionally substituted mono- or dialkylamino. Optional substituents for the $C_1$-$C_6$ alkyl, the $C_1$-$C_6$ alkoxyl or the mono- or dialkylamino are (1) a $C_1$-$C_6$ hydrocarbon chain containing a N or O atom and optionally substituted with halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, and (2) a cycloalkyl which may contain one or more heteroatoms selected from N, O, and S, and which is optionally substituted with halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl;

R3 or R4 are independently H, halogen, -OH, $C_1$-$C_6$ perfluoroalkyl, $C_1$-$C_6$ perfluoroalkoxy, -CN, $-CONH_2$, $-CON(CH_3)_2$, $C_1$-$C_6$ alkyl, $C_1$-$C_6$ alkenyl, $C_1$-$C_6$ alkoxylalkyl, $C_1$-$C_6$ alkynyl, $C_1$-$C_6$ alkoxy, $C_1$-$C_6$ hydroxylalkyl,

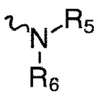,

or phenyl, wherein said $C_1$-$C_6$ alkyl, $C_1$-$C_6$ alkenyl, $C_1$-$C_6$ alkoxylalkyl, $C_1$-$C_6$ alkynyl, $C_1$-$C_6$ alkoxy, and phenyl are optionally substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ perfluoroalkoxy,

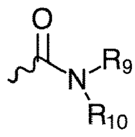,

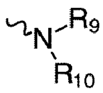;

Or

R3 or R4, are a 5-or 6- membered cycloalkyl or heteroaryl that is substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

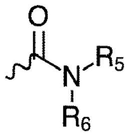, 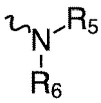, 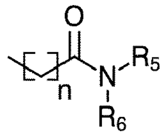

wherein n is 0, 1, or 2; or

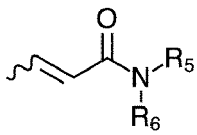

R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;

R7 is -C, =C, O, S or N;

R9 and R10, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
or a salt thereof including a pharmaceutically acceptable salt thereof.

Particular embodiments of the IRE-1A inhibitors of formula (IV), are those wherein one or more of the following restrictions apply;

- R1, R2 and R8 independently are H, =O, halogen, -OH, mono- or dialkylamino, $C_1$-$C_6$ alkenyl, $C_1$-$C_6$ alkynyl, phenyl, $C_1$-$C_6$ alkyl, or $C_1$-$C_6$ alkoxyl;
- R1 is -H and R2 and R8 independently are H, =O, halogen, -OH, mono- or dialkylamino, $C_1$-$C_6$ alkenyl, $C_1$-$C_6$ alkynyl, phenyl, $C_1$-$C_6$ alkyl, or $C_1$-$C_6$ alkoxyl;
- R2 is -H and R1 and R8 independently are H, =O, halogen, -OH, mono- or dialkylamino, $C_1$-$C_6$ alkenyl, $C_1$-$C_6$ alkynyl, phenyl, $C_1$-$C_6$ alkyl, or $C_1$-$C_6$ alkoxyl;
- R8 is =O and R1 and R2 independently are H, =O, halogen, -OH, mono- or dialkylamino, $C_1$-$C_6$ alkenyl, $C_1$-$C_6$ alkynyl, phenyl, $C_1$-$C_6$ alkyl, or $C_1$-$C_6$ alkoxyl;
- R8 is -H and R1 and R2 independently are H, =O, halogen, -OH, mono- or dialkylamino, $C_1$-$C_6$ alkenyl, $C_1$-$C_6$ alkynyl, phenyl, $C_1$-$C_6$ alkyl, or $C_1$-$C_6$ alkoxyl;
- R3 or R4 are independently H, halogen, -OH, CN, $-CONH_2$, $-CON(CH_3)_2$, $C_1$-$C_6$ alkyl, $C_1$-$C_6$ alkoxy, $C_1$-$C_6$ hydrox-

ylalkyl, $C_1$-$C_6$ alkoxylalkyl,

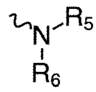,

$C_1$-$C_6$ alkenyl, $C_1$-$C_6$ alkynyl, or phenyl;

- R3 or R4 are a 5-or 6- membered heteroaryl that is substituted with one or more substituents independently selected from the group consisting of halogen, -OH,-CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

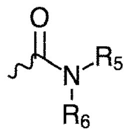, 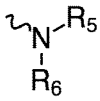, 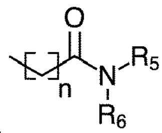

wherein n is 0, 1, or 2; or

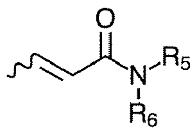;

- R3 is $C_1$-$C_6$ alkyl and R4 is H, halogen, -OH, CN, $-CONH_2$, $-CON(CH_3)_2$, $C_1$-$C_6$ alkyl, $C_1$-$C_6$ alkoxy, $C_1$-$C_6$ hydroxylalkyl, $C_1$-$C_6$ alkoxylalkyl,

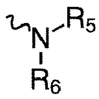,

$C_1$-$C_6$ alkenyl, $C_1$-$C_6$ alkynyl, or phenyl;

- R4 is H and R3 is H, halogen, -OH, CN, $-CONH_2$, $-CON(CH_3)_2$, $C_1$-$C_6$ alkyl, $C_1$-$C_6$ alkoxy, $C_1$-$C_6$ hydroxylalkyl, $C_1$-$C_6$ alkoxylalkyl,

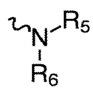,

$C_1$-$C_6$ alkenyl, $C_1$-$C_6$ alkynyl, or phenyl;

- R3 is -H and R4 is a 5-or 6- membered heteroaryl that is substituted with one or more substituents independently selected from the group consisting of halogen,-OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

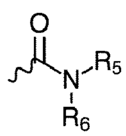, 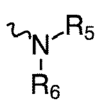, 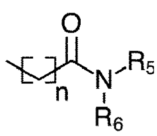

wherein n is 0, 1, or 2; or

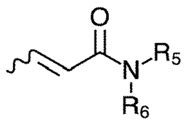;

- R4 is a 5-or 6- membered heteroaryl that is substituted with

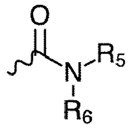

and R3 is H, halogen, -OH, CN, $-CONH_2$, $-CON(CH_3)_2$, $C_1$-$C_6$ alkyl, $C_1$-$C_6$ alkoxy, $C_1$-$C_6$ hydroxylalkyl, $C_1$-$C_6$ alkoxylalkyl,

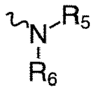,

$C_1$-$C_6$ alkenyl, $C_1$-$C_6$ alkynyl, or phenyl; or R3 is a 5-or 6- membered heteroaryl that is substituted with one or more substituents independently selected from the group consisting of halogen, -OH,-CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

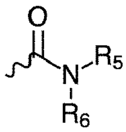, 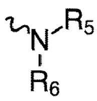, 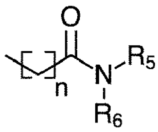

wherein n is 0, 1, or 2; or

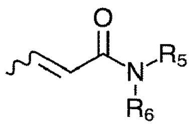;

- R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2,-CON(CH3)2, $C_1$-$C_6$ alkyl, $C_1$-$C_6$ alkoxy, $C_1$-$C_6$ hydroxylalkyl, $C_1$-$C_6$ alkoxylalkyl;
- R7 is =C or O;

In yet a further embodiment, the IRE-1A inhibitors are represented by formula (IV), wherein:

R1, R2 and R8 independently are H or =O;

R3 or R4 are independently H or $C_1$-$C_6$ alkyl or a 5-or 6- membered heteroaryl that is substituted with

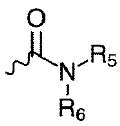;

R5 and R6, together with the nitrogen atom to which they are attached, form a heterocycle containing one or more

N heteroatoms, optionally substituted with one or more $C_1$-$C_6$ alkyl substituents;
R7 is =C or O;
or a salt thereof including a pharmaceutically acceptable salt thereof.

In another embodiment, the IRE-1A inhibitors are represented by the following formula (V),

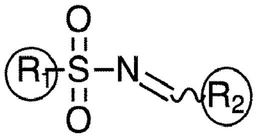
(V)

wherein:
R1 and R2 are a 5- or 6- membered cycloalkyl or heteroaryl that is optionally substituted with one or more substituents independently selected from the group consisting of halogen, -OH, - CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
or a salt thereof including a pharmaceutically acceptable salt thereof.

Particular embodiments of the IRE-1A inhibitors of formula (V), are those wherein one or more of the following restrictions apply;

- R1 is a heteroaryl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl, and R2 is a 5- or 6- membered cycloalkyl or heteroaryl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
- R2 is an aryl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl, and R1 is a 5- or 6- membered cycloalkyl or heteroaryl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;

In a further embodiment, the IRE-1A inhibitors are represented by formula (V), wherein:

R1 is a thiophene that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; more in particular R1 is thiophene.
R2 is a naphthalene that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl; more in particular R2 is naphthalene substituted with -OH;
or a salt thereof including a pharmaceutically acceptable salt thereof.

In another embodiment, the IRE-1A inhibitors are represented by the following formula (VI),

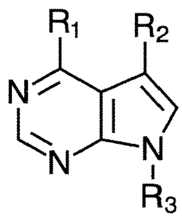
(VI)

wherein:

R1 is $-NH_2$, halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;

R2 is -H, -OH, -CN, -COOH, $-C(O)NH_2$, $-C(S)NH_2$, $-C(NH)NH_2$,

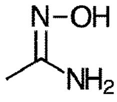 or 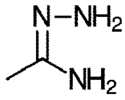 ;

and

R3 is a 5- or 6- membered cycloalkyl, heterocycloalkyl or heteroaryl that is optionally substituted with one or more substituents independently selected from the group consisting of halogen,-OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
or a salt thereof including a pharmaceutically acceptable salt thereof.

Particular embodiments of the IRE-1A inhibitors of formula (VI), are those wherein one or more of the following restrictions apply;

- R1 is $NH_2$ and R2 is -H, -OH, -CN, -COOH, $-C(O)NH_2$, $-C(S)NH_2$, $-C(NH)NH_2$,

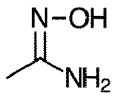 or 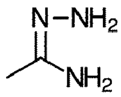 ;

- R1 is -OH and R2 is -H, -OH, -CN, -COOH, $-C(O)NH_2$, $-C(S)NH_2$, $-C(NH)NH_2$,

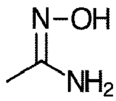 or 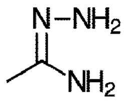 ;

- R2 is -CN and R1 is $-NH_2$, halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
- R3 is a 5- membered heterocycloalkyl that is optionally substituted with one or more substituents independently selected from the group consisting of halogen, -OH,-CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
- R3 is a pentose; and R1 is $-NH_2$, halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl; and R2 is -H, -OH, -CN, -COOH, $-C(O)NH_2$, $-C(S)NH_2$, $-C(NH)NH_2$,

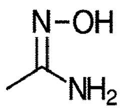 or 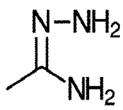 ;

In a further embodiment, the IRE-1A inhibitors are represented by formula (VI), wherein:

R1 is $-NH_2$ or -OH;
R2 is -H or -CN; and
R3 is β-D-ribofuranose or 2',3'-dideoxy- β-D-ribofuranose; more in particular β-D-ribofuranose; or a salt thereof including a pharmaceutically acceptable salt thereof.

In another embodiment, the IRE-1A inhibitors are represented by the following formula (VII),

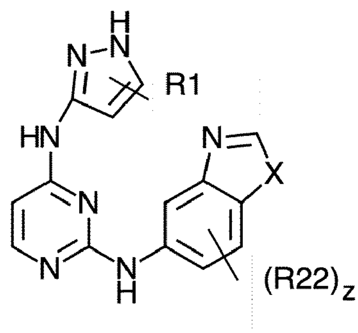

(VII)

wherein

R1 and R22 are independently -H, halogen, -CN, -NO, -$NO_2$, -NR9R10, -OR11, -COOR12, - SR13, -COR14, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
R9, R10, R11, R12, R13, and R14 are independently -H, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
X is NH or S; and z is an integer from 0 to 5;
or a salt thereof including a pharmaceutically acceptable salt thereof.

Particular embodiments of the IRE-1A inhibitors of formula (VII), are those wherein one or more of the following restrictions apply;

- R1 is a $C_1$-$C_6$ cycloalkyl and R22 is -H, halogen, -CN, -NO, -$NO_2$, -NR9R10, -OR11, -COOR12, -SR13, -COR14, optionally substituted $C_1$-$C_6$alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; wherein R9, R10, R11, R12, R13, and R14 are independently -H, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; X is NH or S; and z is an integer from 0 to 5.
- z is 0 and R1 is -H, halogen, -CN, -NO, -$NO_2$, -NR9R10, -OR11, -COOR12, -SR13, -COR14, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, - CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; wherein R9, R10, R11, R12, R13, and R14 are independently -H, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; X is NH or S
- z is 0, R1 is $C_1$-$C_6$ cycloalkyl and X is NH or S

In a further embodiment, the IRE-1A inhibitors are represented by formula (VII), wherein

R1 is a $C_1$-$C_6$ cycloalkyl; more in particular R1 is cyclopropyl;
X is NH; and z is an integer with value 0;

or a salt thereof including a pharmaceutically acceptable salt thereof.

In another embodiment, the IRE-1A inhibitors are represented by the following formula (VIII),

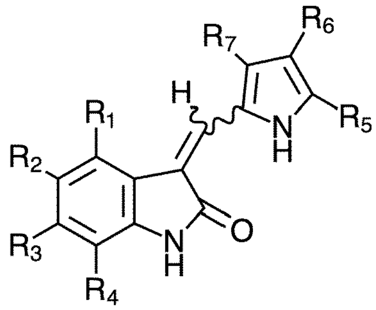

(VIII)

wherein:

R1, R3, R4, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
R2 and R16 are selected from the group consisting of -H, halo, $C_1$-$C_6$ alkyl, trihalomethyl, -OH, $C_1$-$C_6$ alkoxy, -NR13R14, -NR13C(O)R14, -C(O)R15, aryl, heteroaryl, and -$S(O)_2$NR13R14;
R6 is -C(O)R10;
R10 is -N(R11)$(CH_2)_n$R16;
R11 is selected from the group consisting of -H and $C_1$-$C_6$ alkyl;
R13 and R14 are independently selected from the group consisting of -H, $C_1$-$C_6$ alkyl, $C_1$-$C_6$ cycloalkyl, aryl and heteroaryl; or R13 and R14, together with the nitrogen or carbon atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R15 is selected from the group consisting of -H, -OH, $C_1$-$C_6$ alkoxy and aryloxy; and
n is an integer from 0, 1, 2, 3, or 4;
or a salt thereof including a pharmaceutically acceptable salt thereof.

Particular embodiments of the IRE-1A inhibitors of formula (VIII), are those wherein one or more of the following restrictions apply;

- R1 is H and R3, R4, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R3 is H and R1, R4, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R4 is H and R1, R3, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R5 is $C_1$-$C_6$ alkyl and R1, R3, R4 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R7 is $C_1$-$C_6$ alkyl and R1, R3, R4 and R5 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R1 and R3 are H and R4, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R1 and R4 are H and R3, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R3 and R4 are H and R1, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R5 and R7 are $C_1$-$C_6$ alkyl and R1, R3 and R4 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R1, R3 and R4 are H and, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
- R13 is $C_1$-$C_6$ alkyl and R14 is -H, $C_1$-$C_6$ alkyl, $C_1$-$C_6$ cycloalkyl, aryl and heteroaryl;

- R14 is $C_1$-$C_6$ alkyl and R13 is -H, $C_1$-$C_6$ alkyl, $C_1$-$C_6$ cycloalkyl, aryl and heteroaryl;
- R13 and R14, together with the nitrogen or carbon atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, - CON(CH3)2, $C_1$-$C_6$ alkyl, $C_1$-$C_6$ alkoxy, $C_1$-$C_6$ hydroxylalkyl, $C_1$-$C_6$ alkoxylalkyl;

In a further embodiment, the IRE-1A inhibitors are represented by formula (VIII), wherein:

R1, R3, R4 and R11 are -H;
R2 is halo; more in particular R2 is F;
R5 and R7 are $C_1$-$C_6$ alkyl; more in particular R5 and R7 are methyl;
R6 is -C(O)R10;
R10 is $-N(R11)(CH_2)_nR16$;
R13 and R14 are $C_1$-$C_6$ alkyl; more in particular R13 and R14 are ethyl;
R16 is -NR13R14; and
n is 2;
or a salt thereof including a pharmaceutically acceptable salt thereof.

In another embodiment, the IRE-1A inhibitors are represented by the following formula (IX),

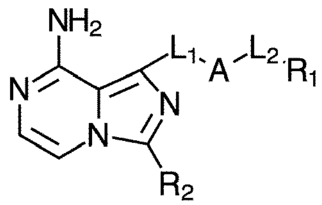

(IX)

wherein:

A is an optionally substituted $C_1$-$C_6$ cycloalkylene, optionally substituted $C_1$-$C_6$ heterocycloalkylene, optionally substituted arylene, or optionally substituted heteroarylene with one or more substituents independently selected from the group consisting of halogen, $-CF_3$, - OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
L1 is a bond or unsubstituted $C_1$-$C_6$ alkylene;
L2 is a bond, -NR6-, -NR6C(O)-, -C(O)NR6-, -NR6C(O)O-, -NR6C(O)NR6-;
R1 is -H, oxo, halogen, $-CX_3$, -CN, $-SO_2Cl$, $-SO_nR10$, $-SO_vNR7R8$, $-NHNH_2$, -ONR7R8, - $NHC=(O)NHNH_2$, -NHC=(O)NR7R8, $-N(O)_m$, -NR7R8, -C(O)R9, -C(O)-OR9, -C(O)NR7R8, - OR10, $-NR7SO_nR10$, -NR7C=(O)R9, -NR7C(O)OR9, -NR7OR9, $-OCX_3$, $-OCHX_2$, $C_1$-$C_6$ alkyl, $C_1$-$C_6$ heteroalkyl, $C_1$-$C_6$ cycloalkyl, $C_1$-$C_6$ heterocycloalkyl, optionally substituted aryl, or optionally substituted heteroaryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
R2 is -H, -CN, -OH, halogen or $C_1$-$C_6$ alkyl;
R6, R7, R8, R9 and R10 are independently selected from -H, halogen, $-CF_3$, -CN, -OH, $-NH_2$, - COOH, $-CONH_2$, $-NO_2$, -SH, $-SO_2Cl$, $-SO_3H$, $-SO_4H$, $-SO_2NH_2$, $-NHNH_2$, $-ONH_2$, - $NHC=(O)NHNH_2$, $-NHC=(O)NH_2$, $-NHSO_2H$, -NHC=(O)H, -NHC(O)OH, -NHOH, $-OCF_3$, - $OCHF_2$, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
each occurrence of the symbol n is independently an integer from 0 to 4; each occurrence of the symbol m and v is independently an integer from 1 to 2; each occurrence of the symbol X is independently a halogen;
or a salt thereof including a pharmaceutically acceptable salt thereof.

Particular embodiments of the IRE-1A inhibitors of formula (IX), are those wherein one or more of the following restrictions apply;

- A is an optionally substituted $C_1$-$C_6$ heterocycloalkylene, optionally substituted arylene, or optionally substituted

heteroarylene with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;

- A is an optionally substituted arylene, or optionally substituted heteroarylene with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
- A is an optionally substituted arylene with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
- R1 is $C_1$-$C_6$ alkyl, $C_1$-$C_6$ heteroalkyl, $C_1$-$C_6$ cycloalkyl, $C_1$-$C_6$ heterocycloalkyl, optionally substituted aryl, or optionally substituted heteroaryl with one or more substituents independently selected from the group consisting of halogen, $-CF_3$, - OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
- R1 is a $C_1$-$C_6$ heteroalkyl, $C_1$-$C_6$ cycloalkyl, $C_1$-$C_6$ heterocycloalkyl, optionally substituted aryl, or optionally substituted heteroaryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
- R1 is a $C_1$-$C_6$ cycloalkyl, $C_1$-$C_6$ heterocycloalkyl, optionally substituted aryl, or optionally substituted heteroaryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
- R1 is a $C_1$-$C_6$ heterocycloalkyl, optionally substituted aryl, or optionally substituted heteroaryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
- R1 is an optionally substituted aryl or optionally substituted heteroaryl with one or more substituents independently selected from the group consisting of halogen, - $CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
- R1 is an optionally substituted aryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
- R2 is -H, -CN, -OH, halogen or $C_1$-$C_6$ alkyl;
- R2 is -CN, -OH, halogen or $C_1$-$C_6$ alkyl;
- R2 is -OH, halogen or $C_1$-$C_6$ alkyl;
- R2 is halogen or $C_1$-$C_6$ alkyl;

In a further embodiment, the IRE-1A inhibitors are represented by formula (IX), wherein:

A is an arylene substituted with an aryl; more in particular A is naphthalene;
L1 is a bond;
L2 is a -NR6C(O)NR6-;
R1 is an aryl substituted with $-CF_3$;
R2 is $C_1$-$C_6$ alkyl; more in particular R2 is $C_4H_9$;
R6 is -H;
or a salt thereof including a pharmaceutically acceptable salt thereof.

In a preferred embodiment, the IRE-1A inhibitors are selected from:

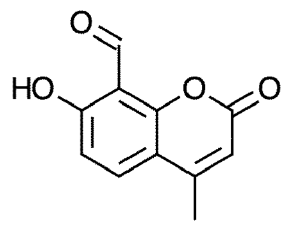

(4μ8c)

or

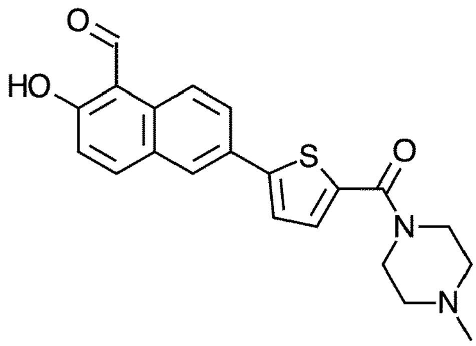

(MKC-3946)

or

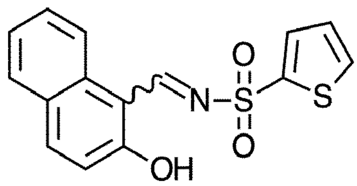

(STF-083010)

or

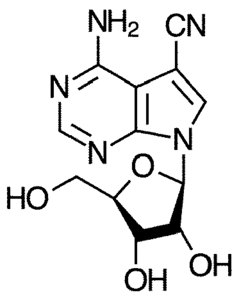

(toyocamycin)

or

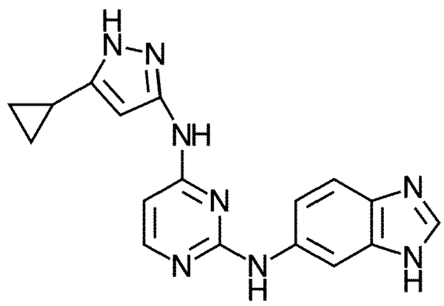

(APY29)

or

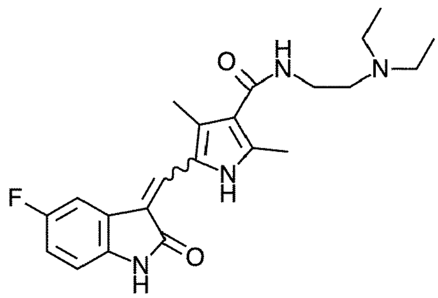

(Sunitinib)

or

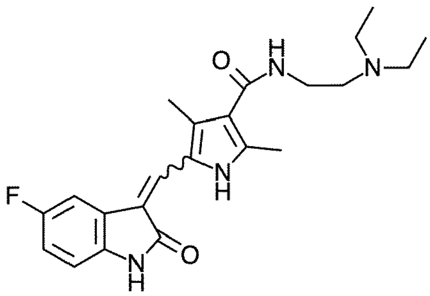

* $C_4H_6O_5$

(Sunitinib malate)

or

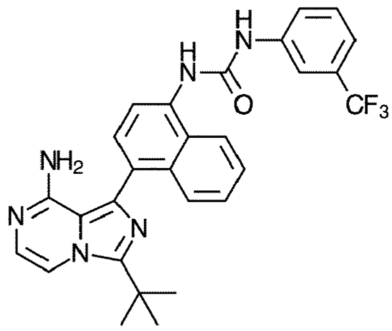

(KIRA-6)

or

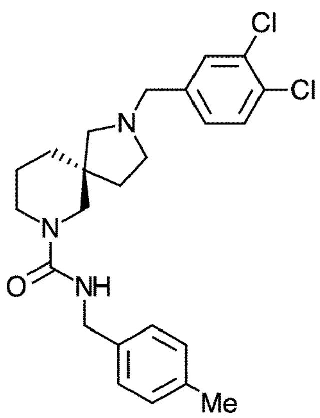

(GSK2850163)

or a salt thereof including a pharmaceutically acceptable salt thereof. Furthermore, IRE-1A inhibitors may be:

- Salicylaldimine analogs, having a

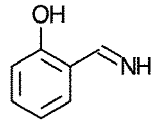

  based structure.
- Mycotoxins, being a secondary metabolite produced by fungal organisms
- (Poly)phenol- or flavonoid derived compounds, having a natural or synthetic origin, such as flavonol quercetin, kaempferol, apigenin and resveratrol

The term "halo" or "halogen" as a group or part of a group is generic for fluoro, chloro, bromo, or iodo.
The term "perfluoro" by itself or as part of another substituent refers to the exhaustive substitution of hydrogen by fluorine in the group or molecule to which it refers
The term "alkyl" by itself or as part of another substituent refers to a fully saturated hydrocarbon of Formula $C_xH_{2x+1}$ wherein x is a number greater than or equal to 1. Generally, alkyl groups of this invention comprise from 1 to 6 carbon atoms. Alkyl groups may be linear or branched and may be substituted as indicated herein. When a subscript is used herein following a carbon atom, the subscript refers to the number of carbon atoms that the named group may contain. Thus, for example, $C_{1-6}$alkyl means an alkyl of one to six carbon atoms. Examples of alkyl groups are methyl, ethyl, n-propyl, i-propyl, butyl, and its isomers (e.g. n-butyl, i-butyl and t- butyl); pentyl and its isomers, hexyl and its isomers. $C_{1-6}$ alkyl includes all linear, branched, or cyclic alkyl groups with between 1 and 6 carbon atoms, and thus includes methyl, ethyl, n-propyl, i- propyl, butyl and its isomers (e.g. n-butyl, i-butyl and t-butyl); pentyl and its isomers, hexyl and its isomers, cyclopentyl, 2-, 3-, or 4-methylcyclopentyl, cyclopentylmethylene, and cyclohexyl.
The term "alkenyl", as used herein, unless otherwise indicated, means straight-chain, cyclic, or branched-chain hydrocarbon radicals containing at least one carbon-carbon double bond. Examples of alkenyl radicals include ethenyl, E- and Z-propenyl, isopropenyl, E- and Z-butenyl, E- and Z-isobutenyl, E- and Z-pentenyl, E- and Z-hexenyl, E,E-, E,Z-, Z,E-, Z,Z-hexadienyl, and the like. An optionally substituted alkenyl refers to an alkenyl having optionally one or more substituents (for example 1, 2, 3 or 4), selected from those defined above for substituted alkyl.
The term "alkynyl", as used herein, unless otherwise indicated, means straight-chain or branched- chain hydrocarbon radicals containing at least one carbon-carbon triple bond. Examples of alkynyl radicals include ethynyl, E- and Z-propynyl, isopropynyl, E- and Z-butynyl, E- and Z-isobutynyl, E- and Z-pentynyl, E, Z-hexynyl, and the like. An optionally substituted alkynyl refers to an alkynyl having optionally one or more substituents (for example 1, 2, 3 or 4), selected from those defined above for substituted alkyl.
The term "alkoxy" or "alkyloxy" as used herein refers to a radical having the Formula -ORb wherein Rb is alkyl. Preferably, alkoxy is $C_{1-6}$ alkoxy. Non-limiting examples of suitable alkoxy include methoxy, ethoxy, propoxy, isopropoxy, butoxy, isobutoxy, sec- butoxy, tert-butoxy, pentyloxy and hexyloxy.
The term "alkenoxy", alone or in combination, refers to a radical of formula alkenyl-O-, provided that the radical is not an enol ether, wherein the term "alkenyl" is as defined above. Examples of suitable alkenoxy radicals include, but are not limited to, allyloxy, E- and Z- 3-methyl-2-propenoxy and the like.
The term "alkynoxy", alone or in combination, refers to a radical of formula alkynyl-O-, provided that the radical is not an -ynol ether. Examples of suitable alkynoxy radicals include, but are not limited to, propargyloxy, 2- butynyloxy and the like.
Where the oxygen atom in an alkoxy group is substituted with sulfur, the resultant radical is referred to as thioalkoxy.
The term "hydroxy" or "hydroxyl" by itself or as part of another substituent refers to the group - OH. Thus, a hydroxyalkyl is an alkyl group as defined above having at least one substituent that is -OH.
The term "aliphatic acyl" alone or in combination, refers to radicals of formula alkyl-C(O)-, alkenyl-C(O)- and alkynyl-C(O)- derived from an alkane-, alkene- or alkyncarboxylic acid, wherein the terms "alkyl", "alkenyl" and "alkynyl" are as defined above. Examples of such aliphatic acyl radicals include, but are not limited to, acetyl, propionyl, butyryl, valeryl, 4-methylvaleryl, acryloyl, crotyl, propiolyl and methylpropiolyl, among others.
The term "carboxy" or "carboxyl" or "hydroxycarbonyl" by itself or as part of another substituent refers to the group $-CO_2H$. Thus, a carboxyalkyl is an alkyl group as defined above having at least one substituent that is $-CO_2H$.
The term "alkylamino" as used herein refers to $R_eNH$- wherein $R_e$ is an alkyl group, for example, ethylamino, butylamino, among others.

The term "alkenylamino" alone or in combination, refers to a radical of formula alkenyl-NH-or (alkenyl)$_2$N-, wherein the term "alkenyl" is as defined above, provided that the radical is not an enamine. An example of such alkenylamino radical is the allylamino radical.

The term "alkynylamino", alone or in combination, refers to a radical of formula alkynyl-NH- or (alkynyl)$_2$N- wherein the term "alkynyl" is as defined above, provided that the radical is not an amine. An example of such alkynylamino radicals is the propargyl amino radical.

The term "dialkylamino" as used herein refers to $R_fR_gN$- wherein $R_f$ and $R_g$ are independently selected from alkyl, for example diethylamino, and methyl propylamino, among others.

The term "amino" as used herein refers to $H_2N$-.

The term "haloalkyl" alone or in combination, refers to an alkyl radical having the meaning as defined above wherein one or more hydrogens are replaced with a halogen as defined above. Non- limiting examples of such haloalkyl radicals include chloromethyl, 1-bromoethyl, fluoromethyl, difluoromethyl, trifluoromethyl, 1,1,1-trifluoroethyl, and the like.

The term "alkoxyalkoxy" as used herein refers to $R_cO$-$R_dO$- wherein $R_c$ is an alkyl as defined above and $R_d$ is alkylene wherein alkylene is -$(CH_2)_{n'}$- wherein n' is an integer from 1 to 6. Representative examples of alkoxyalkoxy groups include methoxymethoxy, ethoxymethoxy, t-butoxymethoxy among others.

The term "carboxaldehyde" as used herein refers to -C(O)R wherein R is hydrogen.

The term "carboxamide" as used herein refers to -C(O)$NR_aR_b$ wherein R and $R_b$ are each independently hydrogen, alkyl or any other suitable substituent.

The term "cycloalkyl" by itself or as part of another substituent is a cyclic alkyl group, that is to say, a monovalent, saturated, or unsaturated hydrocarbyl group having 1, 2, or 3 cyclic structure. Cycloalkyl includes all saturated or partially saturated (containing 1 or 2 double bonds) hydrocarbon groups containing 1 to 3 rings, including monocyclic, bicyclic, or polycyclic alkyl groups. Cycloalkyl groups may comprise 3 or more carbon atoms in the ring and generally, according to this invention comprise from 3 to 6 atoms. The further rings of multi-ring cycloalkyls may be either fused, bridged and/or joined through one or more spiro atoms. Cycloalkyl groups may also be considered to be a subset of homocyclic rings discussed hereinafter. Examples of cycloalkyl groups include but are not limited to cyclopropyl, cyclobutyl, cyclopentyl and cyclohexyl. An "optionally substituted cycloalkyl" refers to a cycloalkyl having optionally one or more substituents (for example 1 to 3 substituents, for example 1, 2, 3 or 4 substituents), selected from those defined above for substituted alkyl.

The term "cycloalkenyl" as used herein alone or in combination refers to a cyclic carbocycle containing from 3 to 6 carbon atoms and one or more double bonds. Examples of such cycloalkenyl radicals include, but are not limited to, cyclopentenyl, cyclohexenyl, cyclopentadienyl and the like.

The term "cycloalkylalkyl" as used herein refers to a cycloalkyl group appended to an alkyl radical, including, but not limited to cyclohexylmethyl.

The term "cycloalkylalkyl" by itself or as part of another substituent refers to a group having one of the aforementioned cycloalkyl groups attached to one of the aforementioned alkyl chains. Examples of such cycloalkylalkyl radicals include cyclopropylmethyl, cyclobutylmethyl, cyclopentylmethyl, cyclohexylmethyl, 1-cyclopentylethyl, 1-cyclohexylethyl, 2-cyclopentylethyl, 2-cyclohexylethyl, cyclobutylpropyl, cyclopentylpropyl, 3-cyclopentylbutyl, cyclohexylbutyl and the like. The term "heterocyclyl-alkyl" by itself or as part of another substituents refers to a group having one of the aforementioned heterocyclyl group attached to one of the aforementioned alkyl group, i.e., to a group -Rd-Rc wherein Rd is alkylene or alkylene substituted by alkyl group and Rc is a heterocyclyl group.

The term "aryl" as used herein refers to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, biphenylyl, biphenylenyl, 5- or 6-tetralinyl, 1-, 2-, 3-, 4-, 5-, 6-, 7-, or 8-azulenyl, 1- or 2-naphthyl, 1-, 2-, or 3-indenyl, 1-, 2-, or 9-anthryl, 1- 2-, 3-, 4-, or 5-acenaphtylenyl, 3-, 4-, or 5-acenaphtenyl, 1-, 2-, 3-, 4-, or 10-phenanthryl, 1- or 2-pentalenyl, 1, 2-, 3-, or 4-fluorenyl, 4- or 5-indanyl, 5-, 6-, 7-, or 8-tetrahydronaphthyl, 1,2,3,4-tetrahydronaphthyl, 1,4-dihydronaphthyl, dibenzo[a,d]cylcoheptenyl, and 1-, 2-, 3-, 4-, or 5-pyrenyl. The aryl ring can optionally be substituted by one or more substituents. An "optionally substituted aryl" refers to an aryl having optionally one or more substituents (for example 1 to 5 substituents, for example 1, 2, 3 or 4) at any available point of attachment. Non-limiting examples of such substituents are selected from halogen, hydroxyl, oxo, nitro, amino, hydrazine, aminocarbonyl, azido, cyano, alkyl, cycloalkyl, alkenyl, alkynyl, cycloalkylalkyl, alkylamino, alkoxy, -SO2-NH2, aryl, heteroaryl, aralkyl, haloalkyl, haloalkoxy, alkoxycarbonyl, alkylaminocarbonyl, heteroarylalkyl, alkylsulfonamide, heterocyclyl, alkylcarbonylaminoalkyl, aryloxy, alkylcarbonyl, acyl, arylcarbonyl, aminocarbonyl, alkylsulfoxide, -SO2Ra, alkylthio, carboxyl, and the like, wherein Ra is alkyl or cycloalkyl. Where a carbon atom in an aryl group is replaced with a heteroatom, the resultant ring is referred to herein as a heteroaryl ring.

The term "heteroaryl" as used herein by itself or as part of another group refers but is not limited to 5 to 12

carbon-atom aromatic rings or ring systems containing 1 to 3 rings which are fused together or linked covalently, typically containing 5 to 8 atoms; at least one of which is aromatic in which one or more carbon atoms in one or more of these rings can be replaced by oxygen, nitrogen or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, benzopyranyl, 1(4H)-benzopyranyl, 1(2H)-benzopyranyl, 3,4-dihydro-1(2H)-benzopyranyl, 3,4-dihydro-1(2H)-benzopyranyl, isobenzofuranyl, benzothiophenyl, isobenzothiophenyl, indazolyl, benzimidazolyl, 1,3-benzoxazolyl, 1,2-benzisoxazolyl, 2,1- benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl, quinoxalinyl, 7-azaindolyl, 6-azaindolyl, 5-azaindolyl, 4-azaindolyl.

The term "arylcarbonyl" or "aroyl" as used herein denotes a group -C(O)-aryl, wherein aryl is as defined above.

The term "aryloxy" as used herein denotes a group -O-aryl, wherein aryl is as defined above.

The term "arylamino", alone or in combination, refers to a radical of formula aryl-NH-, wherein "aryl" is as defined above. Examples of arylamino radicals include, but are not limited to, phenylamino(anilido), naphthlamino, 2-, 3-, and 4- pyridylamino and the like.

The term "biaryl", alone or in combination, refers to a radical of formula aryl-aryl, wherein the term "aryl" is as defined above.

The term "thioaryl", alone or in combination, refers to a radical of formula aryl-S-, wherein the term "aryl" is as defined above. An example of a thioaryl radical is the thiophenyl radical.

The term "diarylamino" refers to a group of formula $-N(Ar^b)_2$ where each Ar is independently an aryl group.

The terms "heterocyclyl" or "heterocyclo" as used herein by itself or as part of another group refer to non-aromatic, fully saturated or partially unsaturated cyclic groups (for example, 3 to 13 member monocyclic, 7 to 17 member bicyclic, or 10 to 20 member tricyclic ring systems, or containing a total of 3 to 10 ring atoms) which have at least one heteroatom in at least one carbon atom- containing ring. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. The rings of multi-ring heterocycles may be fused, bridged and/or joined through one or more spiro atoms. An optionally substituted heterocyclic refers to a heterocyclic having optionally one or more substituents (for example 1 to 4 substituents, or for example 1, 2, 3 or 4), selected from those defined for substituted aryl. Exemplary heterocyclic groups include piperidinyl, azetidinyl, imidazolinyl, imidazolidinyl, isoxazolinyl, oxazolidinyl, isoxazolidinyl, thiazolidinyl, isothiazolidinyl, piperidyl, succinimidyl, 3H- indolyl, isoindolinyl, chromenyl, isochromanyl, xanthenyl, 2H-pyrrolyl, 1-pyrrolinyl, 2-pyrrolinyl, 3- pyrrolinyl, pyrrolidinyl, 4H-quinolizinyl, 4aH-carbazolyl, 2-oxopiperazinyl, piperazinyl, homopiperazinyl, 2-pyrazolinyl, 3-pyrazolinyl, pyranyl, dihydro-2H-pyranyl, 4H-pyranyl, 3,4-dihydro-2H-pyranyl, phthalazinyl, oxetanyl, thietanyl, 3-dioxolanyl, 1,3-dioxanyl, 2,5-dioximidazolidinyl, 2,2,4-piperidonyl, 2-oxopiperidinyl, 2-oxopyrrolodinyl, 2-oxoazepinyl, indolinyl, tetrahydropyranyl, tetrahydrofuranyl, tetrehydrothienyl, tetrahydroquinolinyl, tetrahydroisoquinolinyl, thiomorpholinyl, thiomorpholinyl sulfoxide, thiomorpholinyl sulfone, 1,3-dioxolanyl, 1,4-oxathianyl, 1,4-dithianyl, 1,3,5-trioxanyl, 6H-1,2,5-thiadiazinyl, 2H-1,5,2-dithiazinyl, 2H-oxocinyl, 1H-pyrrolizinyl, tetrahydro- 1,1-dioxothienyl, N-formylpiperazinyl, and morpholinyl.

"Arylalkyl" and "alkylaryl" employ the term "alkyl" as defined above. Rings may be multiply substituted.

The term "aralkenyl", alone or in combination, refers to an aryl substituted alkenyl radical, wherein the terms "aryl" and "alkenyl" are as defined above.

The term "aralkyl", alone or in combination, refers to an aryl substituted alkyl radical, wherein the terms "alkyl" and "aryl" are as defined above. Examples of suitable aralkyl radicals include, but are not limited to, phenylmethyl, phenethyl, phenylhexyl, diphenylmethyl, pyridylmethyl, tetrazolyl methyl, furylmethyl, imidazolyl methyl, indolylmethyl, thienylpropyl and the like.

The term "alkylheterocyclyl" as used herein refers to an alkyl group as previously defined appended to the parent molecular moiety through a heterocyclyl group.

The term "heterocyclylalkyl" as used herein refers to a heterocyclyl group as previously defined appended to the parent molecular moiety through an alkyl group.

The term "aryloxy" as used herein denotes a group -O-aryl, wherein aryl is as defined above.

The term "arylene" as used herein, and unless otherwise specified, refers to a divalent aromatic substituent containing a single aromatic ring.

The term "carbamate" as used herein refers to compounds based on carbamic acid, $NH_2C(O)OH$.

The terms "electron-withdrawing" or "electron-donating" refer to the ability of a substituent to withdraw or donate electrons relative to that of hydrogen if hydrogen occupied the same position in the molecule.

Electron withdrawing groups include halo, nitro, carboxyl, alkenyl, alkynyl, carboxaldehyde, carboxyamido, aryl, quaternary ammonium, trifluoromethyl, and aryl lower alkanoyl among others.

Electron donating groups include such groups as hydroxy, alkyl, amino, alkylamino, dialkylamino, aryloxy, mercapto, alkylthio, alkylmercapto, and disulfide among others. One skilled in the art will appreciate that the aforesaid substituents may have electron donating or electron withdrawing properties under different chemical conditions. Moreover, the present invention contemplates any combination of substituents selected from the above-identified groups.

The most preferred electron donating or electron withdrawing substituents are halo, nitro, alkanoyl, carboxaldehyde, arylalkanoyl, aryloxy, carboxyl, carboxamide, cyano, sulfonyl, sulfoxide, heterocyclyl, guanidine, quaternary ammonium, alkenyl, alkynyl, sulfonium salts, hydroxy, alkoxy, alkyl, amino, alkylamino, dialkylamino, amine alkyl mercapto, mercaptoalkyl, alkylthio and alkyldithio.

"Pentose" refers to C5 sugars and their derivatives.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

More generally, from the above, it will be clear to the skilled person that the compounds of the invention may exist in the form of different isomers and/or tautomers, including but not limited to geometrical isomers, conformational isomers, E/Z-isomers, stereochemical isomers (i.e. enantiomers and diastereoisomers) and isomers that correspond to the presence of the same substituents on different positions of the rings present in the compounds of the invention. All such possible isomers, tautomers and mixtures thereof are included within the scope of the invention.

As mentioned here above, the invention relates to UPR pathway inhibitors, such as PERK and IRE-1A inhibitors (in particular the PERK and IRE-1A inhibitors as herein disclosed) for use in the prevention or treatment of (ER) stress-induced neurodevelopmental disorders. Within the context of the present application neurodevelopmental disorders generally refers to a group of neurological disorders (diseases or dysfunctions) whose root cause is the altered development of the nervous system due to genetic and/or accidental impairments (prenatal injuries, toxic chemicals, viral infections) impeding the ordinary course of events taking place during neural tissue ontogenesis. In particular, it relates to disorders originating from disturbances in the development of the fetal nervous system. These conditions are dependent on the degree of damage to the developing nervous system and may manifest themselves but are not limited to intellectual disability, intellectual and developmental disability, motor disorders, tic disorders, communication disorders, speech disorders, language disorders and the like. In some cases, neurodevelopmental disorders may include Anencephaly; Colpocephaly; Holoprosencephaly; Ethmocephaly; Hydranencephaly; Iniencephaly; Lissencephaly; Megalencephaly; Microcephaly; Porencephaly; and Schizencephaly. It is accordingly an object of the present invention to provide UPR pathway inhibitors, such as PERK and IRE-1A inhibitors (in particular the PERK and IRE-1A inhibitors as herein disclosed) for use in the prevention or treatment of disorders originating from disturbances in the development of the fetal nervous system; in particular neurodevelopmental disorders; more in particular microcephaly.

In a particular embodiment, the neurodevelopmental disorders are related to an endoplasmic reticulum (ER) stress disorder, i.e. the disturbances in the development of the fetal nervous system are originating from endoplasmic reticulum (ER) stress.

In a more specific embodiment, the neurodevelopmental disorders are caused by a viral infection in a fetal phase, i.e. the disturbances in the development of the fetal nervous system are originating from a viral infection during the fetal phase. With fetal phase, the period covering fertilization until birth is meant.

In an even more specific embodiment, the neurodevelopmental disorders are caused by Zika virus (ZIKV); more in particular by a Zika viral infection in a fetal phase.

Furthermore, the IRE-1A inhibitors may be linked to the PERK inhibition pathway as well, via a direct or indirect pathway such as the UPR pathway. In that respect, the IRE-1A inhibitors as indicated above may combined with the PERK inhibitors as indicated above, for use in the prevention or treatment of (ER) stress induced neurodevelopmental disorders, such as microcephaly caused by ZIKV.

Last, in a certain embodiment, the PERK and/or IRE-1A inhibitors are developed for *in vitro* research.

The compounds of the present invention can be prepared according to the reaction schemes mentioned in WO2011119663, WO2014144952 and WO2002022581 but those skilled in the art will appreciate that these are only illustrative for the invention and that the compounds of this invention can be prepared by any of several standard synthetic processes commonly used by those skilled in the art of organic chemistry.

In a preferred embodiment, the compounds of the present invention are useful in the prevention or treatment of neurodevelopmental disorders.

The present invention further provides the use of a compound as defined hereinbefore or the use of a composition comprising said compound, as a human or veterinary medicine for the prevention and/or treatment of a neurodevelopmental disorders; in particular for the prevention and/or treatment of neurodevelopmental disorders originating from disturbances in the development of the fetal nervous system; more in particular microcephaly.

In a preferred embodiment, the invention provides the use of a compound as defined hereinbefore or the use of a composition comprising said compound in the prevention and/or treatment of neurodevelopmental disorders particularly microcephaly.

**METHOD OF TREATMENT**

Treatment may be related to child or adult. For pharmaceutical use, the compounds of the invention may be used as a free acid or base, and/or in the form of a pharmaceutically acceptable acid-addition and/or base-addition salt (e.g. obtained with non-toxic organic or inorganic acid or base), in the form of a hydrate, solvate and/or complex, and/or in the form or a pro-drug or pre-drug, such as an ester. As used herein and unless otherwise stated, the term "solvate" includes any combination which may be formed by a compound of this invention with a suitable inorganic solvent (e.g. hydrates) or organic solvent, such as but not limited to alcohols, ketones, esters and the like. Such salts, hydrates, solvates, etc. and the preparation thereof will be clear to the skilled person; reference is for instance made to the salts, hydrates, solvates, etc. described in US-A-6,372,778, US-A-6,369,086, US-A-6,369,087 and US-A-6,372,733. The pharmaceutically acceptable salts of the compounds according to the invention, i.e. in the form of water-, oil-soluble, or dispersible products, include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalene-sulfonate, nicotinate, oxalate, palmoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. In addition, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

Generally, for pharmaceutical use, the compounds of the inventions may be formulated as a pharmaceutical preparation or pharmaceutical composition comprising at least one compound of the invention and at least one pharmaceutically acceptable carrier, diluent or excipient and/or adjuvant, and optionally one or more further pharmaceutically active compounds.

By means of non-limiting examples, such a formulation may be in a form suitable for oral administration, for parenteral administration (such as by intravenous, intramuscular or subcutaneous injection or intravenous infusion), for topical administration (including ocular), for administration by inhalation, by a skin patch, by an implant, by a suppository, etc.. Such suitable administration forms - which may be solid, semi-solid or liquid, depending on the manner of administration - as well as methods and carriers, diluents and excipients for use in the preparation thereof, will be clear to the skilled person; reference is again made to for instance US-A-6,372,778, US-A-6,369,086, US-A-6,369,087 and US-A-6,372,733, as well as to the standard handbooks, such as the latest edition of Remington's Pharmaceutical Sciences. Some preferred, but non-limiting examples of such preparations include tablets, pills, powders, lozenges, sachets, cachets, elixirs, suspensions, emulsions, solutions, syrups, aerosols, ointments, creams, lotions, soft and hard gelatin capsules, suppositories, eye drops, sterile injectable solutions and sterile packaged powders (which are usually reconstituted prior to use) for administration as a bolus and/or for continuous administration, which may be formulated with carriers, excipients, and diluents that are suitable per se for such formulations, such as lactose, dextrose, sucrose, sorbitol, mannitol, starches, gum acacia, calcium phosphate, alginates, tragacanth, gelatin, calcium silicate, microcrystalline cellulose, polyvinylpyrrolidone, polyethylene glycol, cellulose, (sterile) water, methylcellulose, methyl- and propylhydroxybenzoates, talc, magnesium stearate, edible oils, vegetable oils and mineral oils or suitable mixtures thereof. The formulations can optionally contain other pharmaceutically active substances (which may or may not lead to a synergistic effect with the compounds of the invention) and other substances that are commonly used in pharmaceutical formulations, such as lubricating agents, wetting agents, emulsifying and suspending agents, dispersing agents, desintegrants, bulking agents, fillers, preserving agents, sweetening agents, flavoring agents, flow regulators, release agents, etc.. The compositions may also be formulated so as to provide rapid, sustained or delayed release of the active compound(s) contained therein, for example using liposomes or hydrophilic polymeric matrices based on natural gels or

synthetic polymers. In order to enhance the solubility and/or the stability of the compounds of a pharmaceutical composition according to the invention, it can be advantageous to employ α-, β- or γ-cyclodextrins or their derivatives. An interesting way of formulating the compounds in combination with a cyclodextrin or a derivative thereof has been described in EP-A-721,331. In particular, the present invention encompasses a pharmaceutical composition comprising an effective amount of a compound according to the invention with a pharmaceutically acceptable cyclodextrin.

In addition, co-solvents such as alcohols may improve the solubility and/or the stability of the compounds. In the preparation of aqueous compositions, addition of salts of the compounds of the invention can be more suitable due to their increased water solubility.

Particular reference is made to the compositions, formulations (and carriers, excipients, diluents, etc. for use therein), routes of administration etc., which are known per se for analogous pyridinocarboxamides, such as those described in US-A-4,997,834 and EP-A-0 370 498. More in particular, the compositions may be formulated in a pharmaceutical formulation comprising a therapeutically effective amount of particles consisting of a solid dispersion of the compounds of the invention and one or more pharmaceutically acceptable water-soluble polymers.

The term "a solid dispersion" defines a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase as defined in thermodynamics, such a solid dispersion is referred to as "a solid solution". Solid solutions are preferred physical systems because the components therein are usually readily bioavailable to the organisms to which they are administered.

It may further be convenient to formulate the compounds in the form of nanoparticles which have a surface modifier adsorbed on the surface thereof in an amount sufficient to maintain an effective average particle size of less than 1000 nm. Suitable surface modifiers can preferably be selected from known organic and inorganic pharmaceutical excipients. Such excipients include various polymers, low molecular weight oligomers, natural products and surfactants. Preferred surface modifiers include nonionic and anionic surfactants.

Yet another interesting way of formulating the compounds according to the invention involves a pharmaceutical composition whereby the compounds are incorporated in hydrophilic polymers and applying this mixture as a coat film over many small beads, thus yielding a composition with good bio-availability which can conveniently be manufactured and which is suitable for preparing pharmaceutical dosage forms for oral administration. Materials suitable for use as cores in the beads are manifold, provided that said materials are pharmaceutically acceptable and have appropriate dimensions and firmness. Examples of such materials are polymers, inorganic substances, organic substances, and saccharides and derivatives thereof.

The preparations may be prepared in a manner known per se, which usually involves mixing at least one compound according to the invention with the one or more pharmaceutically acceptable carriers, and, if desired, in combination with other pharmaceutical active compounds, when necessary under aseptic conditions. Reference is again made to US-A-6,372,778, US-A-6,369,086, US-A-6,369,087 and US-A-6,372,733 and the further prior art mentioned above, as well as to the standard handbooks, such as the latest edition of Remington's Pharmaceutical Sciences.

The pharmaceutical preparations of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 1 and 1000 mg, and usually between 5 and 500 mg, of the at least one compound of the invention, e.g. about 10, 25, 50, 100, 200, 300 or 400 mg per unit dosage.

The compounds can be administered by a variety of routes including the oral, rectal, ocular, transdermal, subcutaneous, intravenous, intramuscular or intranasal routes, depending mainly on the specific preparation used and the condition to be treated or prevented, and with oral and intravenous administration usually being preferred. The at least one compound of the invention will generally be administered in an "effective amount", by which is meant any amount of a compound of the Formula I, II or III or any subgroup thereof that, upon suitable administration, is sufficient to achieve the desired therapeutic or prophylactic effect in the individual to which it is administered. Usually, depending on the condition to be prevented or treated and the route of administration, such an effective amount will usually be between 0.01 to 1000 mg per kilogram body weight day of the patient per day, more often between 0.1 and 500 mg, such as between 1 and 250 mg, for example about 5, 10, 20, 50, 100, 150, 200 or 250 mg, per kilogram body weight day of the patient per day, which may be administered as a single daily dose, divided over one or more daily doses, or essentially continuously, e.g. using a drip infusion. The amount(s) to be administered, the route of administration and the further treatment regimen may be determined by the treating clinician, depending on factors such as the age, gender and general condition of the patient and the nature and severity of the disease/symptoms to be treated. Reference is again made to US-A-6,372,778,US-A-6,369,086, US-A-6,369,087 and US-A-6,372,733 and the further prior art mentioned above, as well as to the standard handbooks, such as the latest edition of Remington's Pharmaceutical Sciences.

In accordance with the method of the present invention, said pharmaceutical composition can be administered separately at different times during the course of therapy or concurrently in divided or single combination forms. The

present invention is therefore to be understood as embracing all such regimes of simultaneous or alternating treatment and the term "administering" is to be interpreted accordingly.

For an oral administration form, the compositions of the present invention can be mixed with suitable additives, such as excipients, stabilizers, or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the invention or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant.

For subcutaneous administration, the compound according to the invention, if desired with the substances customary therefore such as solubilizers, emulsifiers or further auxiliaries are brought into solution, suspension, or emulsion. The compounds of the invention can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these formulations may be prepared by mixing the compounds according to the invention with a suitable non-irritating excipient, such as cocoa butter, synthetic glyceride esters or polyethylene glycols, which are solid at ordinary temperatures, but liquefy and/or dissolve in the rectal cavity to release the drug.

In preferred embodiments, the compounds and compositions of the invention are used locally, for instance topical or in both absorbed and non-adsorbed applications.

The compositions are of value in the veterinary field, which for the purposes herein not only includes the prevention and/or treatment of diseases in animals, but also - for economically important animals such as cattle, pigs, sheep, chicken, fish, etc. - enhancing the growth and/or weight of the animal and/or the amount and/or the quality of the meat or other products obtained from the animal. Thus, in a further aspect, the invention relates to a composition for veterinary use that contains at least one compound of the invention and at least one suitable carrier (i.e. a carrier suitable for veterinary use). The invention also relates to the use of a compound of the invention in the preparation of such a composition.

The invention will now be illustrated by means of the following synthetic and biological examples, which do not limit the scope of the invention in any way.

## EXAMPLES

### EXAMPLE 1

qRT-PCR analyses of genes involved in the UPR pathways on extracted RNA from human occipital cortices of three microcephalic ZIKV-infected fetuses in the second trimester (ZIKV#1, 25GW; ZIKV#2 and ZIKV#3, 22GW), and of three uninfected fetuses of similar gestational ages (WT, 21GW, 23GW and 24GW). In contrast to uninfected fetuses, ZIKV-infected cortices exhibited an upregulation of the myxovirus resistance proteins-encoding gene (*MX1*, Figure 1A), which is reported to be induced upon viral infection, as well as several molecular components of the ER stress pathway and of the PERK-ATF4 arm of UPR (Figures 1B-1F)... Together, these results suggest that ZIKV induces ER stress and triggers UPR in cortical progenitors of fetuses during pregnancy (Figure 1P).

To experimentally test this hypothesis, we infected cultured human neural stem cells (hNSCs), which display features of cortical progenitors, with ZIKV (H/PF/2013) for two hours, and analyzed ER stress and UPR molecular

signatures 48 hours later by qRT-PCR and immunocytochemistry (Figures 1G-O). We detected ZIKV RNA in infected hNSCs (Figure 1O), and observed increased immunolabeling for the ER stress markers (calnexin and calreticulin) in these cells in contrast to most uninfected (mock) hNSCs (concomitant with upregulation of *MX1* and the ER stress marker Protein Disulphide Isomerase (PDI; Figures 1G-1H). Genes of the PERK-ATF4 arm of UPR were upregulated upon infection (Figures 1I-1M). Moreover, the increased ratio of spliced/unspliced Xbp1 showed activation of the IRE-1 pathway upon ZIKV infection. Together, these findings directly support that ZIKV triggers ER stress and UPR in hNSCs.

EXAMPLE 2

ZIKV particles were intracerebroventricularly (ICV) injected into the forebrain at E12.5 and brains were analyzed at E18.5. Infected mouse brains were microcephalic and were significantly lighter (Figure 2A), with smaller cortical dimensions (Figure 2B-D) when compared to mock-infected embryos. Similar results were obtained in newborn pups. Importantly, the cortical thickness of ZIKV-infected brains was significantly reduced. Furthermore, ZIKV-infected brains exhibited a strong reduction of deeper (Tbr1+ or Ctip2+ neurons) and upper layer neurons (Satb2+ neurons), as well as a severe disruption in their laminar organization, as compared to uninfected brains. Microdissected cortices from ZIKV-infected embryos showed an upregulation of the AP marker Pax6 (Figure 2E) but not of the intermediate progenitor (IP) marker Tbr2 (Figure 2F). These data were further confirmed by immunolabelings. We also observed an overall relative reduction of expression of neuronal markers (Figures 2G-2H). These findings indicate the depletion of cortical neurons. We next investigated ER stress in ZIKV-infected cortical progenitors in coronal sections of E14.5 brains infected at E12.5. Congruent to our findings in ZIKV-infected hNSCs (Figure 1), we detected increased expression of ER stress markers in ZIKV-infected E14.5 cortices. The qRT-PCR analyses confirmed ER stress induction, with upregulation of *PDI* in ZIKV-infected cortical extracts from E18.5 mouse embryos (Figure 2I). Moreover, in the same samples, we detected increased expression of key molecules of the UPR pathway, which have been shown to control the neurogenic balance in the developing mouse cortex. *Atf4* and its related targets (*Atf5*, *Chac1*, *Chop*, *Slc7a3*) were significantly upregulated in ZIKV-infected cortices (Figures 2L-N, 2P), as well as the ratio of spliced/unspliced Xbp1, which also suggests an activation of the IRE-1 pathway (Figure 2O). These data were supported by Western blot analysis showing activation of the PERK pathway as well as a modest activation of the IRE-1 pathway. We did not detect increased cleavage of ATF6, nor upregulation of its target Xbp1. Similar observations were made in a mouse model of ZIKV maternal-fetal vertical transmission; E14.5 embryonic cortices, collected from *Ifnar1*$^{-/-}$ pregnant dam infected intraperitoneally at gestational day 9.5 showed also upregulation of markers of the UPR pathway (*Zikv, PDI, Atf4, Atf5, Chac1, Chop,* respectively; Figures 2Q-V).

EXAMPLE 3

To test whether the deregulation of UPR induced by ZIKV-infection results in the impairment of this neurogenic balance, we performed ICV injection of ZIKV in E12.5 mouse brains, followed by *in utero* electroporation of GFP-expressing plasmids one day later to fate-map APs at E14.5 (APs, immature IPs and IPs, neurons) (Figures 3A-B). The phenotype of the "direct progeny" of targeted APs was then assessed by immunohistochemistry (Figures 3B-D). In order to characterize the fate of GFP+ cells, we subdivided them into APs (Sox2+, Tbr2- or Tbr2-, Tbr1-), immature IPs (Sox2+,Tbr2+ or Tbr2+, Tbr1-), IPs (Sox2-, Tbr2+ or Tbr2+, Tbr1+), or neurons (Sox2-, Tbr2- or Tbr2-, Tbr1+). The analyses of ZIKV-infected GFP+ APs (as detected by anti-NS1 (non-structural protein 1) antibodies and similarly to anti-flavivirus group antigen antibody 4G2) showed that the majority of GFP+ ZIKV-infected cells were proliferating (65.8 $\pm$ 9.6; n=5 brains; Mean $\pm$ SEM) and, compared to GFP+ cells in uninfected brains, gave rise to a significantly lower proportion of IPs and higher proportion of neurons, without reducing the fraction of self-renewing APs (Figures 3C-D, 3H) or the rate of cell proliferation in the infected cortices (figure 3G). Altogether, these results show that *in vivo* infection of APs by ZIKV directly impacts cortical neurogenesis, by shifting the balance towards direct neurogenesis, at the expense of indirect neurogenesis, thereby reducing the overall projection neuron output. Interestingly, ZIKV also impairs the neurogenic balance through non-cell autonomous mechanism.

To assess if ZIKV directly impacts the neurogenic balance through UPR deregulation in GFP labeled APs, we co-injected GSK2656157 (a PERK inhibitor, named PERKi) with ZIKV into the lateral ventricles of E12.5 embryos and repeated a second PERKi injection at the time of electroporation. Treatment with PERKi (10 $\mu$m, solubilized in DMSO) does not perturb the neurogenic balance, as no significant differences were detected in different cellular compartments between "Mock-DMSO" and "Mock-PERKi" treated brains. Moreover, administration of DMSO did not change the ability of ZIKV (ZIKV-DMSO) to impair neurogenesis as compared to control (Mock-DMSO) (Figure 3E). The co-administration of PERKi with ZIKV remarkably corrected the imbalance between direct and indirect neurogenesis, rescuing the proportions of APs and newborn IPs, IPs and neurons to control levels at E14.5, when compared to "Mock-DMSO" and "Mock-PERKi" treatment (Figure 3E). Altogether, these data suggest that a proper cortical neurogenesis can be restored in the context of ZIKV infection by chemically reducing the activation of UPR induced by ER stress.

EXAMPLE 4

Although an increase of UPR can also lead to apoptosis, we did not observe any reduction of cell survival in E14.5 ZIKV brains infected at E12.5. However, we detected significant neuronal apoptosis at E18.5 upon ZIKV infection at E12.5 together with elevated PDI expression. Detailed analysis revealed that only a fraction of ZIKV-expressing neurons were apoptotic (Figure 4A). No significant apoptosis was observed in the progenitor region of the cortex, likely because ZIKV favors the generation of neurons that exit this region. Indeed, most dying cells were observed in the post-mitotic regions of the cortex and single injection of 50μM PERKi at the time of infection (E12.5) decreased ER stress and apoptosis (Figures 4A). Strikingly, injection of PERKi reduced ZIKV-induced microcephaly, as supported by the significant improvement of brain weight (Figure 4B), cortical dimensions (Figures 4C-D) and numbers of neurons sitting in different cortical layers, compared to vehicle treatment. Since increased splicing of Xbp1 was also detected in ZIKV-infected hNSCs and mouse brains, we injected an inhibitor of IRE-1, 4μ8C (IRE-1i). It rescued the microcephalic phenotype to a similar extent compared to PERKi (Figures 4B-D). Moreover, IRE-1i prevented activation of the PERK-ATF4 UPR arm, suggesting a potential crosstalk between the two UPR pathways. While the effects of PERKi are not associated with a reduction in ZIKV particles, we cannot exclude that the IRE-1 inhibitor-mediated rescue is linked to impairment of ZIKV replication. To model the natural ZIKV infection pathway, we injected viral particles into the placenta of 12.5-days pregnant NMRI mice. Similar to ICV infection, E12.5 intraplacental (IPL) injection of ZIKV led to reduction of cortical thickness at E18.5, together with massive neuronal apoptosis and impairment of cortical cytoarchitecture. These ZIKV-induced cortical defects were rescued by co-injection of PERKi. Similarly to ZIKV, Yellow Fever Virus 17D (YFV17D) and West Nile Virus (WNV) IPL injection resulted in placental infection (Figure 4E). However, in sharp contrast to ZIKV, YFV17D and WNV did not infect APs, neither triggered apoptosis nor induced microcephaly (Figure 4E-H). Of note, in contrast to ZIKV that also infects cells in the ventricular and subventricular zones (VZ/SVZ), YFV17D exhibits a tropism for the intermediate zone (IZ) and cortical plate (CP) populated by postmitotic neurons.

EXAMPLE 5

The neurogenic balances upon Cre-GFP electroporation of Elp3 lox/lox embryos were assessed at E13.5 with or without ISRIB (DMSO used as vehicle control). Percentages of cells were expressed either Tbr1, Tbr2 or both in GFP-labeled cells at E14.5, whereby Tbr1-Tbr2- are apical progenitors, Tbr2+ are intermediate progenitors and Tbr2-Tbr1+ are Neurons (Figure 5)

EXAMPLE 6

To test the efficacy of Salubrinal and ISRIB (PERK inhibitors) in decreasing the expression levels of UPR effectors after ZIKV infection we intracerebroventricularly injected ZIKV + Salubrinal (or ISRIB) in E12.5 mouse cortices and collected brains at E18.5. These samples were stored in Trizol (Invitrogen) and RNA was extracted according to manufacturer instructions. RNA was then retro-transcribed and cDNA was used to perform qRT-PCR to quantify ZIKV and UPR effectors levels in each sample. The number of ZIKV copies in each treated sample were unaffected compared to controls (ZIKV+DMSO brains; Kruskal-Wallis test) (Figure 6A). The expression levels of several PERK pathway effectors were analyzed: mATF4 (Figure 6B), mATF5 (Figure 6C), mCHAC1 (Figure 6D). All tested factors showed a marked decrease with high doses of PERK inhibitors compared to controls (ZIKV + DMSO). (Kruskal-Wallis test, $*p < 0.05$, $** p < 0.005$).

EXAMPLE 7

To test the efficacy of Resveratrol (IRE1a inhibitor) in decreasing the expression levels of UPR effectors after ZIKV infection we intracerebroventricularly injected ZIKV + Resveratrol in E12.5 mouse cortices and collected brains at E18.5. These samples were stored in Trizol (Invitrogen) and RNA was extracted according to manufacturer instructions. RNA was then retro-transcribed and cDNA was used to perform qRT-PCR to quantify ZIKV and UPR effectors levels in each sample. The number of ZIKV copies in each treated sample were unaffected compared to controls (ZIKV+DMSO brains; Kruskal-Wallis test) (Figure 7A). The expression levels of several PERK pathway effectors were analyzed: mATF4 (Figure 7B), mATF3 (Figure 7C), mXbp1 spliced/unspliced and mXbp1 tot (Figure 7D), mChop (Figure 7E). All tested factors, apart Xbp1, showed a marked decrease with high doses of PERK inhibitors compared to controls (ZIKV + DMSO). Xbp1 showed a significant change in spliced/unspliced ratio, indicating that the increased IRE1a activity, due to ZIKV infection, is counteracted by Resveratrol co-injection (Kruskal-Wallis test, $*p < 0.05$, $** p < 0.005$).

EXAMPLE 8 (MATERIAL & METHODS)

***Animals***

Time-mated NMRI (Janvier Labs, Saint Berthevin, France) were housed under standard conditions and all procedures were approved by the Animal Ethics Committee of the University of Liege (#16-1829) and performed in accordance with the guidelines of the Belgian Ministry of Agriculture in agreement with the European Community Laboratory Animal Care and Use Regulations (86/609/CEE, Journal Officiel des Communaute's Européennes L358, 18 December 1986). Mice in which the type I interferon (IFN) receptor gene was knocked out (IFN-$\alpha/\beta R^{-/-}$ mice) were housed in the Institut Pasteur animal facilities accredited by the French Ministry of Agriculture for experimentation in live mice, and were approved by the Ethics Committee #89 and registered under the reference #2016-0018. All experiments on animals were performed in compliance with French and European regulations on care and protection of laboratory animals (EC Directive 2010/63, French Law 2013-118, February 6th, 2013).

***Human fetal cortical samples***

Human fetal brains were obtained following medical pregnancy termination and processed as described in Lambert *et al.* (2011). Frontal and occipital cortex samples were dissected and flash frozen in liquid nitrogen or Trizol at -80°C. RNA was extracted using Total RNA kit I (Omega). The study was approved by all relevant Ethics Committees (Erasme Hospital, Université Libre de Bruxelles, and Belgian National Fund for Scientific Research FRS/FNRS; French Agence de la biomédicine, #PFS15-009) on research involving human subjects. Written informed consent was given by the parents of each donor.

***Immunohistochemistry***

Embryonic (E) 14.5, E16.5 and E18.5 mouse brains were dissected in 0.1M phosphate-buffered saline pH7.4 (PBS), and were fixed in 4% paraformaldehyde (PFA in PBS) for one hour at room temperature, whereas human neural stem cells (hNSCs) were fixed in 4% PFA at 4°C for 10 minutes. Embryonic mouse brains were cryoprotected (30% sucrose in PBS) before embedding in gelatin for cryosectioning (Leica) (14μm) onto slides (SuperFrost Plus, VWR International). Fluorescent immunohistochemistry was performed as previously described (30). In summary, antigen retrieval (Dako Target Retrieval Solution,) of mouse brains were performed at 95°C for 15 minutes, prior to incubation with primary antibodies. The following primary antibodies were used: anti-PDI, anti-Calreticulin and anti-Calnexin (1:300, rabbit, #92516 ER-stress kit from CST (Cell Signaling Technology)); anti-Cleaved Caspase 3 (1:100, rabbit, #9661, CST), anti-Tbr1 (1:200, rabbit, ab31940, Abcam), anti-Tbr2 (1:500, rat, 14-4875-82, Ebioscience), anti-Ki67 (1:100, mouse, 550609, BD Pharmingen), anti-Sox2 (1:200, goat, sc-17320, Santa Cruz), anti-Flavivirus Group Antigen (1:1000, mouse, MAB10216, Millipore), anti-NS1 from Dengue Virus conjugated with Alexa-546 (1:500, isolated by Marie Flamand) anti-Pax6 (1:500, mouse, AB528427, DSHB), anti-GFP (1:1000, Rabbit, TP401, Torrey Pines Biolabs), anti-GFP (1:1500, Goat, ab6673, Abcam), Anti-Sox1 (1:500, Goat, AF3369 RD System), anti-Nestin (1:500, chicken, NB100-1604, Novus Biologicals), anti-Ctip2 (1:100, rat, ab28448, Abcam), anti-Satb2 (1:2000, rabbit, ab92446, Abcam), anti-Tuj1 (1:250, mouse, MMS-435P, Covance). The respective secondary antibodies used were (1:800; anti-mouse, anti-rabbit, anti-rat, anti-chicken or anti-goat) conjugated either with Alexa-488, Alexa-555, Alexa 405 or Alexa-647 (Jackson ImmunoResearch Laboratories or Life Technologies). Nuclei were counterstained with Dapi (1:10000, Sigma) or Hoechst (1:5000, Termo Fisher Scientific) and mounted in Mowiol (SIGMA) solution.

***Quantification of virus copy number in infected tissues and cells.***

The number of Zika particles in the samples analyzed by qRT-PCR (see above) was calculated as previously described (Lanciotti et al, 2008). Briefly, a standard curve was created by performing serial dilutions of a plasmid containing 76-bp long sequence from the Zika genome (from nt 1,086 to 1,162; GCF_000882815.3, as indicated in Nature Neurosciences (2018) Gladwyn et al.) and querying these dilutions by qRT-PCR analysis.

This standard curve allows the conversion of Cp of samples queried for Zika virus (as described above) into N° of Zika copies/μ L. We used the following formula to link this quantification to the amount of RNA retrotranscribed for the analysis of each sample:

$$\text{Number (N°) of copies/μl} \times k \times d \times P = \text{N° copies/μg RNA}$$

where k is a correction factor introduced to calculate the N° of copies with respect to 1 μ g of RNA (in this instance 0.5

μ g of RNA were used to synthetize cDNA, so k = 2), d is a dilution factor, taking into account the dilution of cDNA used for the analysis (in this case the cDNA was diluted 1:30, so d = 30) and P is a correction factor introduced to calculate the N° of Zika copies with respect to the total amount
of cDNA (here 3.4 μ L on a total of 20 μ L of cDNA mix were used, so P = 5.88).

Finally, the N° of copies/μ g RNA was divided for the relative concentration of each sample analyzed by qRT-PCR (since for each analyzed gene in this study we performed a relative quantification compared to the controls of the same experiment). The relative concentration of each sample is automatically calculated by the LightCycler480 and its accompanying software.

***Virus production***

ZIKV H/PF/2013 was amplified on mosquito C6/36 cells and supernatants were harvested and frozen at -80°C. Titer of virus stock was determined by tissue cytopathic infectious dose 50 (TCID50) on Vero cells, and viral titers were expressed as TCID50/ml. Manipulation and in vivo injection of ZIKV was authorized by the Animal Ethics Committee of the University of Liege (#16-1829).

***Induction of hNSCs from hIPSCs and ZIKV infection***

Approvals of the Ethics Committee of the University of Liege for research and protocols (#B70720096309), and patient informed consents were obtained before deriving hiPSCs from skin fibroblasts isolated by punch biopsies. All experiments were conducted according to the guidelines of the Ethics Committee of the University of Liege. The hNSCs had been generated from human skin fibroblast reprogrammed hIPSC from healthy donors following the procedure described in Borgs *et al.* (2016). The hNSC had been maintained on Poly-ornitin/laminin coated dishes and cultured in the neural induction medium: 50%-50% DMEM/F12 - Neurobasal medium supplemented with 2% of B27, 1% of N-2, 0.5% Glutamax (35050-038 Gibco), 10ng/ml of Epidermal Growth Factor (EGF; AF-100-15 Peprotech) and bFGF 10 ng/ml.

For ZIKV infection of hNSCs, cells at early passage were seeded in 6-well plates and 24-well plates with coverslips 24 hours before the infection. In all experiments, human neuronal progenitors were seeded in 6- and 24- well plates on coated coverslips at densities of $3x10^5$ and $6x10^4$ respectively, and either infected at MOI5 (multiplicity of infection) or treated with Mock medium for 2 hours. The medium was then replaced with fresh neural induction medium the NSCs were fixed in Trizol or 4% PFA 48 hours after the infection for qRT-PCR or immunofluorescence experiments respectively.

***RNA extraction and qRT-PCR analyses***

Total RNA was obtained from micro-dissected embryonic mouse brains, and neuronal progenitors seeded in 6 well-plate culture dishes after application of Trizol according to the manufacturer's protocol (Ambion- Life Technologies, 15596018).

The quantity and quality of RNA were assessed by NanoDrop 1000 (Nano-Drop Technologies) before cDNA synthesis by SuperScript III reverse transcriptase (Invitrogen), as used according to the manufacturer's instructions. The resulting cDNA was used for quantitative PCR, using Faststart Universal SYBR Green Master (Roche) in an Applied Biosystem 7900HT Fast Real-Time PCR detection system (Applied Biosystems, Foster city, USA). The following genes of interest (GOI) were normalized to the reference genes β-actin, Glyceraldehyde-3-Phosphate dehydrogenase (GAPDH), hypoxanthine phosphoribosyltransferase 1 (HPRT1), and 36b4 ribosomal protein (36b4).

Tested genes were GAPDH, HPRT1, 36B4, ACTIN, ZIKA, Atf4, Atf3, Atf5, Chac1, Chop, Sesn2, Tbr2, Neurod6, Tubb3, hPDI, Slc7a3, MX1, TLR3, HRD1, OCT4, NESTIN, PAX6, S0X1 and mPDI.

***Intra-cerebral and intraperitoneal ZIKV injection, and in utero electroporation***

Surgeries were performed on timed-pregnant mice (at embryonic day (E)12.5 and E13.5 days), when the noon of the day after mating was considered E0.5. The animals were anaesthetized with isoflurane (Abbot Laboratories Ltd, Kent, UK) in an oxygen carrier prior to administration of Temgesic (Schering-Plough, Brussels, Belgium) (0.1mg/kg body weight). Following which, the uteri horns were carefully extracted through a 1.0 to 1.5-centimeter incision in the ventral peritoneum and placed on humidified gauze pads by using a pair of ring forceps.

All solutions were mixed with 0.05% Fast Green (Sigma, Bornem, Belgium) prior to injections using pulled borosilicate needle and a Femtojet microinjector (VWR International). 1.0 to 2.0 μL of either ZIKV virus ($1.6x10^7$ TCID50/ml) or mock media were injected through the uterine wall into the telencephalic vesicle at (E)12.5, whilst pCAGGS-IRES-GFP (1 to 3μg/μL) was injected into the ipsilaterally infected vesicle at E13.5. The plasmid DNA was purified using

a Plasmid Endofree Maxi Kit (Qiagen, Hilden, Germany) and sequence-verified. The GSK 2606414 (10μM) was concomitantly administered with either ZIKV or mock media at E12.5, and with the pCAGGS-IRES-GFP solution at E13.5.

*In utero* electroporations were performed as previously described Laguesse *et al.* (2015) with minor modifications. Briefly, five electrical pulses were applied at 30mV (50ms duration) across the uterine wall of E13.5 pregnant mice at 950ms interval using 3mm platinum tweezers electrodes (CUY650P3, Sonidel, Ireland) and an ECM-830 BTX square wave electroporator (VWR International).

After the injections and electroporations at E12.5 and E13.5 respectively, the uterine horns were replaced into the abdominal cavity, followed by suturing of the abdominal wall and skin using either Viacryl or Sofsilk surgical needle and thread. The whole procedure was completed within 30 minutes. Following which, the mice were placed in a warmed chamber for post-operative recovery. Embryonic tissues were collected from pregnant mice at E14.5, E16.5 and E18.5, after euthanasia by neck dislocation. Successfully electroporated embryos were identified under a fluorescent binocular microscope and were processed as described above.

Timed-mated IFN-$\alpha$/$\beta R^{-/-}$ mice were infected intra-peritoneally at E9.5 with 100 μl of ZIKV ($1x10^7$ TCID50/ml). Five days after infection, pregnant mice were sacrificed at E14.5 by neck dislocation and cerebral cortex of each fetus was collected.

### *Image acquisition and manipulation*

For each embryonic brain and hNSCs sample, magnified fields (20x, 40x and 60x) were acquired with either the Nikon A1 or the Zeiss LSM 880 AiryScan Elyra S.1 confocal microscopes, and further processed with ImageJ 1.42q 276 (Wayne Rasband, National Institutes of Health) and Fiji (http://pacific.mpi277 cbg.de/wiki/index.php/Main_Page) software.

### *Transmission Electron Microscopy*

E14.5 mouse brains were fixed at 4°C in 2.5% glutaraldehyde in 0.1 M Sörensen's buffer (0.2 M $NaH_2PO_4$, 0.2 M $Na_2HPO_4$, pH 7.4) for 2 hours. Human fetal cortical samples were fixed at 4°C in the same solution for 24 hours. After several washes in Sörensen's buffer, the mouse and human samples were post-fixed at 4 °C with 2% osmium tetroxide in Sörensen's buffer for 60 min, then washed in deionized water, dehydrated at room temperature through a graded ethanol series (70%, 96% and 100%) and embedded in Epon for 48 hours at 60 °C. Ultrathin 70 nm sections were obtained by means of an ultramicrotome (Reichert Ultracut E) equipped with a diamond knife (Diatome). The sections were mounted on copper grids coated with collodion and contrasted with uranyl acetate and lead citrate for 15 minutes each. The ultrathin sections were observed under a JEM-1400 transmission electron microscope (Jeol) at 80 kV and photographed with a 11 MegaPixel bottom-mounted TEM camera system (Quemesa, Olympus). The images were analysed via RADIUS software (Olympus).

### *Cell counting and Statistical analysis*

GraphPad Prism software (version 6) was utilized for the statistical analyses of data obtained from three to eight brains per experimental condition. Dual comparisons were performed using unpaired two-tailed Student's t test, while those for multiple comparisons were performed with two-way ANOVA followed by the Bonferroni post-hoc test as specified in the figure legends; *$p < 0.05$, **$p < 0.01$, ***$p < 0.001$, ****$p < 0.0001$ for all statistics herein. All experiments were replicated at least three times, unless otherwise specified.

### *Data Availability*

The authors declare that all other data supporting the findings of this study are available within the paper and its supplementary information files.

### *Experimental design*

Cultured hNSCs in 6- and 24- well plates were randomly allocated to either the Mock or ZIKV infection. All time-mated pregnant dams and embryos were randomly allocated to the different treatments during the surgeries. The sample sizes were based on previous studies of neurogenic balance during cortical development (Laguesse *et al.*, 2015). Each embryo was considered as a biological replicate. Single blinding was performed, whereby information regarding treatment of each sample was not transferred between the animal surgeon, the microscopist and analyst, until the final data was acquired for statistical analyses.

*Bibliography*

1. Lambert, N. et al. Genes expressed in specific areas of the human fetal cerebral cortex display distinct patterns of evolution. PLoS One 6, e17753, doi:10.1371/journal.pone.0017753 (2011).
2. Cau, E., Gradwohl, G., Fode, C. & Guillemot, F. Mash1 activates a cascade of bHLH regulators in olfactory neuron progenitors. Development 124, 1611-1621 (1997).
3. Borgs, L. et al. Dopaminergic neurons differentiating from LRRK2 G2019S induced pluripotent stem cells show early neuritic branching defects. Scientific reports 6, 33377, doi:10.1038/srep33377 (2016).
4. Laguesse, S. et al. A Dynamic Unfolded Protein Response Contributes to the Control of Cortical Neurogenesis. Dev Cell 35, 553-567, doi:10.1016/j.devcel.2015.11.005 (2015).
5. Walter & Ron. The Unfolded Protein Response: from stress pathway to homeostatic regulation. Science 334: 1081-1086 (2011)
6. Tomasio et al. Selective inhibition of the unfolded protein response: targeting catalytic sites for Schiff base modification. Molecular Biosystems 9, 2408-2416 (2013)
7. Tsuru et al. Novel Mechanisms of enhancing IRE1$\alpha$-XBP1 signalling via the PERK-ATF4 pathway Scientific Reports 6:24217 (2016)
8. Jiang et al. Targeting the IRE1$\alpha$-XBP1 branch of the Unfolded Protein Response in Human Diseases. Semin. Cancer Biol. 33, 48-56 (2015).
9. Halliday & Mallucci. Review: Modulating the unfolded protein response to prevent neurodegeneration and enhance memory. Neuropathology & Applied Neurobiology 41, 414-427 (2015)
10. Lanciotti, R. S. et al. Genetic and serologic properties of Zika virus associated with an epidemic, Yap State, Micronesia, 2007. Emerg. Infect. Dis. 14, 1232-1239 (2008).
11. Gladwyn-Ng et al. Stress-induced unfolded protein response contributes to Zika virus-associated microcephaly. Nature Neurosciences 21, 63-71 (2018)

## Claims

**1.** UPR pathway inhibitors for use in the prevention or treatment of neurodevelopmental disorders. wherein said UPR pathway inhibitors are IRE-1A inhibitors.

**2.** The UPR pathway inhibitors as indicated in claim 1, for use according to claim 1, represented by the following formula (IV),

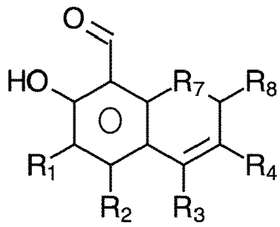

(IV)

wherein:

R1, R2 and R8 independently are H, =O, halogen, -OH, $C_1$-$C_6$ alkenyl, $C_1$-$C_6$ alkynyl, phenyl, optionally substituted $C_1$-$C_6$ alkyl, optionally substituted $C_1$-$C_6$ alkoxyl, or optionally substituted mono- or dialkylamino. Optional substituents for the $C_1$-$C_6$ alkyl, the $C_1$-$C_6$ alkoxyl or the mono- or dialkylamino are (1) a $C_1$-$C_6$ hydrocarbon chain containing a N or O atom and optionally substituted with halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, and (2) a cycloalkyl which may contain one or more heteroatoms selected from N, O, and S, and which is optionally substituted with halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl;

R3 or R4 are independently H, halogen, -OH, $C_1$-$C_6$ perfluoroalkyl, $C_1$-$C_6$ perfluoroalkoxy, -CN, $-CONH_2$, $-CON(CH_3)_2$, $C_1$-$C_6$ alkyl, $C_1$-$C_6$ alkenyl, $C_1$-$C_6$ alkoxylalkyl, $C_1$-$C_6$ alkynyl, $C_1$-$C_6$ alkoxy, $C_1$-$C_6$ hydroxylalkyl,

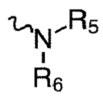

,

or phenyl, wherein said $C_1$-$C_6$ alkyl, $C_1$-$C_6$ alkenyl, $C_1$-$C_6$ alkoxylalkyl, $C_1$-$C_6$ alkynyl, $C_1$-$C_6$ alkoxy, and phenyl are optionally substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ perfluoroalkoxy,

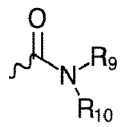

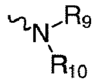

, ;

Or

R3 or R4, are a 5-or 6- membered cycloalkyl or heteroaryl that is substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

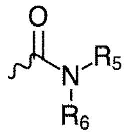 , 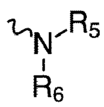 , 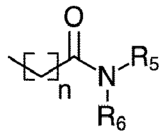

wherein n is 0, 1, or 2; or

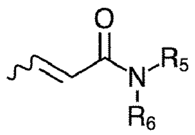

R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R7 is -C, =C, O, S or N;
R9 and R10, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
or a salt thereof including a pharmaceutically acceptable salt thereof.

**3.** The UPR pathway inhibitors as indicated in claim 2, for use according to claim 1, represented by formula (IV) wherein:

R1, R2 and R8 independently are H, =O, halogen, -OH, mono- or dialkylamino, $C_1$-$C_6$ alkenyl, $C_1$-$C_6$ alkynyl, phenyl, $C_1$-$C_6$ alkyl, or $C_1$-$C_6$ alkoxyl;
R3 or R4 are independently H, halogen, -OH, CN, $-CONH_2$, $-CON(CH_3)_2$, $C_1$-$C_6$ alkyl, $C_1$-$C_6$ alkoxy, $C_1$-$C_6$ hydroxylalkyl, $C_1$-$C_6$ alkoxylalkyl,

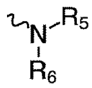
,

$C_1$-$C_6$ alkenyl, $C_1$-$C_6$ alkynyl, or phenyl; Or
R3 or R4 are a 5-or 6- membered heteroaryl that is substituted with one or more substituents independently selected from the group consisting of halogen, -OH, -CN, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl,

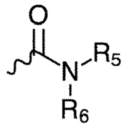
,
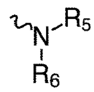
,
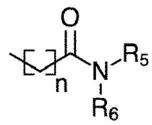

wherein n is 0, 1, or 2; or

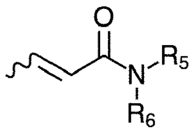
;

R5 and R6, together with the nitrogen atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R7 is =C or O;
or a salt thereof including a pharmaceutically acceptable salt thereof.

**4.** The UPR pathway inhibitors as indicated in claim 1, for use according to claim 1, represented by the following formula (V),

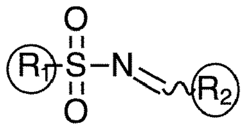
(V)

wherein:

R1 and R2 are a 5- or 6- membered cycloalkyl or heteroaryl that is optionally substituted with one or more substituents independently selected from the group consisting of halogen, -OH, - CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; or a salt thereof including a pharmaceutically acceptable salt thereof.

**5.** The UPR pathway inhibitors as indicated in claim 4, for use according to claim 1, represented by formula (V), wherein:

R1 is a thiophene that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; more in particular R1 is thiophene.
R2 is a naphthalene that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl; more in particular R2 is naphthalene substituted with -OH.
or a salt thereof including a pharmaceutically acceptable salt thereof.

6. The UPR pathway inhibitors as indicated in claim 2, for use according to claim 1, represented by the following formula (VI),

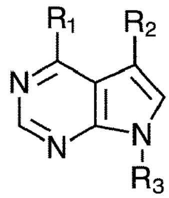

(VI)

wherein:

R1 is $-NH_2$, halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl;
R2 is -H, -OH, -CN, -COOH, $-C(O)NH_2$, $-C(S)NH_2$, $-C(NH)NH_2$,

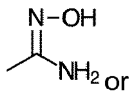
or
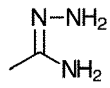
;

and
R3 is a 5- or 6- membered cycloalkyl, heterocycloalkyl or heteroaryl that is optionally substituted with one or more substituents independently selected from the group consisting of halogen, - OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
or a salt thereof including a pharmaceutically acceptable salt thereof.

7. The UPR pathway inhibitors as indicated in claim 6, for use according to claim 1, represented by formula (VI), wherein:

R1 is $-NH_2$ or-OH;
R2 is -H or -CN; and
R3 is a 5- membered heterocycloalkyl that is optionally substituted with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl; in particular R3 is a pentose; more in particular R3 is ribofuranose, ribopyranose, arabinofuranose, arabinopyranose, xylopyranose, or lyxopyranose; even more in particular R3 is ribofuranose.
or a salt thereof including a pharmaceutically acceptable salt thereof.

8. The UPR pathway inhibitors as indicated in claim 2, for use according to claim 1, represented by the following formula (VII),

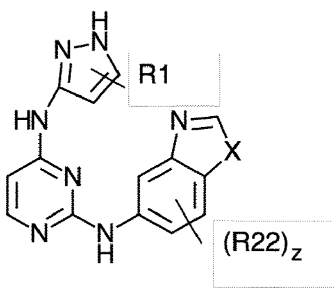

(VII)

wherein:

R1 and R22 are independently -H, halogen, -CN, -NO, $-NO_2$, -NR9R10, -OR11, -COOR12, - SR13, -COR14,

optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
R9, R10, R11, R12, R13, and R14 are independently -H, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
X is NH or S; and z is an integer from 0 to 5;
or a salt thereof including a pharmaceutically acceptable salt thereof.

**9.** The UPR pathway inhibitors as indicated in claim 8, for use according to claim 1, represented by formula (VII), wherein:

R1 is -H, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with one or more substituents independently selected from the group consisting of halogen, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
X is NH; and z is an integer with value 0.
or a salt thereof including a pharmaceutically acceptable salt thereof.

**10.** The UPR pathway inhibitors as indicated in claim 1, for use according to claim 1, represented by the following formula (VIII),

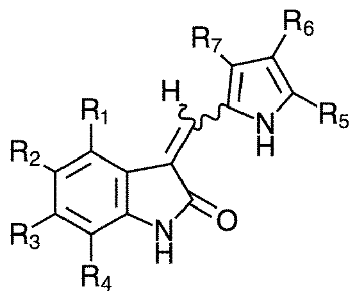

(VIII)

wherein:

R1, R3, R4, R5 and R7 are independently selected from the group consisting of -H, halo, $C_1$-$C_6$ alkyl, $C_1$-$C_6$ cycloalkyl, aryl, heteroaryl, -OH, $C_1$-$C_6$ alkoxy, $C_1$-$C_6$ carbonyl;
R2 and R16 are selected from the group consisting of -H, halo, $C_1$-$C_6$ alkyl, trihalomethyl, -OH, $C_1$-$C_6$ alkoxy, -NR13R14, -NR13C(O)R14, -C(O)R15, aryl, heteroaryl, and $-S(O)_2NR13R14$;
R6 is -C(O)R10;
R10 is -N(R1 1)(CH2)nR16;
R11 is selected from the group consisting of -H and $C_1$-$C_6$ alkyl;
R13 and R14 are independently selected from the group consisting of -H, $C_1$-$C_6$ alkyl, $C_1$-$C_6$ cycloalkyl, aryl and heteroaryl; or R13 and R14, together with the nitrogen or carbon atom to which they are attached, form a heterocycle containing one or more heteroatoms selected from N, O, and S, optionally substituted with one or more substituents selected independently from halogen, -OH, $C_1$-$C_6$ perfluoroalkyl, $C_1$-$C_6$ perfluoroalkoxy, -CN, -CONH2, -CON(CH3)2, $C_1$-$C_6$ alkyl, $C_1$-$C_6$ alkoxy, $C_1$-$C_6$ hydroxylalkyl, $C_1$-$C_6$ alkoxylalkyl;
R15 is selected from the group consisting of -H, -OH, $C_1$-$C_6$ alkoxy and aryloxy; and
n is an integer from 0, 1, 2, 3, or 4;
or a salt thereof including a pharmaceutically acceptable salt thereof.

**11.** The UPR pathway inhibitors as indicated in claim 10, for use according to claim 1, wherein:

R1, R3, R4 and R11 are -H;
R2 is halo; more in particular R2 is F;
R5 and R7 are $C_1$-$C_6$ alkyl; more in particular R5 and R7 are methyl;
R6 is -C(O)R10;
R10 is -N(R11)$(CH_2)_n$R16;
R13 and R14 are $C_1$-$C_6$alkyl; more in particular R13 and R14 are ethyl;
R16 is -NR13R14; and
n is 2;
or a salt thereof including a pharmaceutically acceptable salt thereof.

**12.** The UPR pathway inhibitors as indicated in claim 1, for use according to claim 1, represented by the following formula (IX),

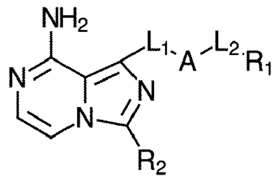

(IX)

wherein:

A is an optionally substituted $C_1$-$C_6$ cycloalkylene, optionally substituted $C_1$-$C_6$ heterocycloalkylene, optionally substituted arylene, or optionally substituted heteroarylene with one or more substituents independently selected from the group consisting of halogen, $-CF_3$, - OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
L1 is a bond or unsubstituted $C_1$-$C_6$ alkylene;
L2 is a bond, -NR6-, -NR6C(O)-, -C(O)NR6-, -NR6C(O)O-, -NR6C(O)NR6-;
R1 is -H, oxo, halogen, $-CX_3$, -CN, $-SO_2Cl$, $-SO_nR10$, $-SO_vNR7R8$, $-NHNH_2$, -ONR7R8, - NHC=(O)$NHNH_2$, -NHC=(O)NR7R8, $-N(O)_m$, -NR7R8, -C(O)R9, -C(O)-OR9, -C(O)NR7R8, - OR10, $-NR7SO_nR10$, -NR7C=(O)R9, -NR7C(O)OR9, -NR7OR9, $-OCX_3$, $-OCHX_2$, $C_1$-$C_6$ alkyl, $C_1$-$C_6$ heteroalkyl, $C_1$-$C_6$ cycloalkyl, $C_1$-$C_6$ heterocycloalkyl, optionally substituted aryl, or optionally substituted heteroaryl with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl,
$C_1$-$C_6$ alkoxy, $C_1$-$C_6$ hydroxylalkyl, $C_1$-$C_6$ alkoxylalkyl, $C_1$-$C_6$ perfluoroalkoxyl;
R2 is -H, -CN, -OH, halogen or $C_1$-$C_6$ alkyl;
R6, R7, R8, R9 and R10 are independently selected from -H, halogen, $-CF_3$, -CN, -OH, $-NH_2$, - COOH, $-CONH_2$, $-NO_2$, -SH, $-SO_2Cl$, $-SO_3H$, $-SO_4H$, $-SO_2NH_2$, $-NHNH_2$, $-ONH_2$, - NHC=(O)$NHNH_2$, -NHC=(O)$NH_2$, $-NHSO_2H$, -NHC=(O)H, -NHC(O)OH, -NHOH, $-OCF_3$, - $OCHF_2$, optionally substituted $C_1$-$C_6$ alkyl, optionally substituted $C_1$-$C_6$ heteroalkyl, optionally substituted $C_1$-$C_6$ cycloalkyl, optionally substituted $C_1$-$C_6$ heterocycloalkyl, optionally substituted aryl, or optionally substituted heteroaryl with with one or more substituents independently selected from the group consisting of halogen, $-CF_3$, -OH, -CN, $C_1$-$C_6$ carboxyl, $C_1$-$C_6$ alkyl, $C_1$-$C_6$ perfluoroalkyl, $C_1$-$C_6$ alkoxy, $C_1$-$C_6$ hydroxylalkyl, $C_1$-$C_6$ alkoxylalkyl, $C_1$-$C_6$ perfluoroalkoxyl or aryl;
each occurrence of the symbol n is independently an integer from 0 to 4; each occurrence of the symbol m and v is independently an integer from 1 to 2; each occurrence of the symbol X is independently a halogen;
or a salt thereof including a pharmaceutically acceptable salt thereof.

**13.** The UPR pathway inhibitors as indicated in claim 1, for use according to claim 1, selected from:

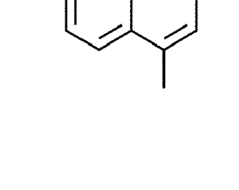

(4μ8c)

or

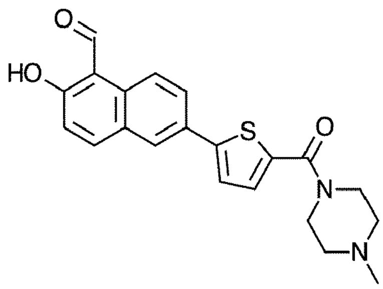

(MKC-3946)

or

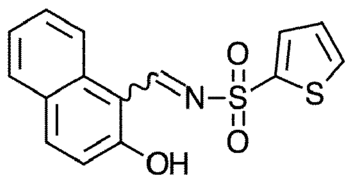

(STF-083010)

or

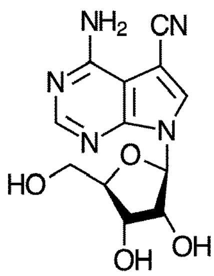

(toyocamycin)

or

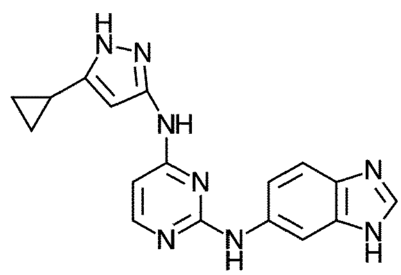

(APY29)

or

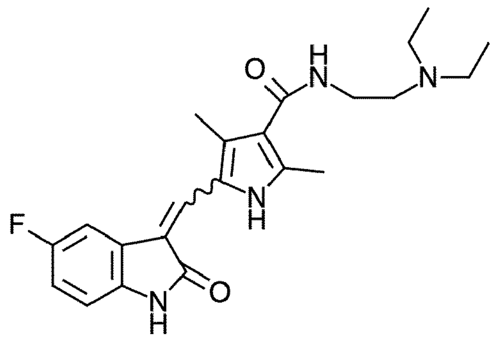

(Sunitinib)

or

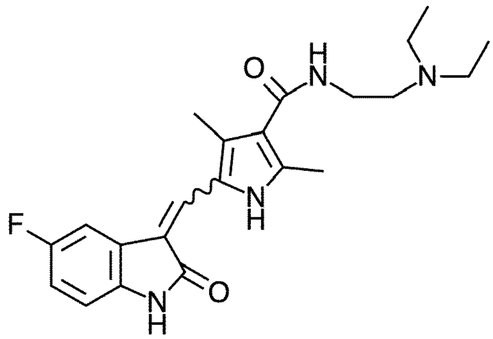

* $C_4H_6O_5$

(Sunitinib malate)

or

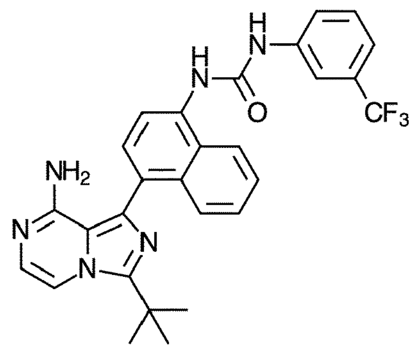

(KIRA-6)

or

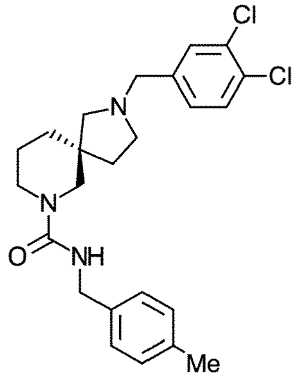

(GSK2850163)

or a salt thereof including a pharmaceutically acceptable salt thereof.

**14.** The UPR pathway inhibitors as indicated in anyone of claims 2-13, for use according to claim 1, whereby the neurodevelopmental disorders are related to an endoplasmic reticulum (ER) stress disorder.

**15.** The UPR pathway inhibitors as indicated in anyone of claims 2-39, for use according to claim 1, whereby the neurodevelopmental disorders comprise but are not limited to microcephaly caused by a viral infection in a fetal phase; in particular microcephaly caused by Zika virus (ZIKV).

Fig. 1

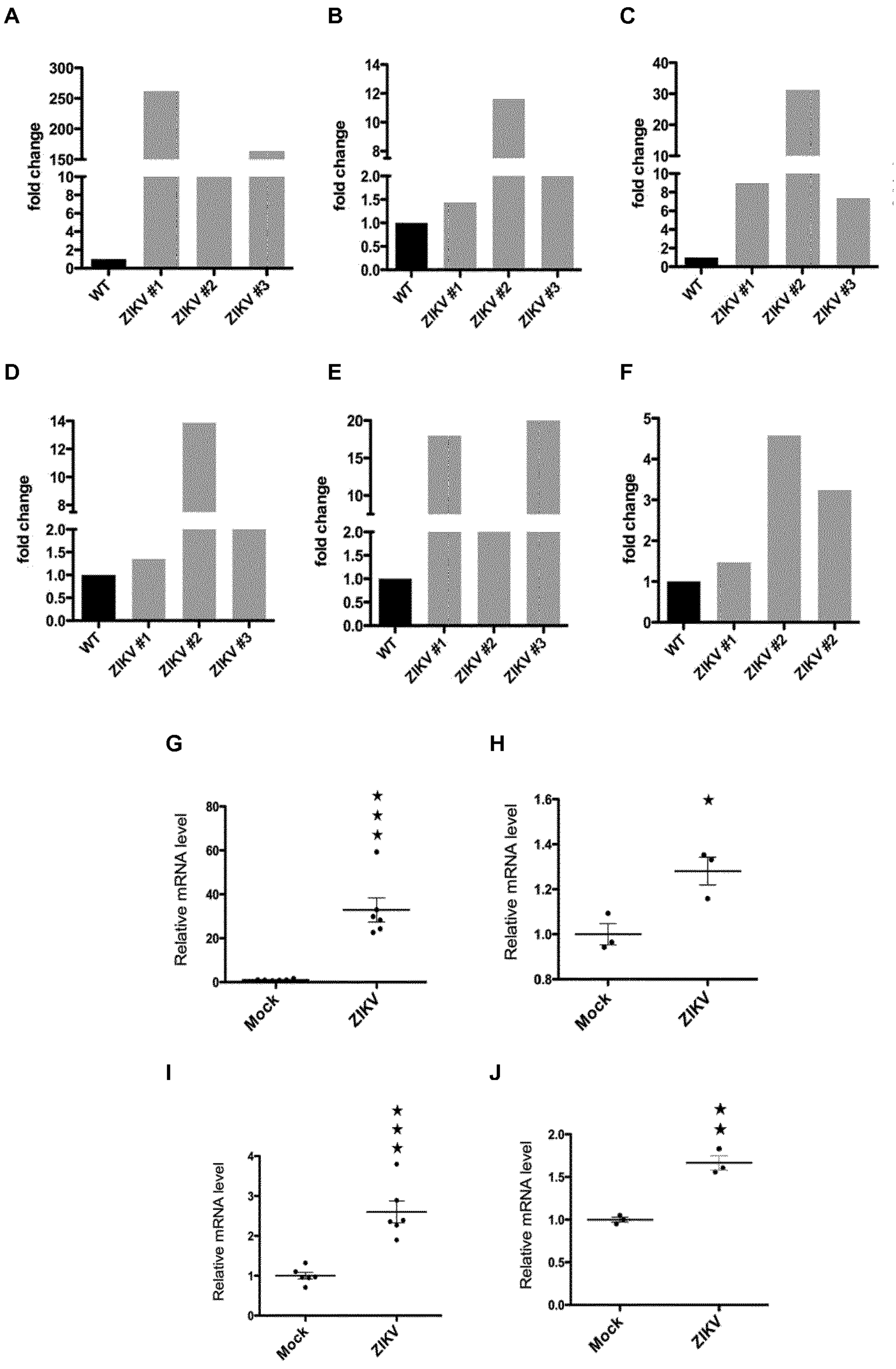
A
B
C
D
E
F
G
H
I
J
fold change
WT
ZIKV #1
ZIKV #2
ZIKV #3
Relative mRNA level
Mock
ZIKV

K

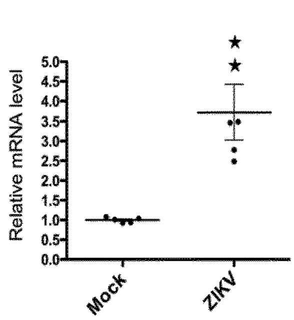
Relative mRNA level
5.0
4.5
4.0
3.5
3.0
2.5
2.0
1.5
1.0
0.5
0.0
Mock
ZIKV

L

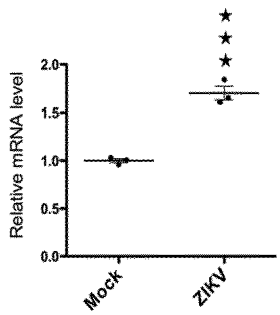
Relative mRNA level
2.0
1.5
1.0
0.5
0.0
Mock
ZIKV

M

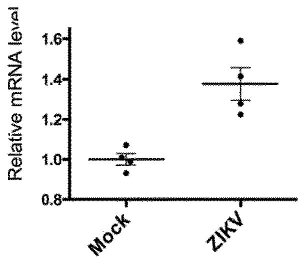
Relative mRNA level
1.6
1.4
1.2
1.0
0.8
Mock
ZIKV

N

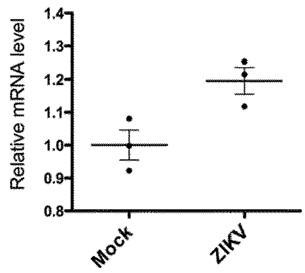
Relative mRNA level
1.4
1.3
1.2
1.1
1.0
0.9
0.8
Mock
ZIKV

O

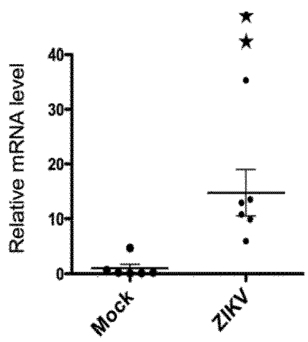
Relative mRNA level
40
30
20
10
0
Mock
ZIKV

P

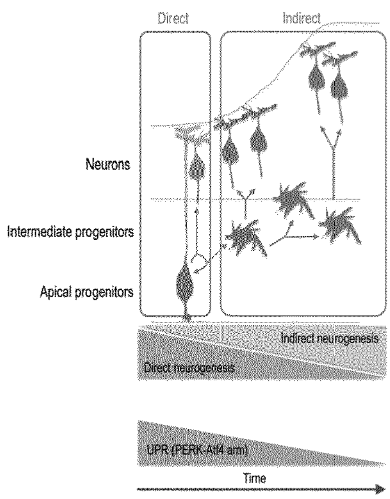
Direct
Indirect
Neurons
Intermediate progenitors
Apical progenitors
Indirect neurogenesis
Direct neurogenesis
UPR (PERK-Atf4 arm)
Time

Fig. 2

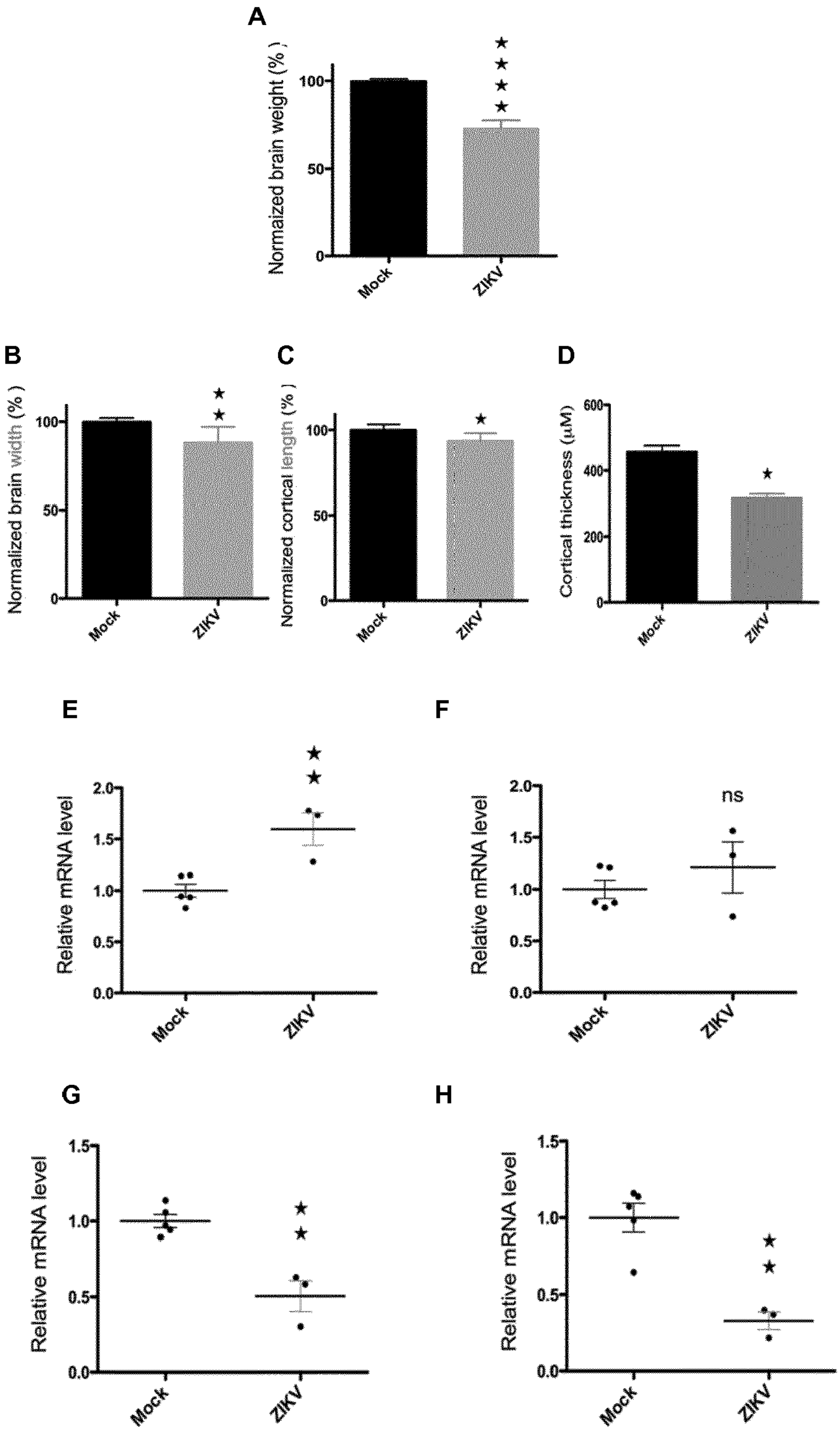
A
Normaized brain weight (%)
100
50
0
Mock
ZIKV
B
Normalized brain width (%)
C
Normalized cortical length (%)
D
Cortical thickness (μM)
600
400
200
E
F
ns
Relative mRNA level
2.0
1.5
1.0
0.5
0.0
G
H

I

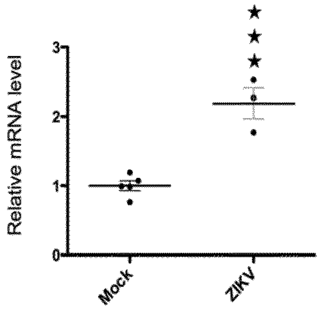
Relative mRNA level
0
1
2
3
Mock
ZIKV

J

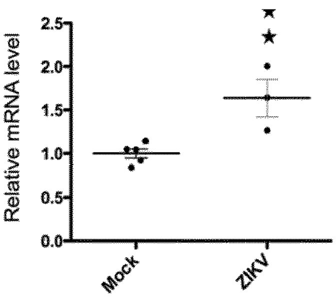
Relative mRNA level
0.0
0.5
1.0
1.5
2.0
2.5
Mock
ZIKV

K

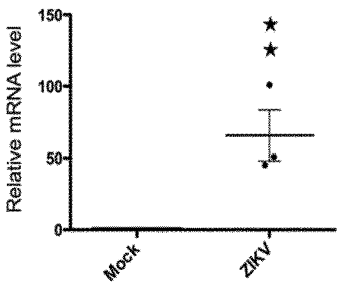
Relative mRNA level
0
50
100
150
Mock
ZIKV

L

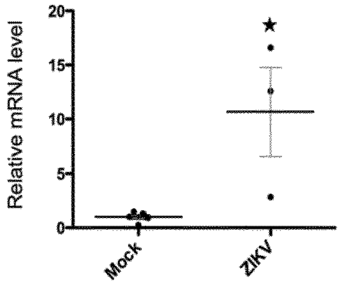
Relative mRNA level
0
5
10
15
20
Mock
ZIKV

M

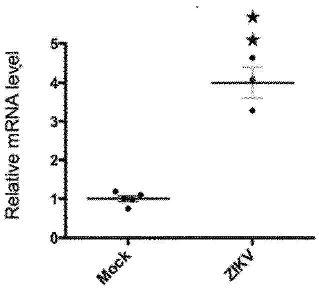
Relative mRNA level
0
1
2
3
4
5
Mock
ZIKV

N

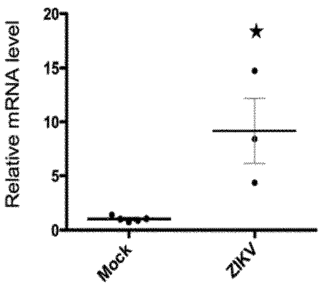
Relative mRNA level
0
5
10
15
20
Mock
ZIKV

O

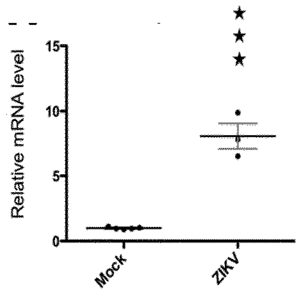
Relative mRNA level
0
5
10
15
Mock
ZIKV

P

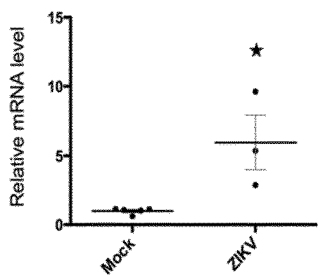
Relative mRNA level
0
5
10
15
Mock
ZIKV

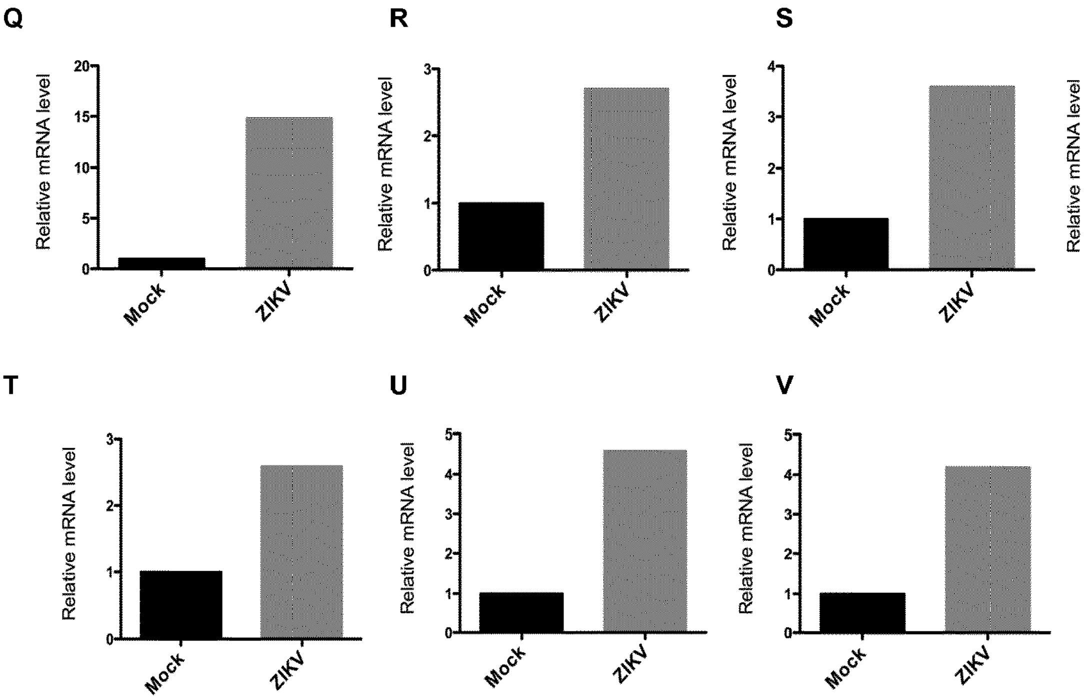
Q
R
S
Relative mRNA level
Mock
ZIKV
T
U
V
Relative mRNA level
Mock
ZIKV

Fig. 3

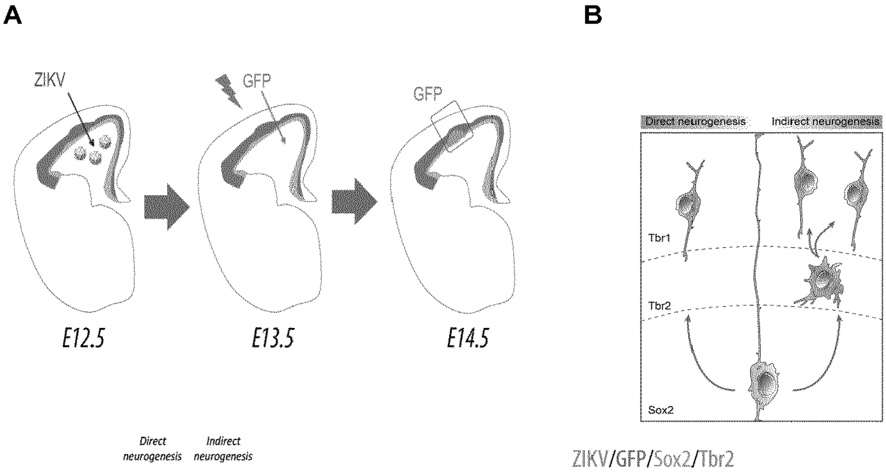
A
ZIKV
GFP
GFP
E12.5
E13.5
E14.5
Direct neurogenesis
Indirect neurogenesis
B
Direct neurogenesis
Indirect neurogenesis
Tbr1
Tbr2
Sox2
ZIKV/GFP/Sox2/Tbr2

C

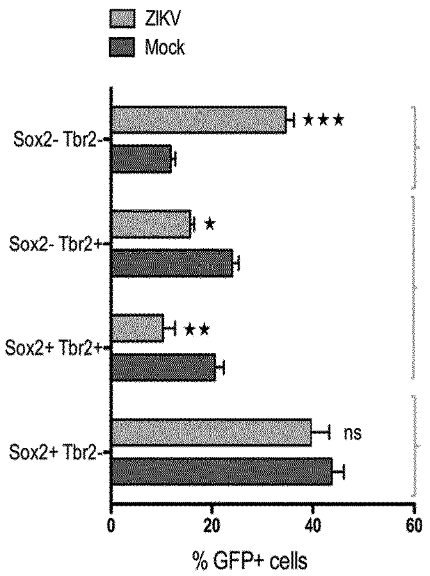
ZIKV
Mock
Sox2- Tbr2-
Sox2- Tbr2+
Sox2+ Tbr2+
Sox2+ Tbr2-
★★★
★
★★
ns
0
20
40
60
% GFP+ cells

D

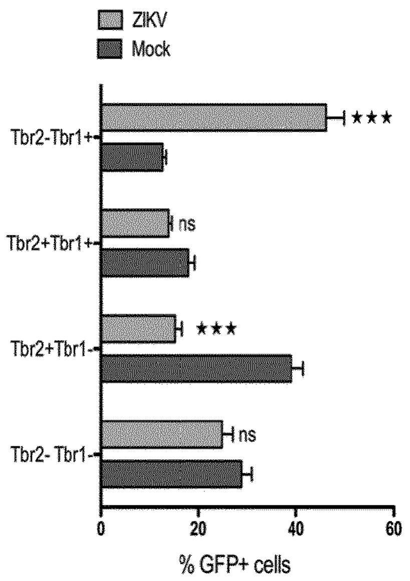
ZIKV
Mock
Tbr2-Tbr1+
Tbr2+Tbr1+
Tbr2+Tbr1-
Tbr2- Tbr1-
★★★
ns
★★★
ns
0
20
40
60
% GFP+ cells

E

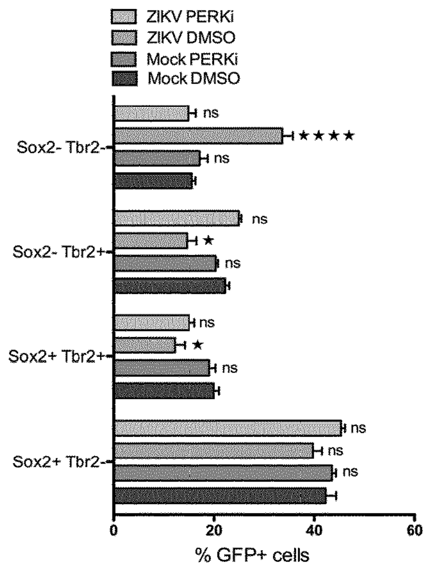
ZIKV PERKi
ZIKV DMSO
Mock PERKi
Mock DMSO
Sox2- Tbr2-
Sox2- Tbr2+
Sox2+ Tbr2+
Sox2+ Tbr2-
ns
★★★★
★
0
20
40
60
% GFP+ cells

F

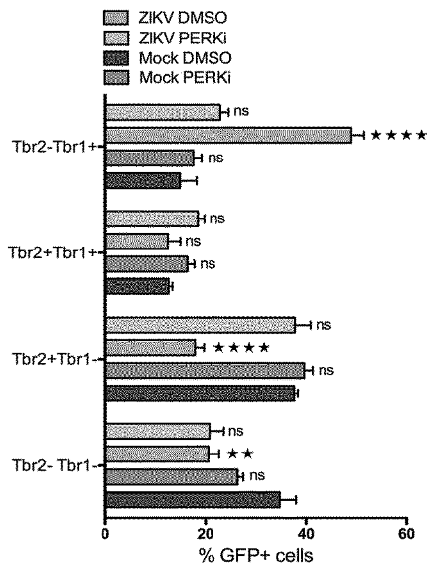
ZIKV DMSO
ZIKV PERKi
Mock DMSO
Mock PERKi
Tbr2-Tbr1+
Tbr2+Tbr1+
Tbr2+Tbr1-
Tbr2- Tbr1-
ns
★★★★
★★
0
20
40
60
% GFP+ cells

G

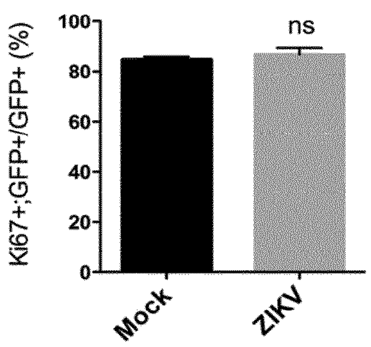
Ki67+;GFP+/GFP+ (%)
100
80
60
40
20
0
ns
Mock
ZIKV

H

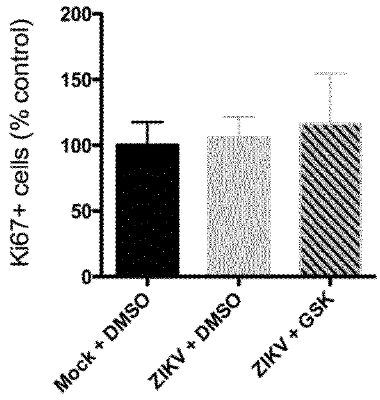
Ki67+ cells (% control)
200
150
100
50
0
Mock + DMSO
ZIKV + DMSO
ZIKV + GSK

Fig. 4

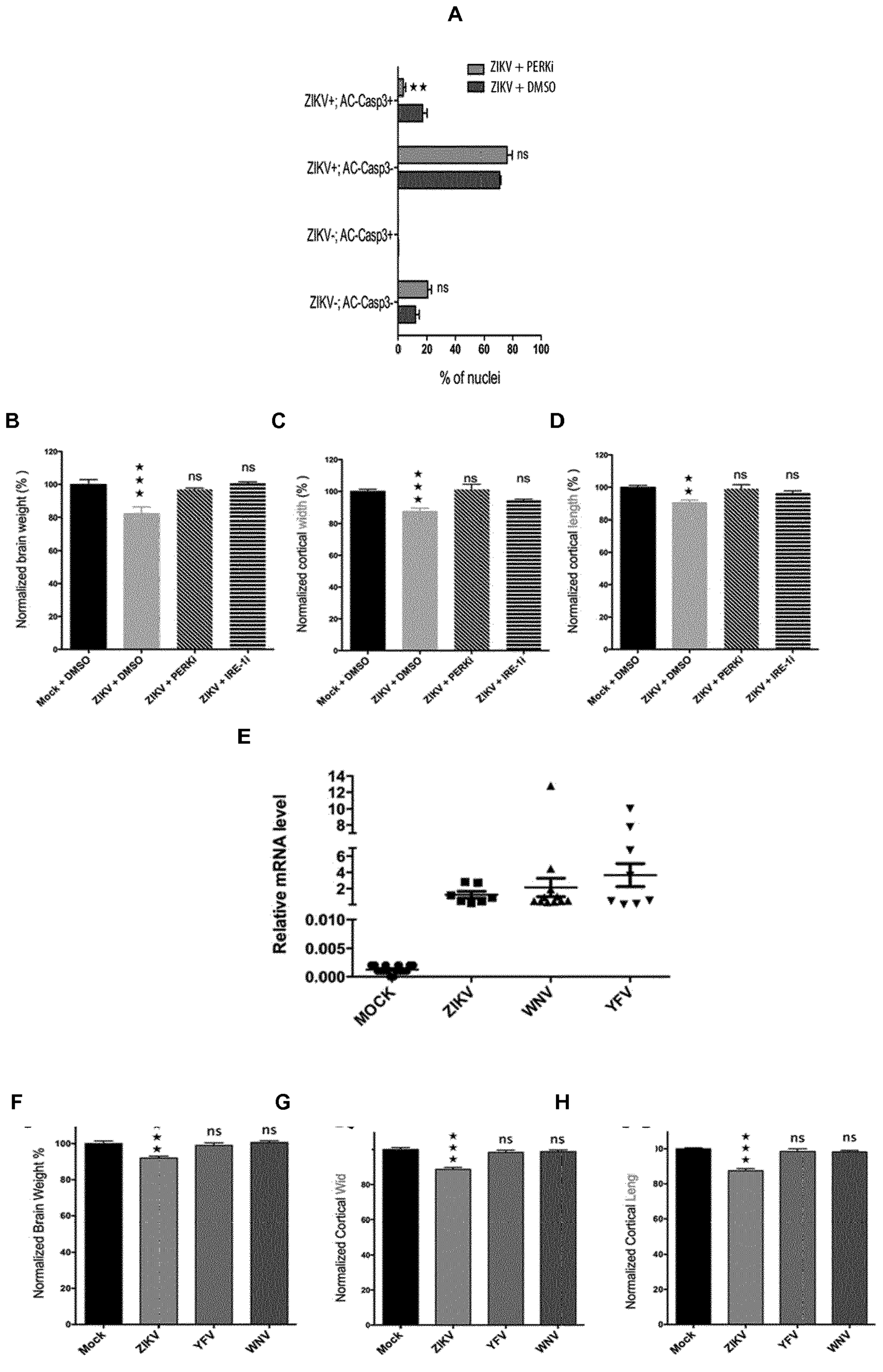
A
ZIKV + PERKi
ZIKV + DMSO
ZIKV+; AC-Casp3+
ZIKV+; AC-Casp3-
ZIKV-; AC-Casp3+
ZIKV-; AC-Casp3-
ns
% of nuclei
B
C
D
Normalized brain weight (%)
Normalized cortical width (%)
Normalized cortical length (%)
Mock + DMSO
ZIKV + DMSO
ZIKV + PERKi
ZIKV + IRE-1i
E
Relative mRNA level
MOCK
ZIKV
WNV
YFV
F
G
H
Normalized Brain Weight %
Normalized Cortical Wid
Normalized Cortical Leng
Mock
ZIKV
YFV
WNV

I

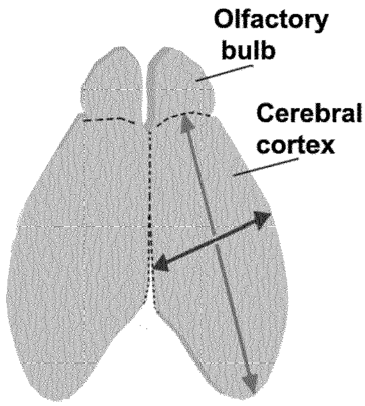
Olfactory
bulb
Cerebral
cortex

J

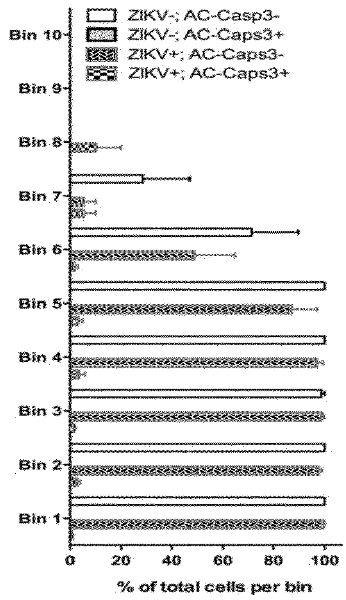
ZIKV-; AC-Casp3-
ZIKV-; AC-Caps3+
ZIKV+; AC-Caps3-
ZIKV+; AC-Caps3+
Bin 10
Bin 9
Bin 8
Bin 7
Bin 6
Bin 5
Bin 4
Bin 3
Bin 2
Bin 1
0
20
40
60
80
100
% of total cells per bin

Fig. 5

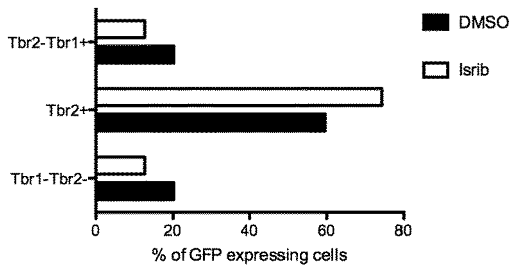
DMSO
Isrib
Tbr2-Tbr1+
Tbr2+
Tbr1-Tbr2-
0
20
40
60
80
% of GFP expressing cells

Fig. 6

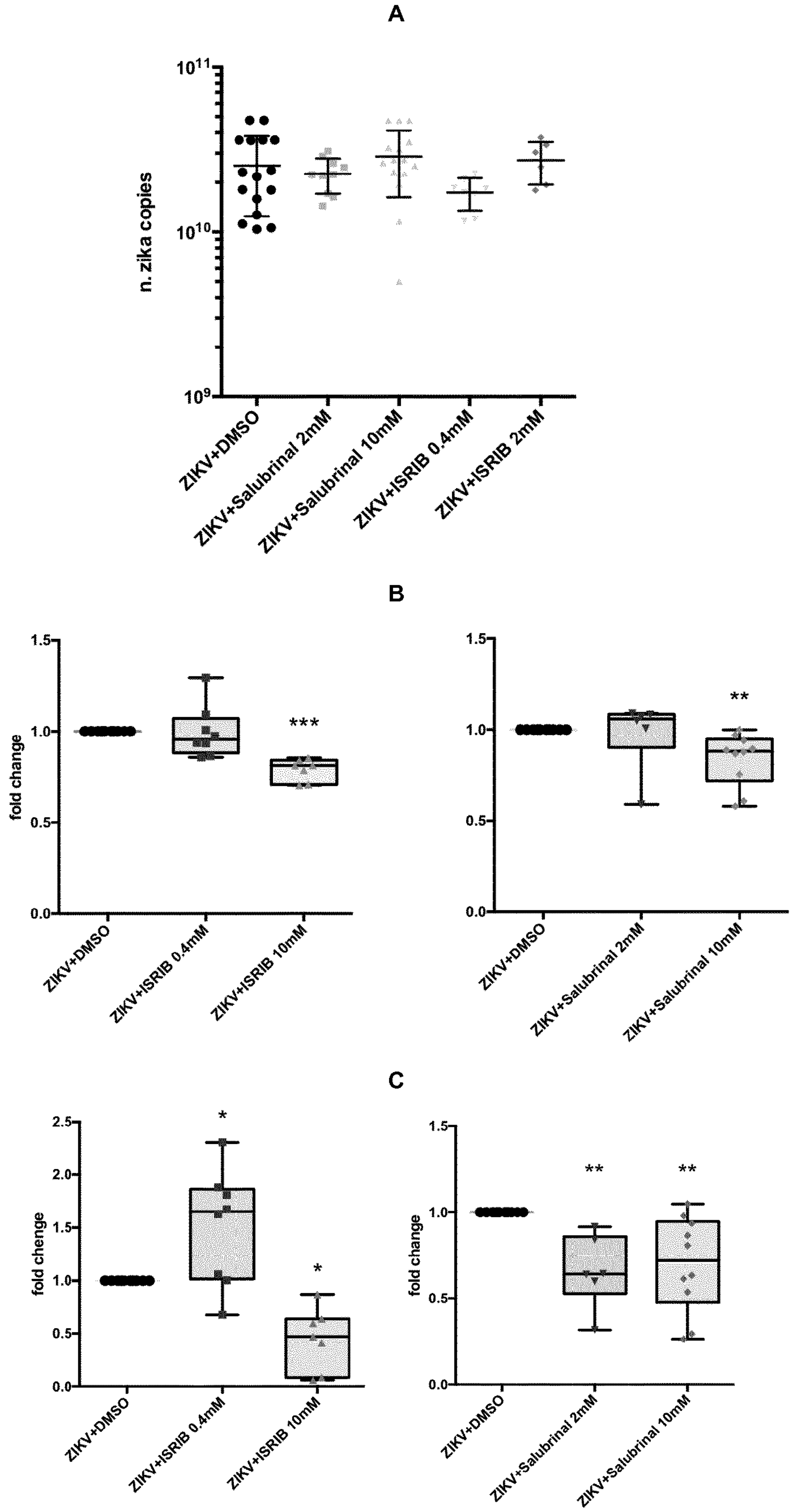
A
n. zika copies
$10^{11}$
$10^{10}$
$10^{9}$
ZIKV+DMSO
ZIKV+Salubrinal 2mM
ZIKV+Salubrinal 10mM
ZIKV+ISRIB 0.4mM
ZIKV+ISRIB 2mM
B
fold change
1.5
1.0
0.5
0.0
***
ZIKV+DMSO
ZIKV+ISRIB 0.4mM
ZIKV+ISRIB 10mM
1.5
1.0
0.5
0.0
**
ZIKV+DMSO
ZIKV+Salubrinal 2mM
ZIKV+Salubrinal 10mM
C
fold change
2.5
2.0
1.5
1.0
0.5
0.0
*
*
ZIKV+DMSO
ZIKV+ISRIB 0.4mM
ZIKV+ISRIB 10mM
fold chenge
1.5
1.0
0.5
0.0
**
**
ZIKV+DMSO
ZIKV+Salubrinal 2mM
ZIKV+Salubrinal 10mM

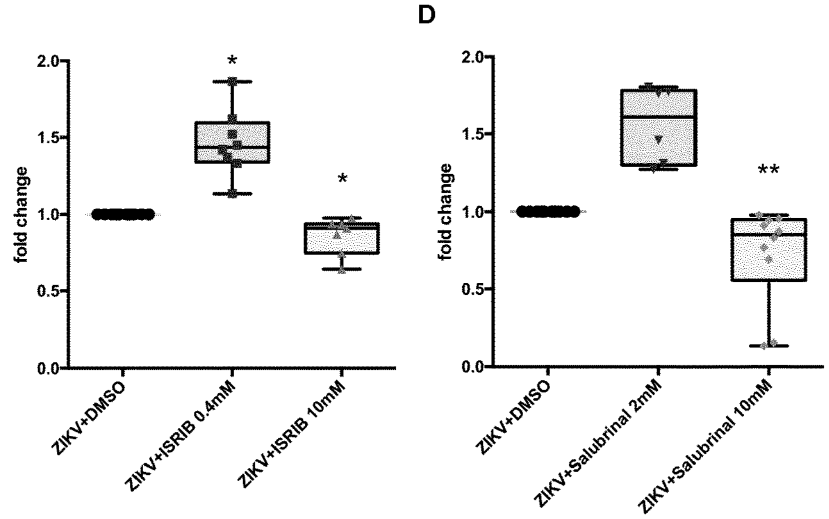
D
2.0
1.5
1.0
0.5
0.0
fold change
*
*
**
ZIKV+DMSO
ZIKV+ISRIB 0.4mM
ZIKV+ISRIB 10mM
ZIKV+Salubrinal 2mM
ZIKV+Salubrinal 10mM

Fig. 7

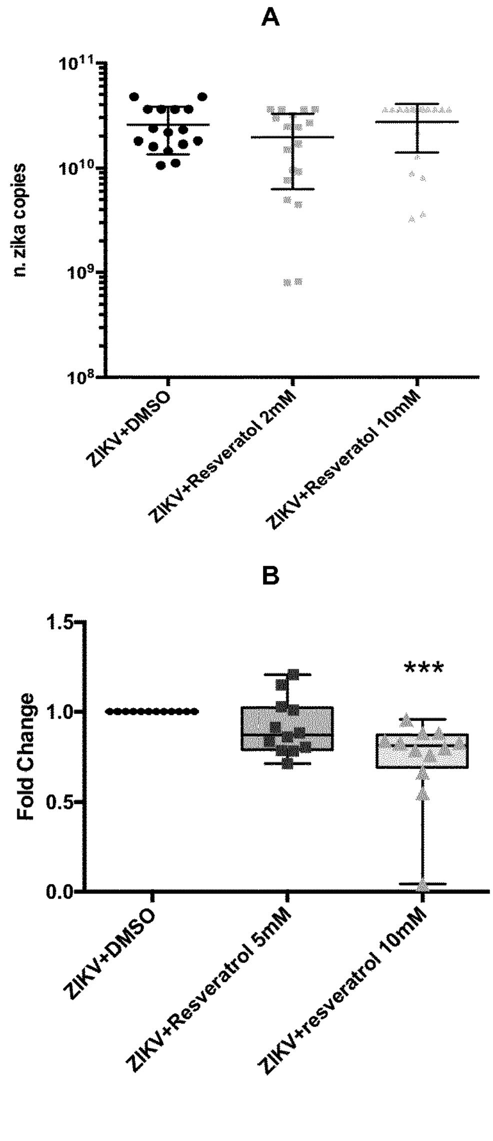
A
$10^{11}$
$10^{10}$
$10^{9}$
$10^{8}$
n. zika copies
ZIKV+DMSO
ZIKV+Resveratol 2mM
ZIKV+Resveratol 10mM
B
1.5
1.0
0.5
0.0
Fold Change
***
ZIKV+DMSO
ZIKV+Resveratrol 5mM
ZIKV+resveratrol 10mM

C

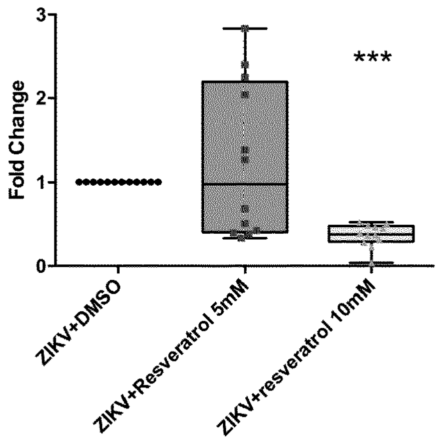
3
2
1
0
Fold Change
***
ZIKV+DMSO
ZIKV+Resveratrol 5mM
ZIKV+resveratrol 10mM

D

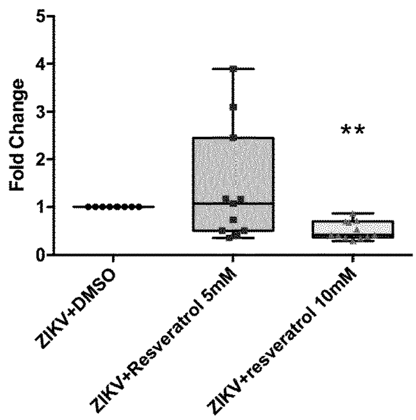
5
4
3
2
1
0
Fold Change
**
ZIKV+DMSO
ZIKV+Resveratrol 5mM
ZIKV+resveratrol 10mM

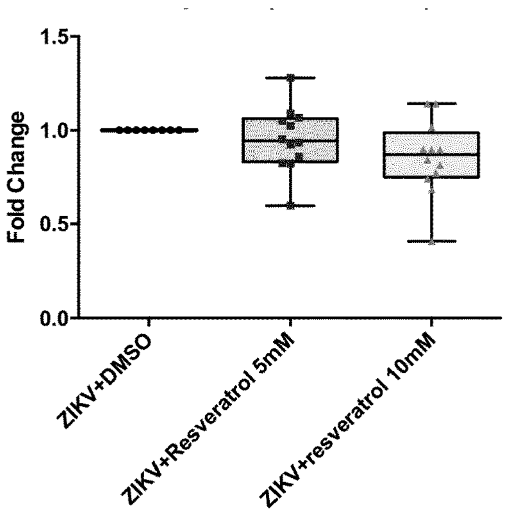
1.5
1.0
0.5
0.0
Fold Change
ZIKV+DMSO
ZIKV+Resveratrol 5mM
ZIKV+resveratrol 10mM

E

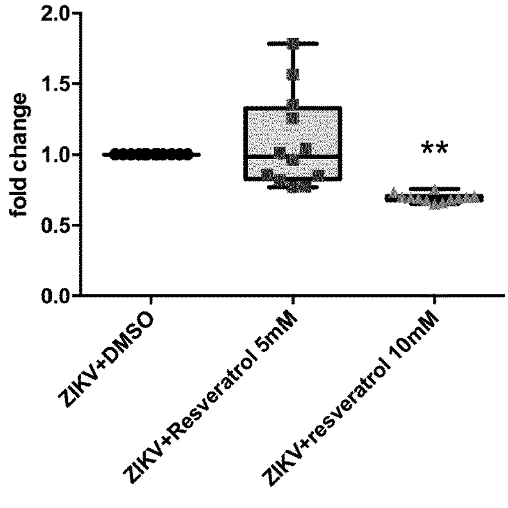
2.0
1.5
1.0
0.5
0.0
fold change
**
ZIKV+DMSO
ZIKV+Resveratrol 5mM
ZIKV+resveratrol 10mM

Europäisches Patentamt
European Patent Office
Office européen des brevets

# EUROPEAN SEARCH REPORT

Application Number
EP 19 19 3344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/176348 A1 (H LEE MOFFITT CANCER CT & RES [US]) 30 October 2014 (2014-10-30)<br>* page 32, line 14 *<br>* figure 10 *<br>* page 76, lines 11-12 *<br>----- | 1-5, 13-15 | INV.<br>A61K31/165<br>A61K31/4706<br>A61K31/519<br>A61P25/00 |
| X | WO 2008/060448 A2 (MASSACHUSETTS INST TECHNOLOGY [US]; AMBIT BIOSCIENCES CORP [US] ET AL.) 22 May 2008 (2008-05-22)<br>* abstract *<br>* claims *<br>----- | 1,10,11, 13-15 | |
| X | WO 2010/098888 A1 (MASSACHUSETTS INST TECHNOLOGY [US]; GEN HOSPITAL CORP [US] ET AL.) 2 September 2010 (2010-09-02)<br>* claims 102,115,117,120,121 *<br>----- | 1,10,11, 13-15 | |
| X | XU LE ET AL: "Protective effects of resveratrol on the inhibition of hippocampal neurogenesis induced by ethanol during early postnatal life", BIOCHIMICA ET BIOPHYSICA ACTA. MOLECULAR BASIS OF DISEASE, AMSTERDAM, NL, vol. 1852, no. 7, 24 March 2015 (2015-03-24), pages 1298-1310, XP029232455, ISSN: 0925-4439, DOI: 10.1016/J.BBADIS.2015.03.009<br>* abstract *<br>-----<br>-/-- | 1 | TECHNICAL FIELDS SEARCHED (IPC)<br>A61K<br>A61P |

The present search report has been drawn up for all claims

3

| Place of search | Date of completion of the search | Examiner |
|---|---|---|
| Munich | 17 April 2020 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches Patentamt
European Patent Office
Office européen des brevets

# EUROPEAN SEARCH REPORT

Application Number
EP 19 19 3344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BAKHEET SALEH A ET AL: "Resveratrol Ameliorates Dysregulation of Th1, Th2, Th17, and T Regulatory Cell-Related Transcription Factor Signaling in a BTBR T + tf/J Mouse Model of Autism", MOLECULAR NEUROBIOLOGY, HUMANA PRESS, US, vol. 54, no. 7, 30 August 2016 (2016-08-30), pages 5201-5212, XP036287471, ISSN: 0893-7648, DOI: 10.1007/S12035-016-0066-1 [retrieved on 2016-08-30] * abstract * ----- | 1 | |
| X | BR 1020 1202 9382 A2 (UNIV FED DO RIO GRANDE DO SUL [BR]) 23 September 2014 (2014-09-23) * abstract * * claims * ----- | 1 | |
| X | WO 2010/031056 A2 (UNIV CALIFORNIA [US]; WALTER PETER [US] ET AL.) 18 March 2010 (2010-03-18) * abstract * * paragraph [0134] * * claims 1,47,54 * ----- | 1,10,11, 13-15 | TECHNICAL FIELDS SEARCHED (IPC) |
| X | WO 2016/004254 A1 (UNIV CALIFORNIA [US]; MALY DUSTIN J [US]) 7 January 2016 (2016-01-07) * abstract * * paragraphs [0104], [0133] * * claims * ----- -/-- | 1-15 | |

The present search report has been drawn up for all claims

3

| Place of search | Date of completion of the search | Examiner |
|---|---|---|
| Munich | 17 April 2020 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 19 3344

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | CLAUDIO HETZ ET AL: "Targeting the unfolded protein response in disease", NATURE REVIEWS. DRUG DISCOVERY, vol. 12, no. 9, 1 September 2013 (2013-09-01), pages 703-719, XP055557628, GB ISSN: 1474-1776, DOI: 10.1038/nrd3976 * abstract * * page 707; table 2 * * page 710 * ----- | 1-15 | |
| A | JUAN R. CUBILLOS-RUIZ ET AL: "Molecular Pathways: Immunosuppressive Roles of IRE1[alpha]-XBP1 Signaling in Dendritic Cells of the Tumor Microenvironment", CLINICAL CANCER RESEARCH, vol. 22, no. 9, 15 March 2016 (2016-03-15), pages 2121-2126, XP055673622, US ISSN: 1078-0432, DOI: 10.1158/1078-0432.CCR-15-1570 * abstract * * page 12; figure 2 * ----- | 1-15 | TECHNICAL FIELDS SEARCHED (IPC) |
| A | LUDEK EYER ET AL: "Nucleoside Inhibitors of Zika Virus", JOURNAL OF INFECTIOUS DISEASES. JID, vol. 214, no. 5, 27 May 2016 (2016-05-27), pages 707-711, XP055672762, US ISSN: 0022-1899, DOI: 10.1093/infdis/jiw226 * abstract * * page 709, right-hand column, last line * ----- -/-- | 1-15 | |

The present search report has been drawn up for all claims

3

| Place of search | Date of completion of the search | Examiner |
|---|---|---|
| Munich | 17 April 2020 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 19 3344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARCO ONORATI ET AL: "Zika Virus Disrupts Phospho-TBK1 Localization and Mitosis in Human Neuroepithelial Stem Cells and Radial Glia", CELL REPORTS, vol. 16, no. 10, 1 September 2016 (2016-09-01), pages 2576-2592, XP055383655, US ISSN: 2211-1247, DOI: 10.1016/j.celrep.2016.08.038 * abstract * * page 2586, left-hand column, paragraph 2 - right-hand column, paragraph 1 * ----- | 1-15 | |
| A | MIAO XU ET AL: "Identification of small-molecule inhibitors of Zika virus infection and induced neural cell death via a drug repurposing screen", NATURE MEDICINE, vol. 22, no. 10, 29 August 2016 (2016-08-29), pages 1101-1107, XP055383662, ISSN: 1078-8956, DOI: 10.1038/nm.4184 * abstract * ----- | 1-15 | TECHNICAL FIELDS SEARCHED (IPC) |
| T | ELENA P. KOLPIKOVA ET AL: "IRE1[alpha] Promotes Zika Virus Infection via XBP1", VIRUSES, vol. 12, no. 3, 3 March 2020 (2020-03-03), page 278, XP055673654, DOI: 10.3390/v12030278 * page 9, line 9 - page 10, line 4 * ----- | 1-15 | |

The present search report has been drawn up for all claims

3

| Place of search | Date of completion of the search | Examiner |
|---|---|---|
| Munich | 17 April 2020 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 19 19 3344

This annex lists the patent family members relating to the patent documents cited in the above-mentioned European search report. The members are as contained in the European Patent Office EDP file on
The European Patent Office is in no way liable for these particulars which are merely given for the purpose of information.

17-04-2020

| Patent document cited in search report | Publication date | Patent family member(s) | Publication date |
|---|---|---|---|
| WO 2014176348 A1 | 30-10-2014 | US 2016083361 A1 | 24-03-2016 |
| | | US 2020024247 A1 | 23-01-2020 |
| | | WO 2014176348 A1 | 30-10-2014 |
| WO 2008060448 A2 | 22-05-2008 | CA 2669084 A1 | 29-05-2008 |
| | | EP 2089029 A2 | 19-08-2009 |
| | | EP 2433635 A1 | 28-03-2012 |
| | | ES 2397637 T3 | 08-03-2013 |
| | | US 2010247552 A1 | 30-09-2010 |
| | | US 2011294782 A1 | 01-12-2011 |
| | | WO 2008060448 A2 | 22-05-2008 |
| | | WO 2008063933 A2 | 29-05-2008 |
| WO 2010098888 A1 | 02-09-2010 | US 2011008468 A1 | 13-01-2011 |
| | | WO 2010098888 A1 | 02-09-2010 |
| BR 102012029382 A2 | 23-09-2014 | NONE | |
| WO 2010031056 A2 | 18-03-2010 | AU 2009290617 A1 | 18-03-2010 |
| | | AU 2015210470 A1 | 03-09-2015 |
| | | CA 2737388 A1 | 18-03-2010 |
| | | CN 102264728 A | 30-11-2011 |
| | | CN 104230901 A | 24-12-2014 |
| | | EP 2340248 A2 | 06-07-2011 |
| | | HK 1163689 A1 | 04-12-2015 |
| | | JP 6144873 B2 | 07-06-2017 |
| | | JP 6412968 B2 | 24-10-2018 |
| | | JP 2012502916 A | 02-02-2012 |
| | | JP 2015017099 A | 29-01-2015 |
| | | JP 2017160197 A | 14-09-2017 |
| | | US 2011319436 A1 | 29-12-2011 |
| | | US 2015011575 A1 | 08-01-2015 |
| | | WO 2010031056 A2 | 18-03-2010 |
| WO 2016004254 A1 | 07-01-2016 | US 2017165259 A1 | 15-06-2017 |
| | | WO 2016004254 A1 | 07-01-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016126026 A
- WO 2012158123 A
- WO 2016043874 A
- WO 2016025635 A
- US 20120077828 A
- WO 2011119663 A
- US 20160015709 A
- US 20140364453 A
- WO 2015002729 A
- WO 2014144952 A
- WO 2008154484 A
- EP 3150589 A
- EP 2532643 A
- WO 2002022581 A
- US 6372778 A
- US 6369086 A
- US 6369087 A
- US 6372733 A
- EP 721331 A
- US 4997834 A
- EP 0370498 A


### Non-patent literature cited in the description


- Remington's Pharmaceutical Sciences
- *Journal Officiel des Communaute's Européennes L358,* 18 December 1986
- **GLADWYN.** *Nature Neurosciences,* 2018
- **LAMBERT, N. et al.** Genes expressed in specific areas of the human fetal cerebral cortex display distinct patterns of evolution. *PLoS One,* 2011, vol. 6, e17753
- **CAU, E. ; GRADWOHL, G. ; FODE, C. ; GUILLEMOT, F.** Mash1 activates a cascade of bHLH regulators in olfactory neuron progenitors. *Development,* 1997, vol. 124, 1611-1621
- **BORGS, L. et al.** Dopaminergic neurons differentiating from LRRK2 G2019S induced pluripotent stem cells show early neuritic branching defects. *Scientific reports,* 2016, vol. 6, 33377
- **LAGUESSE, S. et al.** A Dynamic Unfolded Protein Response Contributes to the Control of Cortical Neurogenesis. *Dev Cell,* 2015, vol. 35, 553-567
- **WALTER ; RON.** The Unfolded Protein Response: from stress pathway to homeostatic regulation. *Science,* 2011, vol. 334, 1081-1086
- **TOMASIO et al.** Selective inhibition of the unfolded protein response: targeting catalytic sites for Schiff base modification. *Molecular Biosystems,* 2013, vol. 9, 2408-2416
- **TSURU et al.** *Novel Mechanisms of enhancing IRE1α-XBP1 signalling via the PERK-ATF4 pathway Scientific Reports,* 2016, vol. 6, 24217
- **JIANG et al.** Targeting the IRE1α-XBP1 branch of the Unfolded Protein Response. *Human Diseases. Semin. Cancer Biol.,* 2015, vol. 33, 48-56
- **HALLIDAY ; MALLUCCI.** Review: Modulating the unfolded protein response to prevent neurodegeneration and enhance memory. *Neuropathology & Applied Neurobiology,* 2015, vol. 41, 414-427
- **LANCIOTTI, R. S. et al.** Genetic and serologic properties of Zika virus associated with an epidemic, Yap State, Micronesia, 2007. *Emerg. Infect. Dis.,* 2008, vol. 14, 1232-1239
- **GLADWYN-NG et al.** Stress-induced unfolded protein response contributes to Zika virus-associated microcephaly. *Nature Neurosciences,* 2018, vol. 21, 63-71